# EUROPEAN PATENT SPECIFICATION

(11) **EP 0 594 702 B1**
(45) Date of publication and mention of the grant of the patent: **29.01.1997**
(21) Application number: 92915109.0
(22) Date of filing: 23.06.1992
(51) Int. Cl.: C07D 401/12, C07D 401/14, C07D 417/14, A61K 31/395

(54) **SUBSTITUTED INDOLES AS ANTI-AIDS PHARMACEUTICALS**
SUBSTITUIERTE INDOLE ALS ANTI-AIDS PHARMACEUTISCHE ZUBEREITUNGEN
INDOLES SUBSTITUES UTILISES COMME PRODUITS PHARMACEUTIQUES DANS LE TRAITEMENT DU SIDA

(30) Priority: 03.07.1991 US 725053
(43) Date of publication of application: 04.05.1994
(73) Proprietor: PHARMACIA & UPJOHN COMPANY, Kalamazoo, Michigan 49001 (US)
(72) Inventor: ROMERO, Donna, Lee, Kalamazoo, MI 49008 (US); THOMAS, Richard, Charles, Kalamazoo, MI 49009 (US)
(74) Representative: Perry, Robert Edward
(86) International application number: US9205067
(87) International publication number: WO9301181

(56) References cited:
- WO-A-91/09849
- US-A- 4 302 589
- PROCEEDINGS OF THE NATIONAL ACADEMY OF SCIENCES OF USA. vol. 88, 1991, WASHINGTON US pages 8806 - 8810; D.L. ROMERO ET AL.: 'Nonnucleoside reverse transcriptase inhibitors that potently and specifically block human immunodeficiency virus type 1 replication.'

## Description

### BACKGROUND OF THE INVENTION

### 1. Field of the Invention

The oxygen and nitrogen substituted indoles described are useful in the treatment of AIDS.

### 2. Description of the Related Art

International Publication No. WO 87/01797 discloses compounds which can be visualized as steroid-piperazine-[substituted aromatic] or steroid-piperazine-[substituted heteroaromatic]. The steroid and piperazine being "connected" via the C₁₇ side-chain of the steroid.

International Publication No. WO 88/08424 disclosed compounds which can be visualized as aromatic-connector-piperazine-[substituted aromatic] or aromatic-connector-piperazine[substituted heteroaromatic], in particular see the compounds of formulas (I) and (III). None of those compounds were disclosed as having the utility set forth in this invention. In U.S. Patent application 07/425,726, filed 10-23-89, it was disclosed that the compounds of formula (I) of International Publication No. WO 88/08424 were useful against AIDS.

An estimated one to one and one-half million people in the United States are infected with a human retrovirus, the human immunodeficiency virus type I (HIV-1) which is the etiological agent of acquired immunodeficiency syndrome, AIDS, see Science, 661-662 (1986). Of those infected, an estimated two hundred and fifty thousands people will develop AIDS in the next five years, see Science, 1352-1357 (1985). On March 20, 1987, the FDA approved the use of the compound, AZT (zidovudine), to treat AIDS patients with a recent initial episode of pneumocystis carinii pneumonia, AIDS patients with conditions other than pneumocystis carinii pneumonia or patients infected with the virus with an absolute CD4 lymphocyte count of less than 200/mm³ in the peripheral blood. AZT is a known inhibitor of viral reverse transcriptase, an enzyme necessary for human immunodeficiency virus replication.

U.S. Patent 4,724,232 claims a method of treating humans having acquired immunodeficiency syndrome utilizing 3'-azido-3'-deoxy-thymidine (azidothymidine, AZT).

It is known in the art that certain antibiotics and polyanionic dyes inhibit retrovirus reverse transcriptase.

Many publications have reported the ability of various sulfated compounds to inhibit virus replication, including HIV.

Nature 343, 470 (1990) and Science 250, 1411 (1990) discloses potent benzodiazepin type reverse transcriptase inhibitors. The compounds of the present invention are not benzodiazepin type compounds.

US Patent 3,188,313 discloses compounds of the general formula

[substituted indol-2-yl]-(CH₂)ₙ-[piperazinyl type]-[pyridinyl/pyrimidinyl].

The substituted indoles (I) of the present invention differ from the prior art compounds in that the substitution on the -φ ring of the indole is a different group than that of the group in US Patent 3,188,313.

US Patent 3,472,855 and 3,562,278 disclose 3-indolinyl compounds which are useful as psychomotor depressants. The substituted indoles (I) of the present invention are useful for a totally different purpose, inhibition of HIV-RT and treatment of AIDS.

US Patent 3,362,956 discloses compounds of the general formula

[3-quinolyl]-(CH₂)ₙ-[piperazinyl type]-[pyridinyl/phenyl].

The substituted indoles (I) of the present invention differ from the prior art compounds in that they do not include 3-quinolyl type compounds.

US Patent 3,472,854 discloses compounds of the general formula

[2-benzimidazolyl]-(CH₂)ₙ-[piperazinyl type]-[pyridinyl/phenyl].

The substituted indoles (I) of the present invention differ from the prior art compounds in that they are indoles and not 2-benzimidazolyl type compounds.

US Patent 3,491,098 discloses compounds of the general formula

[4(5)-imidazolyl]-(CH₂)ₙ-[piperazinyl type]-[pyridinyl/phenyl].

The substituted indoles (I) of the present invention differ from the prior art compounds in that they are indoles and not imidazolyl type compounds.

US Patent 3,511,841 discloses compounds of the general formula

[azaindolyl]-(CH₂)ₙ-[piperazinyl type]-[pyridinyl/phenyl]

[azaindolyl]-CO-[piperazinyl type]-[pyridinyl/phenyl]

The substituted indoles (I) of the present invention differ from the prior art compounds in that they are they have substituted oxygen or substituted amino groups on the -φ portion of the indole and do not contain nitrogen in the ring.

US Patent 4,302,589 discloses 3-indolinyl compounds with a methyl group at the C₂ position of the indole and an ethyl bridge between the indole and piperazine which are useful as anti-psychotics. The substituted indoles (I) of the present invention are useful for a totally different purpose, inhibition of HIV-RT and treatment of AIDS.

European patent publication 345,808 discloses 3-indolinyl-piperazinyl-[substituted 2-pyridinyl] compounds (example 66) which are useful as anti-depressants. The substituted indoles (I) of the present invention are useful for a totally different purpose, inhibition of HIV-RT and treatment of AIDS.

There are a number of other chemically unrelated compounds which have been reported to inhibit HIV and/or be useful in the treatment of AIDS.

### SUMMARY OF INVENTION

Disclosed are substituted indoles of formula (I) 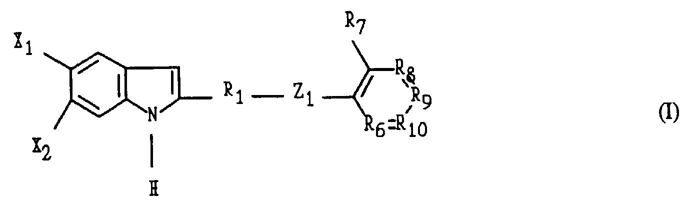 where R₁ is -CH₂- or -CO-;
where Z₁ is 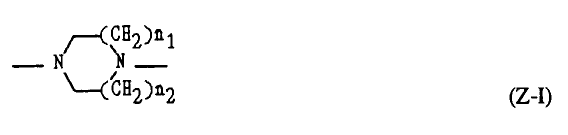 where n₁ is 1 or 2 and n₂ is 1 or 2, or 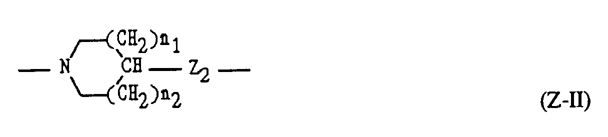 where Z₂ is -N(Z₂₋₁)- where Z₂₋₁ is C₁-C₄ alkyl and where n₁ and n₂ are as defined above; where one of X₁ and X₂ is H and the other is
-O-(CH₂CH₂-O-)ₙ₃-X₁₋₁ where n₃ is 1 thru 4, where X₁₋₁ is -H or C₁-C₄ alkyl,
-O-SO₂-(CH₂)ₙ₄-N(X₁₋₂)(X₁₋₃) where n₄ is 1 thru 3, where either X₁₋₂ and X₁₋₃ are the same or different and are -H or C₁-C₆ alkyl, or X₁₋₂ and X₁₋₃ are taken together with the attached nitrogen atom, to form a ring selected from 1-pyrrolidinyl, 1-piperidinyl, 1-piperazinyl, 1-morpholinyl and 1-piperazinyl substituted in the 4-position with C₁-C₅ alkyl,
-O-SO₂-(CH₂)ₙ₄-X₁₋₈, where X₁₋₈ is 2-pyridinyl, 3-pyridinyl and 4-pyridinyl and where n₄ is as defined above,
-O-CO-(CH₂)ₙ₄-NX₁₋₂X₁₋₃ where n₄, X₁₋₂ and X₁₋₃ are as defined above,
-NH-CO-(CH₂)ₙ₄-NX₁₋₂X₁₋₃ where n₄, X₁₋₂ and X₁₋₃ are as defined above,
-N(X₁₋₄)-SO₂-X₁₋₅ where X₁₋₄ is C₁-C₃ alkyl and X₁₋₅ is C₁-C₄ alkyl,
-N(X₁₋₇)-CO-NH-(CH₂)ₙ₄-N(X₁₋₂)(X₁₋₃) where X₁₋₇ is -H or C₁-C₃ alkyl, and where n₄, X₁₋₂ and X₁₋₃ are as defined above,
-N(X₁₋₇)-SO₂-(CH₂)ₙ₄-X₁₋₈ where n₄, X₁₋₇ and X₁₋₈ are as defined above,
-N(X₁₋₇)-CO-NH-(CH₂)ₙ₅-X₁₋₈ where n₅ is 0 thru 3 and where X₁₋₇ and X₁₋₈ are as defined above,
-N(X₁₋₇)-SO₂-(CH₂)ₙ₄-N(X₁₋₂)(X₁₋₃) where n₄, X₁₋₂, X₁₋₃ and X₁₋₇ are as defined above,
-N(X₁₋₇)-CO-O-X₁₋₆ where X₁₋₆ is C₁-C₄ alkyl or -(CH₂)ₙ₄-N(X₁₋₂)(X₁₋₃) where n₄, X₁₋₂, X₁₋₃ and X₁₋₇ are as defined above,
-N(X₁₋₇)-CO-N(X₁₋₂)(X₁₋₃) where X₁₋₂, X₁₋₃ and X₁₋₇ are as defined above,
-NH-CO-CF₃, or
-N(X₁₋₇)-SO₂-N(X₁₋₂)(X₁₋₃) where X₁₋₂, X₁₋₃ and X₁₋₇ are as defined above,
R₆ is -N=, -CH=, or -N(O)=,
R₇ is -COO-R₇₋₁₁ where R₇₋₁₁ is C₁-C₆ alkyl,
-CO-N(R₇₋₃)(R₇₋₄) where R₇₋₃ and R₇₋₄ are the same or different and are -H or C₁-C₆ alkyl,
-N(R₇₋₅)(R₇₋₆) where R₇₋₅ is
   C₁-C₆ alkyl,
   -C(R₇₋₁₅)(R₇₋₁₆)-(R₇₋₁₇) where R₇₋₁₅ and R₇₋₁₆ are the same or different and are -H or C₁-C₃ alkyl and R₇₋₁₇ is C₂-C₅ alkenyl containing 1 or 2 double bonds or C₂-C₅ alkynyl containing 1 triple bond,
   -CH₂-CH₂-OH,
   -CH₂-CH₂-CH₂-OH,
   -CH(CH₃)CH₂-O-CH₃,
   -CH(CH₃)CH₂-OH,
   -CH₂-CF₃,
   -CH₂-cyclopropyl,
   -CH₂-CH₂F,
   -CH₂-CH₂-C≡N,
   -C*R₇₋₁₈-(CH₂)ₙ₁₄-C*H₂- where R₇₋₁₈ is -H or -CH₃, n₁₄ is 1 thru 5 and the carbon atoms marked with an asterisk (*) are bonded to each other to resulting in the formation of a ring, or
   -(CH₂)ₙ₁-N(R₇₋₇)(R₇₋₈) where n₁ is as defined above and R₇₋₇ and R₇₋₈ are the same or different and are -H or C₁-C₄ alkyl, or R₇₋₇ and R₇₋₈ are taken together with the attached nitrogen atom to form a heterocyclic ring selected from 1-pyrrolidinyl, 1-piperidinyl, 1-piperazinyl, N-morpholinyl and 1-aziridinyl,
and where R₇₋₆ is -H,
   C₁-C₆ alkyl,
   -C(R₇₋₁₅)(R₇₋₁₆)-(R₇₋₁₇) where R₇₋₁₅, R₇₋₁₆ and R₇₋₁₇ are as defined above,
   -CH₂-CH₂-OH,
   -CH₂-CH₂-CH₂-OH,
   -CH₂CF₃,
   -CH₂-CH₂F, or
   -CH₂-CH₂-C≡N,
   or where R₇₋₅ and R₇₋₆ are taken together with the attached nitrogen atom to form a heterocyclic ring selected from 1-pyrrolidinyl, 1-piperidinyl, 1-piperazinyl, N-morpholinyl and 1-aziridinyl, or
-(CH₂)ₙ₄-N(R₇₋₉)(R₇₋₁₀) where n₄ is as defined above and where R₇₋₉ and R₇₋₁₀ are the same or different and are -H or C₁-C₄ alkyl, and where R₇₋₉ and R₇₋₁₀ are taken together with the attached nitrogen atom to form a heterocyclic ring selected from 1-pyrrolidinyl, 1-piperidinyl, 1-piperazinyl and N-morpholinyl,
R₈ is -N= or -CR₈₋₁ = where R₈₋₁ is
   -H, -F, -Cl, -Br, -CF₃,
   -NO₂, -COCF₃,
   C₁-C₆ alkyl,
   C₁-C₃ alkylthio,
   -OH,
   -O-R₈₋₂ where R₈₋₂ is C₁-C₆ alkyl, -φ or -CO-R₈₋₃ where R₈₋₃ is C₁-C₆ alkyl or -φ,
   -NH(R₈₋₄) where R₈₋₄ is C₁-C₆ alkyl or -C(R₈₋₇)(R₈₋₈)-(R₈₋₉) where R₈₋₇ and R₈₋₈ are the same or different and are -H or C₁-C₃ alkyl and R₈₋₉ is C₂-C₅ alkenyl containing 1 or 2 double bonds or C₂-C₅ alkynyl containing 1 triple bond, or
   -NR₈₋₅-CO-R₈₋₆ where R₈₋₅ is -H or C₁-C₆ alkyl and R₈₋₆ is -H, C₁-C₆ alkyl or C₁-C₃ alkoxy;
R₉ is -N= or -CR₉₋₁ = where R₉₋₁ is
   -H, -F, -Cl, -Br,
   -NO₂, -COCF₃,
   C₁-C₆ alkyl,
   C₁-C₃ alkylthio,
   -OH,
   -O-R₉₋₂ where R₉₋₂ is C₁-C₆ alkyl, -φ, -CO-R₉₋₃ where R₉₋₃ is C₁-C₆ alkyl or -φ,
   -N(R₉₋₄)(R₉₋₅) where R₉₋₄ and R₉₋₅ are the same or different and are -H, C₁-C₆ alkyl, or -C(R₉₋₈)(R₉₋₉)-(R₉₋₁₀) where R₉₋₈ and R₉₋₉ are the same or different and are -H or C₁-C₃ alkyl and R₉₋₁₀ is C₂-C₅ alkenyl containing 1 or 2 double bonds or C₂-C₅ alkynyl containing 1 triple bond, or R₉₋₄ and R₉₋₅ are taken together with the attached nitrogen atom to form a heterocyclic ring selected from 1-pyrrolidinyl, 1-piperidinyl, 1-piperazinyl and N-morpholinyl, or
   -NR₉₋₆-CO-R₉₋₇ where R₉₋₆ is -H or C₁-C₆ alkyl and R₉₋₇ is -H, C₁-C₆ alkyl or C₁-C₃ alkoxy;
R₁₀ is -N= or -CR₁₀₋₁ = where R₁₀₋₁ is
   -H, -F, -Cl, -Br, -CF₃,
   -NO₂, -COCF₃,
   C₁-C₆ alkyl,
   C₁-C₃ alkylthio,
   -OH,
   -O-R₁₀₋₂ where R₁₀₋₂ is C₁-C₆ alkyl, -φ, -CO-R₁₀₋₃ where R₁₀₋₃ is C₁-C₆ alkyl or -φ,
   -N(R₁₀₋₄)(R₁₀₋₅) where R₁₀₋₄ and R₁₀₋₅ are the same or different and are -H, C₁-C₆ alkyl, or -C(R₁₀₋₈)(R₁₀₋₉)-(R₁₀₋₁₀) where R₁₀₋₈ and R₁₀₋₉ are the same or different and are -H or C₁-C₃ alkyl and R₁₀₋₁₀ is C₂-C₅ alkenyl containing 1 or 2 double bonds or C₂-C₅ alkynyl containing 1 triple bond, or
   -NR₁₀₋₆-CO-R₁₀₋₇ where R₁₀₋₆ is -H or C₁-C₆ alkyl and R₁₀₋₇ is -H, C₁-C₆ alkyl or C₁-C₃ alkoxy;
   with the proviso that not more than two of R₆, R₈, R₉ and R₁₀ are -N=;
and enantiomers, pharmaceutically-acceptable salts, hydrates and solvates thereof.

### DETAILED DESCRIPTION OF THE INVENTION

The substituted indoles (I) of the present invention are prepared by known methods from known starting materials.

Depending on whether the heteroatom attaching the X group to the indole is oxygen (-O-) or nitrogen (-NH-), the substituted indoles (I) are made by different procedures.

When the heteroatom is an oxygen atom, the substituted indoles (I) are prepared as set forth in CHART A. CHART A exemplifies production of the substituted indole (I) with an oxygen atom at the 6-position of the indole nitrogen, at X₂. However, it should be realized the synthetic process set forth in CHART A is equally applicable when the oxygen function is at the 5-position of the indole, at X₁. The synthetic process begins with the appropriate hydroxyaldehyde (II). The hydroxyaldehyde (II) then has the remaining portion of the desired X group added, by known means, to form X-O-, to produce the substituted aldehyde (III). The substituted aldehyde (III) is then reacted with α-azidomethylester, preferably the acetate to form the corresponding azide (IV). The reaction is performed in a polar solvent, preferably an alcohol such as methanol and cooled to about -10°. A base such as sodium methoxide in methanol is added dropwise. The reaction is warmed to 20-25° until TLC indicates the reaction is complete. The azide (IV) is transformed to the corresponding indole ester (V) by heating to reflux in a solvent such as xylene. The indole ester (V) is then hydrolyzed to the corresponding indole acid (VI). The indole acid (VI) is the coupled with the desired amine substituent (Z₁-aromatic portion) to form the substituted indole (I), see EXAMPLE 5. The desired amine substitutent (Z₁-aromatic portion) are either known to those skilled in the art (in particular see International Publication No. WO 87/01797, PREPARATION A-1 thru PREPARATION A-50) or can readily be prepared be prepared from known compounds by methods well known to those skilled in the art. The reaction of the indole acid (VI) and the desired amine substitutent (Z₁-aromatic portion) is a very well known reaction. When Z₁ is the molecular fragment (Z-I) which is a piperazine, the amine substitutent is a secondary amine and the reaction with the appropriate indole acid (VI) produces an amide. Should the indole acid be reduced to an indole alcohol (R₁ is -CH₂-), alkyl halide formed then the substituted indole (I) will be a tertiary amine. The reaction to produce tertiary amines or amides from cyclic amines such as piperazine is very well known to those skilled in the art and requires no special mention. See International Publication Nos. WO 87/01797 and WO 88/08424.

When the heteroatom attaching the X group to the indole is a nitrogen, the substituted indoles are made by the process of CHART B. The amino indoles (VII) are known to those skilled in the art or can be readily be prepared by known means from known starting materials. The amino indoles (VII) are reacted with the sulfonyl chloride, Cl-(CH₂)₂-SO₂-Cl (VIII) to produce the corresponding ethylene sulfonamide (IX). The ethylene sulfonamide (IX) is then reacted with the desired amine and powdered copper catalyst under reflux to produce the desired substituted indole (I). Similarly amino indoles (VII) can be reacted with Cl-(CH₂)₃-SO₂-Cl to produce the 3-chloropropylsulfonamides. Alkylation with an amine would produce the desired substituted indoles. Alternatively, one can react the amino or hydroxy indole with functionalized sulfonyl chlorides, which are known in the art, such as 2-(4-pyridyl)ethane sulfonyl chloride to provide the sulfonamides or sulfonates directly.

The process used for synthesizing the substituted indoles (I) where the atom attaching the X group to the indole is nitrogen does not work well when the atom is oxygen because if one starts with the hydroxy indole corresponding to the amino indole (VII), and attempts to alkylate as is done in the synthesis of compound (III), CHART A, the nitrogen atom of the indole would be competitively alkylated. This is not a problem with sulfonylation of the amino indole (VII) because the amino group on the benzene reacts preferentially over the indole nitrogen with R-SO₂- leaving group.

The amino indole (VII) can be transformed into the desired indole (I) where X₁ or X₂ is a urea (RₐR_{b}-N-CO-NH-indole-) by reacting the free amino group on the indole with an isocyanate, by known means, see EXAMPLES 18-20. Another way to produce the substituted indole (I) where X₁ or X₂ is a urea is by reaction of the free amino group in the indole with phosgene (or a phosgene equivalent such as carbonyldiimidazole) and trapping with an amine. As is apparent to those skilled in the art, isothiocyanates or thiophosgene equivalents could be used to produce the corresponding -CS- compounds. Likewise, reaction of the amino indole (VII) with X-SO₂-X followed by reaction with an amine produces sulfamides, RₐR_{b}-SO₂-NH-indole- by means known to those skilled in the art.

The substituted indoles (I) where R₁ is -CH₂- can be produced from the corresponding substituted indoles (I) where R₁ is -CO- by reaction with a reducing agent, such as lithium aluminum hydride in an ether such as tetrahydrofuran or ether. Alternatively, one can use starting materials in which R₁ is methylene rather than a carbonyl group.

It is preferred that Z₁ is (Z-I) where n₁ and n₂ are 1. It is also preferred that Z₁ is is (Z-II) where n₁ and n₂ are 1. When Z₁ is Z-II, it is preferred that Z₂₋₁ is methyl.

It is preferred that R₁ is -CO-.

It is preferred that R₆ is -N=.

It is preferred that R₈, R₉ and R₁₀ are each -CH=.

It is preferred that R₇ is is -N(R₇₋₅)(R₇₋₆) where R₇₋₅ is C₂-C₄ alkyl and R₇₋₆ is -H.

It is preferred that one of X₁ and X₂ is selected from

-N(X₁₋₇)-CO-N(X₁₋₂)(X₁₋₃) and

-N(X₁₋₇)-SO₂-N(X₁₋₂)(X₁₋₃).

It is preferred that the substituted indole (I) is selected from compounds of EXAMPLES 5,9 and 14-20. It is also preferred that the substituted indole (I) is selected from compounds of EXAMPLES 23-26, 29 and 34.

The substituted indoles (I) are amines, and as such form acid addition salts when reacted with acids of sufficient strength. Pharmaceutically acceptable salts include salts of both inorganic and organic acids. The pharmaceutically acceptable salts are preferred over the corresponding free amines since they produce compounds which are more water soluble and more crystalline. The preferred pharmaceutically acceptable salts include salts of the following acids methanesulfonic, hydrochloric, hydrobromic, sulfuric, phosphoric, nitric, benzoic, citric, tartaric, fumaric, maleic, CH₃-(CH₂)ₙ-COOH where n is 0 thru 4, HOOC-(CH₂)n-COOH where n is as defined above.

The substituted indoles (I) are useful as inhibitors of viral reverse transcriptase, an enzyme necessary for human immunodeficiency virus replication and therefore would be useful in the treatment of such diseases as AIDS.

The term human retrovirus (HRV) indicates human immunodeficiency virus type I, or strains thereof apparent to one skilled in the art, which belong to the same viral families and which create similar physiological effects in humans as various human retroviruses.

Patients to be treated would include those individuals (1) infected with one or more than one strain of a human retrovirus as determined by the presence of either measurable viral antibody or antigen in the serum and (2) having either a symptomatic AIDS defining infection such as (a) disseminated histoplasmosis, (b) isopsoriasis, (c) bronchial and pulmonary candidiasis including pneumocystic pneumonia (d) non-Hodgkin's lymphoma or (e) Kaposi's sarcoma and being less than sixty years old; or having an absolute CD4 lymphocyte count of less than 200/mm³ in the peripheral blood.

The substituted indoles (I) can be given orally. Suitable dosage forms include tablets, capsules, suspensions, solutions and elixirs. An effective amount is from about 0.1 to about 500 mg/kg/day. A typical unit dose for a 70 kg human would be from about 10 mg to about 2000 mg, preferably about 100 mg to about 1000 mg taken one to six times per day.

The exact dosage and frequency of administration depends on the particular substituted indole (I) used, the particular condition being treated, the severity of the condition being treated, the age, weight, general physical condition of the particular patient, other medication the individual may be taking as is well known to those skilled in the art and can be more accurately determined by measuring the blood level or concentration of the diaromatic substituted compounds (III), the anti-AIDS piperazines (IV) and the indoles (V) in the patient's blood and/or the patient's response to the particular condition being treated.

The substituted indoles (I) of this invention can be used in conjunction with other antiviral agents such as AZT.

The utility of the substituted indoles (I) of this invention can be determined by their ability to inhibit viral reverse transcriptase, an enzyme essential for human immunodeficiency virus replication. This enzyme has characteristics which differentiate it from other known cellular polymerases and it is a unique enzyme which is not found in uninfected cells. Viral reverse transcriptase is found in extracts from bacterial clones prepared according to the procedure described in AIDS Virus Reverse Transcriptase defined by high level expression in *Escherichia coli,* EMBO J. 6:3133-3137 (1987). Inhibition of this enzyme is determined in a cell free assay which measures the level of radioactive precursors incorporated into DNA. Extracts prepared according to the procedure of *Science,* 1125-1129 (1981) are incubated in a mixture of inhibitor, 20 mM dithiothreitol, 60 mM sodium chloride, 0.05% NP-40, 10 mM magnesium chloride, 50 mM Tris pH 8.3, 10 µM [³⁵S]-labeled deoxynucleoside-5'-triphosphate, 10 µg/ml RNA template (poly rC or poly rG) and 5 µg/ml DNA primer (oligo dG or oligo dT) for 30 minutes at 37°C. Incorporation of radio-labeled precursor is determined by spotting aliquots of the reaction mixture on DE81 paper, washing the papers to remove unincorporated precursor, drying and determining counts. The results (IC₅₀ means the concentration, in µM of drug, required to inhibit the reverse transcriptase activity to the extent of 50%) of various assay(s) are combined and reported as % inhibition and IC₅₀ (calculated).

The utility of this invention is further demonstrated by the ability of the substituted indoles (I) to inhibit HIV-induced syncytia formation in a tissue culture assay using MT-2 cells infected with HIV-1. This test is described in Quantitative Infectivity Assay for HIV-1 and -2., *Nature* 332: 469-470, 1988 as well as in AIDS RESEARCH AND HUMAN RETROVIRUSES, Vol. 4, No. 6, pages 449-455 (1988), Mary Ann Liebent, Inc., Publishers; in an article entitled "Nucleotide Dimers Suppress HIV Expression In VITRO". The results (IC₅₀ means the concentration, in µM of drug, required to inhibit syncytia formation to the extent of 50%) of various assay(s) are combined and reported as % inhibition and IC₅₀ (calculated). The known commercial compound, AZT, exhibited anti-HIV potency in this assay with 100 percent and 50 percent reduction in syncytia formation at concentrations of approximately 1µm and 0.5 µM, respectively.

The exact dosage and frequency of administration depends on the particular substituted indole (I) used, the particular condition being treated, the severity of the condition being treated, the age, weight, general physical condition of the particular patient, other medication the individual may be taking as is well known to those skilled in the art and can be more accurately determined by measuring the blood level or concentration of the substituted indoles (I) in the patient's blood and/or the patient's response to the particular condition being treated.

### DEFINITIONS AND CONVENTIONS

The definitions and explanations below are for the terms as used throughout this entire document.

### I. CONVENTIONS FOR FORMULAS AND DEFINITIONS OF VARIABLES

The chemical formulas representing various compounds or molecular fragments in the specification and claims may contain variable substituents in addition to expressly defined structural features. These variable substituents are identified by a letter or a letter followed by a numerical subscript, for example, "Z₁" or "Rᵢ" where "i" is an integer. These variable substituents are either monovalent or bivalent, that is, they represent a group attached to the formula by one or two chemical bonds. For example, a group Z₁ would represent a bivalent variable if attached to the formula CH₃-(=Z₁)H. Groups Rᵢ and Rⱼ would represent monovalent variable substituents if attached to the formula CH₃-CH₂-C(Rᵢ)(Rⱼ)-H· When chemical formulas are drawn in a linear fashion, such as those above, variable substituents contained in parentheses are bonded to the atom immediately to the left of the variable substituent enclosed in parenthesis. When two or more consecutive variable substituents are enclosed in parentheses, each of the consecutive variable substituents is bonded to the immediately preceding atom to the left which is not enclosed in parentheses. Thus, in the formula above, both Rᵢ and Rⱼ are bonded to the preceding carbon atom. Also, for any molecule with an established system of carbon atom numbering, such as steroids, these carbon atoms are designated as Cᵢ, where "i" is the integer corresponding to the carbon atom number. For example, C₆ represents the 6 position or carbon atom number in the steroid nucleus as traditionally designated by those skilled in the art of steroid chemistry. Likewise the term "R₆" represents a variable substituent (either monovalent or bivalent) at the C₆ position.

Chemical formulas or portions thereof drawn in a linear fashion represent atoms in a linear chain. The symbol "-" in general represents a bond between two atoms in the chain. Thus CH₃-O-CH₂-CH(Rᵢ)-CH₃ represents a 2-substituted-1-methoxypropane compound. In a similar fashion, the symbol "=" represents a double bond, e.g., CH₂=C(Rᵢ)-O-CH₃, and the symbol "≡" represents a triple bond, e.g., HC≡C-CH(Rᵢ)-CH₂-CH₃. Carbonyl groups are represented in either one of two ways: -CO- or -C(=O)-, with the former being preferred for simplicity.

Chemical formulas of cyclic (ring) compounds or molecular fragments can be represented in a linear fashion. Thus, the compound 4-chloro-2-methylpyridine can be represented in linear fashion by N*=C(CH₃)CH=CCl-CH=C*H with the convention that the atoms marked with an asterisk (*) are bonded to each other resulting in the formation of a ring. Likewise, the cyclic molecular fragment, 4-(ethyl)-1-piperazinyl can be represented by -N*-(CH₂)₂-N(C₂H₅)-CH₂-C*H₂.

A rigid cyclic (ring) structure for any compounds herein defines an orientation with respect to the plane of the ring for substituents attached to each carbon atom of the rigid cyclic compound. For saturated compounds which have two substituents attached to a carbon atom which is part of a cyclic system, -C(X₁)(X₂)- the two substituents may be in either an axial or equatorial position relative to the ring and may change between axial/equatorial. However, the position of the two substituents relative to the ring and each other remains fixed. While either substituent at times may lie in the plane of the ring (equatorial) rather than above or below the plane (axial), one substituent is always above the other. In chemical structural formulas depicting such compounds, a substituent (X₁) which is "below" another substituent (X₂) will be identified as being in the alpha (α) configuration and is identified by a broken, dashed or dotted line attachment to the carbon atom, i.e., by the symbol "---" or "...". The corresponding substituent attached "above" (X₂) the other (X₁) is identified as being in the beta (β) configuration and is indicated by an unbroken line attachment to the carbon atom.

When a variable substituent is bivalent, the valences may be taken together or separately or both in the definition of the variable. For example, a variable Rᵢ attached to a carbon atom as -C(=Rᵢ)- might be bivalent and be defined as oxo or keto (thus forming a carbonyl group (-CO-) or as two separately attached monovalent variable substituents α-Rᵢ₋ⱼ and β-Rᵢ₋ₖ. When a bivalent variable, Rᵢ, is defined to consist of two monovalent variable substituents, the convention used to define the bivalent variable is of the form "α-Rᵢ₋ⱼ:β-Rᵢ₋ₖ" or some variant thereof. In such a case both α-Rᵢ₋ⱼ and β-Rᵢ₋ₖ are attached to the carbon atom to give -C(α-Rᵢ₋ⱼ)(β-Rᵢ₋ₖ)-. For example, when the bivalent variable R₆, -C(=R₆)- is defined to consist of two monovalent variable substituents, the two monovalent variable substituents are α-R₆₋₁:β-R₆₋₂, .... α-R₆₋₉:β-R₆₋₁₀, etc, giving -C(α-R₆₋₁)(β-R₆₋₂)-, .... -C(α-R₆₋₉)(β-R₆₋₁₀)-, etc. Likewise, for the bivalent variable R₁₁, -C(=R₁₁)-, two monovalent variable substituents are α-R₁₁₋₁:β-R₁₁₋₂. For a ring substituent for which separate α and β orientations do not exist (e.g. due to the presence of a carbon carbon double bond in the ring), and for a substituent bonded to a carbon atom which is not part of a ring the above convention is still used, but the α and β designations are omitted.

Just as a bivalent variable may be defined as two separate monovalent variable substituents, two separate monovalent variable substituents may be defined to be taken together to form a bivalent variable. For example, in the formula -C₁(Rᵢ)H-C₂(Rⱼ)H- (C₁ and C₂ define arbitrarily a first and second carbon atom, respectively) Rᵢ and Rⱼ may be defined to be taken together to form (1) a second bond between C₁ and C₂ or (2) a bivalent group such as oxa (-O-) and the formula thereby describes an epoxide. When Rᵢ and Rⱼ are taken together to form a more complex entity, such as the group -X-Y-, then the orientation of the entity is such that C₁ in the above formula is bonded to X and C₂ is bonded to Y. Thus, by convention the designation "... Rᵢ and Rⱼ are taken together to form -CH₂-CH₂-O-CO- ..." means a lactone in which the carbonyl is bonded to C₂. However, when designated "... Rⱼ and Rᵢ are taken together to form -CO-O-CH₂-CH₂-the convention means a lactone in which the carbonyl is bonded to C₁.

The carbon atom content of variable substituents is indicated in one of two ways. The first method uses a prefix to the entire name of the variable such as "C₁-C₄", where both "1" and "4" are integers representing the minimum and maximum number of carbon atoms in the variable. The prefix is separated from the variable by a space. For example, "C₁-C₄ alkyl" represents alkyl of 1 through 4 carbon atoms, (including isomeric forms thereof unless an express indication to the contrary is given). Whenever this single prefix is given, the prefix indicates the entire carbon atom content of the variable being defined. Thus C₂-C₄ alkoxycarbonyl describes a group CH₃-(CH₂)ₙ-O-CO- where n is zero, one or two. By the second method the carbon atom content of only each portion of the definition is indicated separately by enclosing the "Cᵢ-Cⱼ" designation in parentheses and placing it immediately (no intervening space) before the portion of the definition being defined. By this optional convention (C₁-C₃)alkoxycarbonyl has the same meaning as C₂-C₄ alkoxycarbonyl because the "C₁-C₃" refers only to the carbon atom content of the alkoxy group. Similarly while both C₂-C₆ alkoxyalkyl and (C₁-C₃)alkoxy(C₁-C₃)alkyl define alkoxyalkyl groups containing from 2 to 6 carbon atoms, the two definitions differ since the former definition allows either the alkoxy or alkyl portion alone to contain 4 or 5 carbon atoms while the latter definition limits either of these groups to 3 carbon atoms.

When the claims contain a fairly complex (cyclic) substituent, at the end of the phrase naming/designating that particular substituent will be a notation in (parentheses) which will correspond to the same name/designation in one of the CHARTS which will also set forth the chemical structural formula of that particular substituent.

### II. DEFINITIONS

All temperatures are in degrees Centigrade.

TLC refers to thin-layer chromatography.

THF refers to tetrahydrofuran.

DMF refers to dimethylformamide.

EDC refers to 1-ethyl-3-(dimethylaminopropyl)carbodiimide.

Saline refers to an aqueous saturated sodium chloride solution.

IR refers to infrared spectroscopy.

NMR refers to nuclear (proton) magnetic resonance spectroscopy, chemical shifts are reported in ppm (δ) downfield from tetramethylsilane.

-φ refers to phenyl (C₆H₅).

MS refers to mass spectrometry expressed as m/e or mass/charge unit. [M + H]⁺ refers to the positive ion of a parent plus a hydrogen atom. EI refers to electron impact. CI refers to chemical ionization. FAB refers to fast atom bombardment.

Ether refers to diethyl ether.

Alcohol refers to ethyl alcohol.

Pharmaceutically acceptable refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

Pharmaceutically acceptable anion salts include mesylate, chloride, sulfate, phosphate, nitrate, citrate, CH₃-(CH₂)ₙ₁-COO⁻¹ where n₁ is 0 thru 4, ⁻¹OOC-(CH₂)n₁-COO⁻¹ where n is as defined above, ⁻¹OOC-CH=CH-COO⁻¹, φ-COO⁻¹.

When solvent pairs are used, the ratios of solvents used are volume/volume (v/v).

When the solubility of a solid in a solvent is used the ratio of the solid to the solvent is weight/volume (wt/v).

Pyridinyl refers to the pyridyl radical as defined by IUPAC nomenclature. For example 2-pyridyl refers to the pyridine ring substituted in the 2-position.

The compounds of this invention are named (when possible) by the following method: first the [aryl/heteroaryl] moiety, next the aryl/heteroaryl portion and last the linker (Z). However, a few were named by other methods for simplicity and convenience. The names of the radicals within each group follow IUPAC convention.

### EXAMPLES

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

### PREPARATION 1 1-[1,1-Dimethylethoxycarbonyl]-4-[3-(1-methylethylamino)-2-pyridinyl]piperazine

1-[1,1-Dimethylethoxycarbonyl]-4-[3-amino-2-pyridinyl]piperazine (International Publication 88/08424, 2.0 g) is dissolved in 35 ml of methanol and acetone (0.48 g) is added. The reaction is cooled to 0° and acetic acid (to pH 4.0) is added. The reaction is stirred 15 min at 0° and then sodium cyanoborohydride (0.50 g) is added. The reaction is allowed to warm slowly to 20-25° and followed by TLC until completion. Additional acetic acid, sodium cyanoborohydride and acetone are sometimes necessary to force the reaction to completion. The reaction is diluted with chloroform (100 ml), washed with saturated aqueous sodium bicarbonate (50 ml), saline (75 ml), dried over anhydrous sodium sulfate and concentrated in vacuo. Purification by flash column chromatography (75 g silica gel, 4:1 hexane/ethyl acetate) affords the title compound, NMR (300 MHz, CDCl₃) 7.67, 6.91, 4.15, 3.57, 3.00, 1.48 and 1.23 δ.

### PREPARATION 2 1-[3-(1-Methylethylamino)-2-pyridinyl]piperazine (Amine)

1-[1,1-Dimethylethoxycarbonyl]-4-[3-(1-methylethylamino)-2-pyridinyl]piperazine (PREPARATION 1) is dissolved in methylene chloride (56 ml) and cooled to 0°. Then trifluoroacetic acid is added dropwise. The reaction is warmed to 20-25° and additional trifluoroacetic acid is added (26.6 g). When the reaction is complete by TLC, it is poured into 200 ml of water and ice, basified to pH 12 with 2N aqueous sodium hydroxide, and extracted with 10 % tetrahydrofuran/chloroform (2l) followed by 10 % methanol/chloroform (1l). The organic layers are dried over anhydrous sodium sulfate, concentrated in vacuo, and used without further purification, NMR (300 MHz, CDCl₃) 7.65, 6.85, 6.76, 4.16, 3.50, 2.98 and 1.20 δ.

### PREPARATION 3 1-(1,1-Dimethylethoxy)carbonyl-4-methylaminopiperidine

Methylamine hydrochloride (2.36 g) is dissolved in methanol (50 ml) and potassium hydroxide pellets (0.60 g) and N-(1,1-di-methylethoxycarbonyl)-4-piperidone are added. Sodium cyanoborohydride (0.69 g) in methanol (5 ml) is added and the mixture is stirred 2 hrs. Potassium hydroxide pellets (1.96 g) are added to the mixture which is stirred 1 hr and acidified to pH 2 with 6M hydrochloric acid and concentrated. The mixture is diluted with water (50 ml) and extracted with ether (3 x 80 ml) which is discarded. The aqueous layer is basified to pH 11 with potassium hydroxide pellets, saturated with sodium chloride and extracted with ether (6 x 80 ml). The combined organic extracts are dried with magnesium sulfate and concentrated to afford an oil which is chromatographed on silica gel with a methanol/chloroform gradient (5-30%). Fractions are pooled on the basis of TLC (R_{f} = 0.13, 20% methanol/chloroform) to give the title product, NMR (CDCl₃) 4.04, 2.79, 2.54, 2.46, 2.33, 1.88, 1.46, and 1.26 δ.

### PREPARATION 4 1-(1,1-Dimethylethoxy)carbonyl)-4-(N-methyl-N-(3-nitro-2-pyridinyl)amino)piperidine

Anhydrous potassium carbonate (2.71 g) and 2-chloro-3-nitropyridine (0.93 g) are added to a solution of 1-((1,1-dimethylethoxy)carbonyl)-4-methylaninopiperidine (PREPARATION 3, 1.40 g) in acetonitrile (50 ml). The mixture is stirred 21 hours at 20-25° and additional 2-chloro-3-nitropyridine (100 mg) and acetonitrile (5 ml) are added. The mixture is stirred 2.8 days, concentrated and dissolved in methylene chloride (175 ml) and water (50 ml). The phases are separated and the organic phase is extracted with water (2 x 50 ml) and saline (40 ml) and dried over sodium sulfate. Concentration under reduced pressure affords an oil which is chromatographed on silica gel (120 g) eluting with 10% ethyl acetate/hexane. Fractions with R_{f} = 0.29 by TLC (silica gel, 25% ethylacetate/hexane) are pooled and concentrated to give the title compound, NMR (CDCl₃) 8.29, 8.11, 6.68, 4.62, 4.26, 2.85, 2.67 and 1.48 δ.

### PREPARATION 5 4-(N-methyl-N-(3-nitro-2-pyridinyl)amino)piperidine

Trifluoroacetic acid (13.0 ml) is added to a solution of 1-((1,1-dimethylethoxy)carbonyl)-4-(N-methyl-N-(3-nitro-2-pyridinyl)amino)piperidine (PREPARATION 4) in methylene chloride (100 ml) with cooling to -78°. The mixture is warmed to 20-25°, stirred 17 hrs, cooled to 0° and basified to pH 12 with 5% sodium hydroxide. The phases are separated and the aqueous phase is extracted with methylene chloride (2 x 50 ml). The combined organic phases are dried over sodium sulfate and concentrated to give the title compound, mp 115.5-117°.

### PREPARATION 6 1-(1,1-Dimethylethoxyl)carbonyl)-4-(N-methyl-N-(3-amino-2-pyridinyl)amino)piperidine

1-(1,1-Dimethylethoxy)carbonyl)-4-(N-methyl-N-(3-nitro-2-pyridinyl)alnino)piperidine (PREPARATION 4, 7.49g) is dissolved in ethanol (135 ml) and palladium on carbon (10%, 0.65 g) is added. The reaction is hydrogenated at 275 kPa (40 psi) for 18 hours and filtered through a pad of celites and concentrated under reduced pressure, NMR (d-methanol) 7.54, 6.99, 6.80, 3.90, 2.61, 2.56, 1.63, 1.47 and 1.35 δ.

### PREPARATION 7 1-(1,1-Dimethylethoxyl)carbonyl)-4-(N-methyl-N-(3-(1-methylethylamino-2-pyridinyl)amino)piperidine

1-(1,1-Dimethylethoxy)carbonyl)-4-(N-methyl-N-(3-amino-2-pyridinyl)amino)piperidine (PREPARATION 6) is dissolved in ethanol (137 ml) and cooled to 0°. Glacial acetic acid (39.3 ml) and acetone (2.22 ml) are added. After 15 min sodium cyarboborohydride (4.99 g) is added and the reaction is warmed to 20-25°. Since reaction did not appear complete by TLC (ethyl acetate/hexane, 75/25), a total of 3.5 equivalents of acetone and acetic acid are added over a 24 hour period. The reaction is poured into water, extracted with chloroform, dried over sodium sulfate and concentrated under reduced pressure. Flash column chromatography eluting with ethyl acetate/hexane (20/80), pooling the appropriate fractions and concentrating gives the title compound, NMR (d-methanol) 7.47, 6.90, 3.92, 3.51, 2.71, 2.51, 1.35, 1.61 and 1.11 δ.

### PREPARATION 8 4-(N-Methyl-N-(3-(1-methylethylamino)-2-pyridinyl)amino)-piperidine

Following the procedure of PREPARATION 5, but substituting 1-(1,1-dimethylethoxy)carbonyl)-4-(N-methyl-N-(3-(1-Methylethylamino-2-pyridinyl)amino)piperidine (PREPARATION 7) for 1-(1,1-dimethylethoxy)carbonyl)-4-(N-methyl-N-(3-nitro-2-pyridinyl)amino)piperidine and starting the reaction at 0°, the title compound is obtained, NMR (d-methanol) 7.47, 6.89, 3.50, 3.05, 2.93, 2.52, 2.42, 1.62, 1.45 and 1.11 δ.

### PREPARATION 9 1-[5-Nitroindolyl-2-carbonyl]-4-[N-methyl-N-(3-(1-methylethylamino)-2-pyridinyl)amino]piperidine

A mixture of 5-nitroindole-2-carboxylic acid (2.39 g) and 1-(3-dimethylaminopropyl)-3-ethylcarbodiimide hydrochloride (2.33 g) in dry THF (600 ml) are stirred with a mechanical stirrer under nitrogen at 20-25° for 45 min. To this is added a solution of 4-(N-methyl-N-(3-(1-methylethylamino)-2-pyridinyl)amino)-piperidine (PREPARATION 8, 2.88 g) in dry THF (20 ml) and the mixture is stirred for 1.8 days. The supernatant liquid is removed, concentrated to dryness, and diluted with methylene chloride (75 ml) and water (25 ml). The layers are separated and the organic phase is washed with saturated aqueous sodium bicarbonate (30 ml) and saline and dried over sodium sulfate. Removal of solvent under reduced pressure gives a solid which is chromatographed on silica gel (70-230 mesh, 400 g), eluting with a gradient of methanol/chloroform (0.5/99.5 - 5/95). Pooling of the appropriate fractions, having an R_{f} of about 0.34 by TLC (methanol/chloroform 5/95) and removal of solvent under reduced pressure gives the title compound, mp 204-206°.

### PREPARATION 10 1-[5-Aminoindolyl-2-carbonyl]-4-[N-methyl-N-(3-(1-methylethylamino)-2-pyridinyl)amino]-piperidine (VII)

Palladium on carbon (10%, 200 mg) is added to a mixture of 1-[5-nitroindolyl-2-carbonyl]-4-[N-methyl-N-(3-(1-methylethylamino)-2-pyridinyl)amino]piperidine(PREPARATION 9,400mg) in DMF/methanol under nitrogen. The mixture is put under a hydrogen atmosphere (balloon), stirred for 5 hrs, and the catalyst is filtered off through a pad of diatomaceous earth. The filtrate is concentrated under reduced pressure to give the title compound.

### PREPARATION 11 1-Methyl 4-methoxy-α-azidocinnamate

p-Methoxybenzaldehyde (5.0 g) and methyl azidoacetate (16.9 g) are dissolved in 125 ml of methanol and cooled to -10° (ice-acetone bath). Then sodium methoxide (7.93 g, 25% in methanol) is added dropwise such that the temperature does not rise above -5°. After 2 hr the cooling bath is removed and the reaction is warmed to 20-25° while being monitored by TLC. When no starting material remained, the reaction is diluted with ether and saturated ammonium chloride. After extracting with ether the organic layers are washed with ammonium chloride, saline, dried over anhydrous sodium sulfate and concentrated under reduced pressure. The concentrate is purified by flash column chromatography eluting with ethyl acetate/hexane (1/99) to ethyl acetate/hexane (10/90). The appropriate fractions are pooled and concentrated to give the title compound, NMR (300 MHz, CDCl₃) 7.80-7.76, 6.92-6.87, 3.88 and 3.79 δ.

### PREPARATION 12 Methyl 6-Methoxyindole-2-carboxylate

Toluene (185 ml) is added to methyl 4-methoxy-α-azidocinnamate (PREPARATION 11, 7.73 g) and the reaction is brought to reflux and maintained at reflux for 3 hr. Then the reaction is concentrated under reduced pressure and triturated with hexane. The solids are filtered and dried under reduced pressure to give the title indole, HRMS Calcd. for C₁₁H₁₁NO₃: 205.0739, found: 205.0736; NMR (300 MHz, CDCl₃) 8.75, 7.47, 7.11, 6.76-6.73, 3.86, and 3.79 δ.

### PREPARATION 13 6-Methoxyindole-2-carboxylic acid (I)

Methyl 6-methoxyindole-2-carboxylate (PREPARATION 12, 5.71 g) is dissolved in 70 ml of dioxane and 7 ml of water and 1.87 g of crushed potassium hydroxide are added. The reaction is heated to 50° and stirred 1.5 hr. The reaction mixture is acidified to pH 4-5 and extracted several times with methanol/chloroform (10/90). The organic layers are combined and dried over anhydrous magnesium sulfate and concentrated under reduced pressure to give the title acid, NMR (300 MHz, d4-CD₃OD) 7.47, 7.08, 6.90, 6.72 and 3.82 δ.

### PREPARATION 14 Methyl 4-nitro-α-azidocinnamate

Following the general procedure of PREPARATION 11 and making non-critical variations but starting with p-nitrobenzaldehyde (10 g) and methyl azidoacetate (30.4 g), the title compound is obtained, NMR 8.34, 8.07, 7.02, and 4.07 δ.

### PREPARATION 15 Methyl 6-nitroindole-2-carboxylate

Following the general procedure of PREPARATION 12 and making non-critical variations but starting with methyl 4-nitro-α-azidocinnamate (PREPARATION 14, 6.75 g), the title compound is obtained, NMR (300 MHz, CD₃OD) 8.30, 7.87, 7.72, 7.18, and 3.86 δ.

### PREPARATION 16 6-Nitroindole-2-carboxylic acid (I)

Following the general procedure of PREPARATION 13 and making non-critical variations but starting with methyl 6-nitroindole-2-carboxylate (PREPARATION 15), the title compound is obtained.

### PREPARATION 17 1-[6-Nitroindoyl-2-carbonyl]-4-[3-(1-methylethylamino)-2-pyridinyl]piperazine

Following the general procedure of PREPARATION 9 and making non-critical variations but starting with 6-nitroindole-2-carboxylic acid (PREPARATION 16) and 1-[3-(1-methylethylamino)-2-pyridinyl]piperazine (PREPARATION 2), the title compound is obtained.

### PREPARATION 18 1-[1,1-Dimethylethoxycarbonyl]-4-[3-(1,1-dimethylprop-2-ynylamino)-2-pyridinyl]piperazine

To a mixture of 1-[1,1-dimethylethoxycarbonyl]-4-[3-amino-2-pyridinyl]piperazine (International Publication No. WO 88/08424, 5.40 g), cuprous chloride (1.00 g), copper powder (1.00 g), and dry dimethylformamide (25 ml) under nitrogen at 0° is added a solution of 3-chloro-3-methyl-1-butyne (2.00 g) in dry dimethylformamide (5 ml) in 4 portions over 15 min. The resulting mixture is then stirred at 20-25° for 16 hrs, concentrated, and diluted with methylene chloride (75 ml) and water (20 ml). The layers are separated and the aqueous phase is extracted with methylene chloride (25 ml). The combined organic phase is washed with saline (20 ml), dried over anhydrous magnesium sulfate, and concentrated under reduced pressure to give a residue which is chromatographed on silica gel (70-230 mesh, 500 g), eluting with a gradient of ethyl acetate/hexane (10/90-20/80). Pooling of fractions giving an R_{f} = 0.31 (ethyl acetate/hexane, 25/75) and removal of solvent under reduced pressure gives the title compound, NMR (CDCl₃) 7.75, 7.47, 6.94, 4.61, 3.56, 3.00, 2.39, 1.64 and 1.48 δ.

### PREPARATION 19 1-[1,1-Dimethylethoxycarbonyl]-4-[3-(1,1-dimethylpropylamino)-2-pyridinyl]piperazine

To a solution of 1-[1,1-dimethylethoxycarbonyl]-4-[3-(1,1-dimethylprop-2-ynylamino)-2-pyridinyl]piperazine (PREPARATION 18, 1.00 g) in absolute ethanol (20 ml) under nitrogen is added wet Raney Nickel (650 mg). The mixture is put under a hydrogen atmosphere at 275 kPa (40 psi) (Parr) for 20 hr, filtered through a pad of celite, and concentrated. The residue is then taken up in methylene chloride (40 ml), washed with saline (10 ml), dried over sodium sulfate, and concentrated under reduced pressure to give the title compound, NMR (CDCl₃) 7.64, 7.03, 6.85, 4.56, 3.56, 2.97, 1.71, 1.48, 1.33 and 0.88.

### PREPARATION 20 1-[5-Nitroindole-2-carbonyl]-4-[3-(1,1-dimethylpropylamino)-2-pyridinyl]piperazine

To a solution of 1-[1,1-dimethylethoxycarbonyl]-4-[3-(1,1-dimethylpropylamino)-2-pyridinyl]piperazine (PREPARATION 19, 4.99 g) in methylene chloride (75 ml) under nitrogen at 0° is added trifluoroacetic acid (14.3 ml) over 1 min. The resulting mixture is warmed to 20-25°, stirred for 15 hrs, and then added to a solution of sodium hydroxide (7.44 g) in water (175 ml) at 0°. The layers are separated, the aqueous phase is extracted with methylene chloride (3 x 70ml), and the combined organic layer is washed with saline (60 ml), dried over sodium sulfate, and concentrated under reduced pressure to give the unprotected intermediate (R_{f} = 0.14, TLC, methanol/chloroform, 10/90). In a flame-dried flask under nitrogen, 5-nitroindole-2-carboxylic acid (2.13 g) and 1,1'-carbonyldiimidazole (1.75 g) are dissolved in dry tetrahydrofuran and stirred for 3 hrs at 20-25°. Then a solution of the intermediate (2.57 g) in dry tetrahydrofuran (3ml) is added and the resulting mixture is stirred for 1.8 days. The reaction mixture is filtered to give the title compound, NMR (dimethylsulfoxide-d₆) 12.41, 8.65, 8.09, 7.58, 7.16, 7.11, 6.91, 4.57, 3.93, 3.00, 1.71, 1.31 and 0.84. The filtrate is concentrated and is triturated with cold methanol to give an additional amount of the title compound.

### PREPARATION 21 1-[1,1-Dimethylethoxycarbonyl]-4-[N-methyl-N-(3-(1,1-dimethylprop-2-ynylamino)-2-pyridinyl)amino]piperidine

Following the general procedure of PREPARATION 18, but substituting 1-[1,1-dimethylethoxycarbonyl]-4-[N-methyl-N-(3-amino-2-pyridinyl)amino]piperidine(PREPARATION 6) for 1-[1,1-dimethylethoxycarbonyl]-4-[3-amino-2-pyridinyl]piperazine and working up the reaction after 2 hrs, the title compound is obtained, NMR (CDCl₃) 7.79, 7.51, 6.95, 4.92, 4.05, 3.19, 2.75, 2.61, 2.38, 1.75, 1.62, 1.49 and 1.45 δ.

### PREPARATION 22 1-[1,1-Dimethylethoxycarbonyl]-4-[N-methyl-N-(3-(1,1-dimethylpropylamino)-2-pyridinyl)amino]piperidine

Following the general procedure of PREPARATION 19, but substituting 1-[1,1-dimethylethoxycarbonyl]-4-[N-methyl-N-(3-(1,1-dimethylprop-2-ynylamino)-2-pyridinyl)amino]piperidine (PREPARATION 21) for 1-[1,1-dimethylethoxycarbonyl]-4-[3-(1,1-dimethylprop-2-ynylamino)-2-pyridinyl]piperazine, the crude product is obtained. This is then chromatographed on silica gel (70-230 mesh, 250 g), eluting with a gradient of ethyl acetate/hexane (10/90-35/65), and the appropriate fractions are pooled and concentrated to give the title compound, NMR (CDCl₃) 7.67, 7.04, 6.87, 4.87, 4.04, 3.20, 2.76, 2.61, 1.71, 1.50, 1.45, 1.31 and 0.86 δ.

### PREPARATION 23 4-[N-Methyl-N-(3-(1,1-dimethylpropylamino)-2-pyridinyl)amino]piperidine

Following the general procedure of PREPARATION 5, but substituting 1-[1,1-dimethylethoxycarbonyl]-4-[N-methyl-N-(3-(1,1-dimethylpropylamino)-2-pyridinyl)amino]piperidine (PREPARATION 22) for 1-[1,1-dimethylethoxycarbonyl]-4-[N-methyl-N-(3-nitro-2-pyridinyl)amino]piperidine, starting the reaction at 0°, and working up the reaction after 1.5 hr, the title compound is obtained, NMR (CDCl₃) 7.66, 7.02, 6.84, 4.86, 3.39, 3.13, 2.63, 1.81-1.52, 1.31 and 0.87 δ.

### PREPARATION 24 1-[5-Nitroindole-2-carbonyl]-4-[N-methyl-N-(3-(1,1-dimethylpropylamino)-2-pyridinyl)amino]piperidine

Under nitrogen 5-nitroindole-2-carboxylic acid (977 mg) and 1,1'-carbonyldiimidazole (807 mg) are dissolved in dry tetrahydrofuran (25 ml) and the mixture is stirred at 20-25° for 2 hr. A solution of 4-[N-methyl-N-(3-(1,1-dimethylpropylamino)-2-pyridinyl)amino]piperidine (PREPARATION 23, 1.31 g) in dry tetrahydrofuran is then added, and the resulting mixture is stirred for 18 hr and filtered. The filtrate is concentrated, diluted with methylene chloride (75 ml), washed with water and saline (20 ml), dried over sodium sulfate, and concentrated under reduced pressure to give a solid which is chromatographed on silica gel (70-230 mesh, 200 g), eluting with a gradient of ethyl acetate/hexane (50/50-90/10). Pooling of fractions giving an R_{f} = 0.08 by TLC (ethyl acetate/hexane, 50/50) and removal of solvent under reduced pressure gives the title compound, mp 205.5-207.5°.

### PREPARATION 25 1-[5-Aminoindole-2-carbonyl]-4-[N-methyl-N-(3-(1,1-dimethylpropylamino)-2-pyridinyl)amino]piperidine

Following the general procedure of PREPARATION 42, but substituting 1-[5-nitroindole-2-carbonyl]-4-[N-methyl-N-(3-(1,1-dimethylpropylamino)-2-pyridinyl)amino]piperidine (PREPARATION 24) for 1-[5-nitroindole-2-carbonyl]-4-[3-(1,1-dimethylpropylamino)-2-pyridinyl]piperazine and using dimethylformamide/methanol (25/75) as solvent, the title compound is obtained, NMR (CDCl₃) 9.95, 7.69, 7.20, 7.05, 6.88, 6.71, 6.55, 4.91, 4.64, 3.86, 3.40, 3.11, 2.62, 1.91, 1.63, 1.31 and 0.86 δ.

### PREPARATION 26 4-(2-(2-Methoxyethoxy)ethoxy)benzaldehyde

Following the general procedure of EXAMPLE 1, and making non-critical variations but substituting 1-bromo-2-(2-methoxyethoxy)ethane (Aldrich) for 2-[2-(2-chloroethoxy)ethoxy]ethanol, the title compound is obtained, mp 44-47°; NMR (300 MHz, CDCl₃) 9.78, 7.72, 6.92, 4.12, 3.79, 3.63, 3.48, 3.29 δ.

### PREPARATION 27 Methyl 4-(2-(2-methoxyethoxy)ethoxy)-α-azidocinnamate

Following the general procedure of EXAMPLE 2, and making non-critical variations but substituting 4-(2-(2-methoxyethoxy)ethoxy)benzaldehyde (PREPARATION 26) for 4-[(2-(2-hydroxyethoxy)ethoxy)ethoxy]benzaldehyde, the title compound is obtained, NMR (300 MHz, CD₃OD) 7.68, 6.85, 6.78, 4.05, 3.78, 3.72, 3.61, 3.48, 3.28 δ.

### PREPARATION 28 Methyl 6-(2-(2-methoxyethoxy)ethoxy)indole-2-carboxylate

Following the general procedure of EXAMPLE 3, and making non-critical variations but substituting 4-(2-(2-methoxyethoxy)ethoxy)-α-azidocinnamate (PREPARATION 27) for 4-[(2-(2-hydroxyethoxy)ethoxy)ethoxy]-α-azidocinnamate, the title compound is obtained, NMR (300 MHz, CD₃OD) 7.59, 7.20, 7.02, 6.87, 4.25, 4.00, 3.96, 3.80, 3.67 and 3.47.

### PREPARATION 29 6-(2-(2-Methoxyethoxy)ethoxy)indole-2-carboxylic acid

Following the general procedure of EXAMPLE 4, and making non-critical variations but substituting methyl 6-(2-(2-methoxyethoxy)ethoxy)indole-2-carboxylate (PREPARATION 28) for methyl 6-[(2-(2-hydroxyethoxy)ethoxy)ethoxy]indole-2-carboxylate, the title compound is obtained, mp 99-102°.

### PREPARATION 30 Ethyl 5-[(3-chloropropyl)sulfonamido]indole-2-carboxylate

Ethyl 5-aminoindole-2-carboxylate (2.0 g) and pyridine (0.82 ml) are dissolved in 15 ml of methylene chloride and 10 ml of THF. Then the reaction is cooled to 0° and 3-chloropropanesulfonyl chloride (1.25 ml) is added and the reaction is allowed to slowly warm to 20-25° and stir for 16 hr. Then the reaction is diluted with chloroform, washed with saturated aqueous sodium bicarbonate and saline. The organic layers are dried over anhydrous sodium sulfate and concentrated under reduced pressure. The product is dissolved in ethyl acetate and filtered through a plug of silica gel and crystallized from methanol, mp 195-196°.

### PREPARATION 31 Ethyl 5-[(3-(piperidin-1-yl)propyl)sulfonamido]indole-2-carboxylate

Ethyl 5-[(3-chloropropyl)sulfonamido]indole-2-carboxylate (PREPARATION 30, 2.3 g) is dissolved in acetonitrile (10 ml) and piperidine (27 ml). Solid sodium iodide (1.0 g) is added and the reaction is stirred overnight at 20-25°. The reaction is diluted with methylene chloride, washed with saturated aqueous sodium bicarbonate, dried over anhydrous sodium sulfate and concentrated under reduced pressure. Purification by flash column chromatography, eluting with a gradient of 5% methanol/methylene chloride to 10 % methanol/methylene chloride, pooling and concentration of the appropriate fractions gives the title compound, NMR (300 MHz, d₄-CD₃OD) 7.45, 7.32, 7.12, 7.03, 4.28, 2.96, 2.28, 1.18, 1.42, 1.33 and 1.30.

### PREPARATION 32 5-[(3-(Piperidin-1-yl)propyl)sulfonamido]indole-2-carboxylic acid

Ethyl 5-[(3-(piperidin-1-yl)propyl)sulfonamido]indole-2-carboxylate (PREPARATION 31, 1.63 g) is dissolved in 10 ml of dioxane and 5 ml of water. Then 0.59 g of solid potassium hydroxide pellets are added and the reaction is stirred at 20-25° for 24 hr. Then 8.91 ml of 1N hydrochloric acid is added after diluting the reaction with 100 ml of water. The mixture is extracted with n-butanol (3 X 50 ml) and the organic layer is separated and evaporated under reduced pressure to provide the title compound, NMR (300 MHz, d₆-DMSO) 7.37, 7.27, 7.00, 6.90, 2.91, 2.25, 1.75, 1.31, (peak at 4.3 ppm obscured by DMSO).

### PREPARATION 33 1-[5-(3-Chloropropyl)sulfonamidoindolyl-2-carbonyl]-4-[3-(1-methylethylamino)-2-pyridinyl]piperazine

1-[5-Aminoindolyl-2-carbonyl]-4-[3-(1-methylethylamino)-2-pyridinyl]piperazine (EXAMPLE 7, 2.0 g) is dissolved in 17.6 ml of pyridine and cooled to 0°. Then 3-chloropropanesulfonyl chloride (1.28 ml) is added and the reaction is allowed to warm to 20-25° and stirred 8 hr. The reaction is diluted with chloroform, washed with saturated aqueous sodium bicarbonate, water, saline, dried over anhydrous sodium sulfate and concentrated under reduced pressure. Purification by flash column chromatography eluting with a gradient of 50/50 ethyl acetate/hexane to 100% ethyl acetate, pooling and concentrating of the appropriate fractions gives the title compound, mp 219-221°.

### PREPARATION 34 1-Benzyl-4-[3-(1-cyano-1-methylethylamino)pyridyl]piperazine

The 1-[5-Aminoindolyl-2-carbonyl]-4-[3-(1-methylethylamino)-2-pyridinyl]piperazine (EXAMPLE 7, 11.68 g, 43.51 mmol) is dissolved in 130 ml methanol and 130 ml acetic acid. Acetone (25.6 ml, 348.1 mmol) and trimethylsilylcyanide (46.4 ml, 348.1 mmol) are added and the mixture is stirred twenty hours. The solution is poured into cold aqueous sodium hydroxide solution, stirring vigorously, to give a basic solution. The aqueous solution is then extracted with ethyl acetate, the organic extract is washed with saline, dried over sodium sulfate and evaporated to dryness to give an oily residue of 12.6 g. Chromatography on a 400 g silica gel column with a step gradient of ethyl acetate/hexane (2/3 to 3/2, v/v), pooling and concentrating the appropriate fractions gives the title compound, NMR (300 MHz, CD₃OD) 7.65 (m, 1 H), 7.29-7.17 (m, 6 H), 6.96-6.92 (m, 1 H), 3.51 (s, 2 H), 2.98 (t, 4 H, J=5 Hz), 2.55 (bs, 4 H), 1.14 (s, 6 H).

### PREPARATION 35 1-Benzyl-4-[3-(1,1-dimethylethylamino)pyridyl]piperazine

Methyllithium (61 ml, 85 mmol) as a 1.4M solution in diethyl ether is added to 30 ml tetrahydrofuran and cooled to -78°. 1-Benzyl-4-[3-(1-cyano-1-methylethylamino)pyridyl]-piperazine (PREPARATION 34, 7.14 g, 21.28 mmol), dissolved in 30 ml cooled tetrahydrofuran, is cannulated into the methyllithium solution, rinsing in with 10 ml THF. The reaction is stirred at -78° and allowed to warm to 20-25° overnight. The reaction is cautiously quenched with water, then extracted from water with methylene chloride. The extract is dried over sodium sulfate and concentrated. The concentrate is chromatographed on a 500 g silica gel column, eluting with ethyl acetate/hexane (2/3, v/v), the appropriate fractions are pooled and concentrated to give the title compound, NMR (300 MHz, CD₃OD) 7.44 (m, 1 H), 7.43-7.07 (m, 6 H), 6.84-6.80 (m, 1 H), 3.49 (s, 2 H), 2.92 (t, 4 H, J=5 Hz), 2.52 (bs, 4 H), 1.28 (s, 9H).

### PREPARATION 36 1-[3-(1,1-Dimethylethylamino)pyridyl]piperazine

1-Benzyl-4-[3-(1,1-dimethylethylamino)pyridyl]piperazine (PREPARATION 35, 4.25 g, 13.10 mmol) is dissolved in 100 ml ethanol. Palladium (10% on carbon, 1.0 g) is added and the solution hydrogenated at 275 kPa (40 psi) hydrogen gas. The mixture is filtered and concentrated to give the title compound, NMR (300 MHz, CD₃OD) 7.53 (dd, 1 H, J=1.5, 5 Hz), 7.19 (dd, 1 H, J=1.5, 8 Hz), 6.93 (dd, 1 H, J=5, 8 Hz), 2.95 (s, 8 H), 1.39 (s, 9 H).

### PREPARATION 37 Ethyl 5-(4-methylpiperazin-1-ylcarbonylamino)indole-2-carboxylate

Following the general procedure of EXAMPLE 20 and making non-critical variations but substituting methyl 5-aminoindole-2-carboxylate for 1-[5-aminoindolyl-2-carbonyl]-4-[3-(1-methylethylamino)-2-pyridinyl]piperazine, the title compound is obtained, NMR(CDCl₃) 7.62, 7.48, 7.22, 7.18, 7.08, 6.97, 6.75, 4.36, 3.49, 2.39, 2.28, 1.38 δ.

### PREPARATION 38 5-(4-Methylpiperazin-1-ylcarbonylamino)indole-2-carboxylic acid

Following the general procedure of PREPARATION 32 and making non-critical variations but substituting ethyl 5-(4-methylpiperazin-1-ylcarbonylamino)indole-2-carboxylate (PREPARATION 37) for ethyl 5-[(3-(piperadin-1-yl)propyl)sulfonamido]indole-2-carboxylate, the title compound is obtained, NMR(CD₃OD) 7.70, 7.46, 7.32, 7.10, 7.05, 6.87, 3.54, 2.48, 2.32 δ.

### EXAMPLE 1 4-[2-(2-(2-Hydroxyethoxy)ethoxy)ethoxy]benzaldehyde (III)

4-Hydroxybenzaldehyde (II, 10.0g) is dissolved in DMF (198 ml) and cooled to 0°, sodium hydride (60% in mineral oil, 3.44 g) is added, after bubbling ceased (15-20 min), 2-[2-(2-chloroethoxy)ethoxy]ethanol (17.8 ml) and sodium iodide (0.61 g) is added. The reaction is stirred at 20-25° for 0.5 hours and heated to reflux for 78 hours. The reaction is poured into water and washed with ethyl acetate (3 x). The phases are separated and the organic layer is washed with water, saline and concentrated under reduced pressure with heat. The material is purified with flash column chromatography, eluting with ethyl acetate/hexane (50/50) to (75/25). The appropriate fractions are pooled and concentrated to give the title compound, MS theory: 254.1154. Found: 254.1163.

### EXAMPLE 2 Methyl 4-[(2-(2-hydroxyethoxy)ethoxy)ethoxy]-α-azidocinnamate (IV)

4-[2-(2-(2-Hydroxyethoxy)ethoxy)ethoxy]benzaldehyde (III, EXAMPLE 1, 17.3 g) and α-azidomethyl acetate (31.3 g) are dissolved in methanol (136 ml) and cooled to -10°. Sodium methoxide in methanol (25 %, 77.7 ml) is added dropwise. The reaction is warmed to 20-25° until TLC indicates the reaction is complete. The reaction mixture is poured into water and extracted with ethyl acetate. The phases are separated and the organic phase is concentrated and purified by flash column chromatography to give the title compound.

### EXAMPLE 3 Methyl 6-[(2-(2-hydroxyethoxy)ethoxy)ethoxy]indole-2-carboxylate (V)

Methyl 4-[(2-(2-hydroxyethoxy)ethoxy)ethoxy]-α-azidocinnamate (IV, EXAMPLE 2, 3.0 g) is dissolved in xylene (86 ml) and quickly heated to reflux. TLC indicates disappearance of starting material, the reaction is cooled to 20-25° and the material is purified by flash column chromatography eluting with ethyl acetate/hexane (75/25). The appropriate fractions are pooled and concentrated to give the title compound, MS theory: 323.1369. Found: 323.1366.

### EXAMPLE 4 6-[(2-(2-hydroxyethoxy)ethoxy)ethoxy]indole-2-carboxylic acid (VI)

Methyl 6-[(2-(2-hydroxyethoxy)ethoxy)ethoxy]indole-2-carboxylate (V, EXAMPLE 3, 2.9 g) and potassium hydroxide (1.0 g) are dissolved in dioxane (25 ml) and water (5 ml). The reaction is warmed to 50° for 3 hours. The reaction is neutralized by the addition of hydrochloric acid (1N, 17 ml), extracted with THF/chloroform (50/50, 3 x), washed with saline and concentrated under reduced pressure with heat. The material is purified by flash column chromatography eluting with methanol/chloroform/acetic acid (5/94/1). The appropriate fractions are pooled and concentrated to give the title compound, MS theory: 309.1212. Found: 309.1208.

### EXAMPLE 5 1-[6-(2-(2-Hydroxyethoxy)ethoxy)ethoxyindolyl-2-carbonyl]-4-[3-(1-methylethylamino)-2-pyridinyl]piperazine (I)

6-[(2-(2-Hydroxyethoxy)ethoxy)ethoxy]indole-2-carboxylic acid (VI, EXAMPLE 4, 0.20 g) and 3-(1-methylethylamino)-2-pyridinyl]piperazine (0.157 g) is dissolved in THF (4 ml) and 1-(ethyl)-3-(dimethylaminopropyl)carbodiimide (0.16 g) is added. The reaction is stirred at 20-25° for 2.75 hours, poured into chloroform, washed with saturated sodium bicarbonate and saline. The material is concentrated under reduced pressure with heat and purified by flash column chromatography eluting with methanol/chloroform (5/95). The appropriate fractions are pooled and concentrated to give the title compound, mp 157-159°.

### EXAMPLE 6 1-[5-Nitroindolyl-2-carbonyl]-4-[3-(1-methylethylamino)-2-pyridinyl]piperazine

1-(Ethyl)-3-(dimethylaminopropyl)carbodiimide (0.45 g) is added to a solution of 1-[3-(1-methylethyl)amino)-2-pyridinyl]piperazine (PREPARATION 2, 0.43 g) and 5-nitroindole-2-carboxylic acid (0.86 g) in THF (5 ml). The reaction is stirred at 20-25° for 3 hr, then it is dissolved in chloroform (50 ml) and extracted with saturated aqueous sodium bicarbonate, saline, dried over anhydrous sodium sulfate and concentrated under reduced pressure. Purification by flash column chromatography (200 g silica) eluting with ethyl acetate/hexane (50/50), the appropriate fractions are pooled and concentrated to give the title compound, mp 153-154°.

### EXAMPLE 7 1-[5-Aminoindolyl-2-carbonyl]-4-[3-(1-methylethylamino)-2-pyridinyl]-piperazine (VII)

1-[5-Nitroindolyl-2-carbonyl]-4-[3-(1-methylethylamino)-2-pyridinyl]piperazine (EXAMPLE 6, 1.0 g) is dissolved in ethanol (60 ml) and THF, (60 ml) and palladium on carbon (10%, 0.15 g) is added. The reaction is hydrogenated at 275 kPa (40 psi) for 14 hr, then filtered through celite and concentrated under reduced pressure. Purification by flash chromatography, eluting with ethyl acetate/hexane (50/50 → 75/25), pooling and concentrating the appropriate fractions gives the title compound, mp 212-214°.

### EXAMPLE 8 1-[5-Ethylenesulfonamidoindolyl-2-carbonyl]-4-[3-(1-methylethylamino)-2-pyridinyl]piperazine (IX)

1-[(5-Aminoindolyl-2-carbonyl]-4-[3-(1-methylethylamino)-2-pyridinyl]piperazine (V, EXAMPLE 7, 250 mg) is dissolved in pyridine (2 ml) and cooled to 0°. Then 2-chloroethanesulfonyl chloride (VIII, 0.138 ml) is added dropwise. The reaction is warmed to 20-25° and stirred for 3 hours. The reaction mixture is poured into aqueous saturated sodium bicarbonate and extracted with methylene chloride. The phases are separated and the organic layer is dried over anhydrous sodium sulfate and concentrated under reduced pressure with heat. Purification by flash column chromatography eluting with ethyl acetate/hexane (80/20). The appropriate fractions are pooled and concentrated to give the title compound, MS. m/e: 468, 453, 249, 219, 1922, 177 and 164.

### EXAMPLE 9 1-[5-(2-(1-Pyrrolidino)ethyl)sulfonamidoindolyl-2-carbonyl]-4-[3-(1-methylethylamino)-2-pyridinyl]piperazine (I)

1-[5-Ethylenesulfonamidoindolyl-2-carbonyl]-4-[3-(1-methylethylamino)-2-pyridinyl]-piperazine (IX, EXAMPLE 8, 173 mg), pyrrolidine (0.10 ml) and a catalytic amount of copper powder are refluxed together in xylene (5 ml) for 3.5 hours. The reaction is filtered through a pad of celite washing with ethyl acetate and methanol. After concentration under reduced pressure with heat, the product is purified by flash column chromatography eluting with methanol/chloroform (10/90). The appropriate fractions are pooled and concentrated to give the title compound which is crystallized from aqueous ethanol, mp 145-146°.

### EXAMPLE 10 4-[2-(2-hydroxyethoxy)ethoxy]benzaldehyde (III)

Following the general procedure of EXAMPLE 1, and making non-critical variations but substituting 2-[2-chloroethoxy]ethanol for 2-[2-(2-chloroethoxy)ethoxy]ethanol, the title compound is obtained, NMR (CDCl₃) 7.82, 7.01, 4.21, 3.89, 3.76 and 3.66 δ.

### EXAMPLE 11 Methyl 4-[2-(2-hydroxyethoxy)ethoxy]-α-azidocinnamate (IV)

Following the general procedure of EXAMPLE 2, and making non-critical variations but but substituting 4-[2-(2-hydroxyethoxy)ethoxy]benzaldehyde (III, EXAMPLE 10) for 4-[2-(2-(2-hydroxyethoxy)ethoxy)ethoxy]benzaldehyde (EXAMPLE I), the title compound is obtained, NMR (CDCl₃) 7.76, 6.92, 6.86, 4.15, 3.88, 3.86, 3.75 and 3.66 δ.

### EXAMPLE 12 Methyl 6-[(2-hydroxyethoxy)ethoxy]indole-2-carboxylate (V)

Following the general procedure of EXAMPLE 3, and making non-critical variations but substituting methyl 4-[2-(2-hydroxyethoxy)ethoxy]-2-azidocinnamate (IV, EXAMPLE 11) for methyl 4-[(2-(2-hydroxyethoxy)ethoxy)ethoxy]-α-azidociannamate, the title compound is obtained, NMR (CDCl₃) 7.53, 7.14, 6.83, 4.14, 3.91, 3.87 and 3.68 δ.

### EXAMPLE 13 6-[(2-Hydroxyethoxy)ethoxy]indole-2-carboxylic acid (VI)

Following the general procedure of EXAMPLE 4, and making non-critical variations but substituting methyl 6-[(2-hydroxyethoxy)ethoxy]indole-2-carboxylate (V, EXAMPLE 12) the title compound is obtained, NMR (d-methanol) 7.38, 6.95, 6.80, 6.63, 4.03, 3.74, 3.57, 3.51 and 3.17 δ.

### EXAMPLE 14 1-[6-(2-Hydroxyethoxy)ethoxyindolyl-2-carbonyl]-4-[3-(1-methylethylamino)-2-pyridinyl]piperazine (I)

Following the general procedure of EXAMPLE 5, and making non-critical variations but substituting 6-[(2-hydroxyethoxy)ethoxy]indole-2-carboxylic acid (VI, EXAMPLE 13) for 6-[(2-(2-hydroxyethoxy)ethoxy)ethoxy]indole-2-carboxylic acid, the title compound is obtained, mp 74-75°.

### EXAMPLE 15 1-[5-(2-(1-Piperidinyl)ethyl)sulfonamidoindolyl-2-carbonyl]-4-[3-(1-methylethylamino)-2-pyridinyl]piperazine (I)

Following the general procedure of EXAMPLE 6, and making non-critical variations but substituting piperidine for pyrrolidine, the title compound is obtained, mp 150°.

### EXAMPLE 16 1-[5-(2-(1-Morpholinyl)ethyl)sulfonamidoindolyl-2-carbonyl]-4-[3-(1-methylethylamino)-2-pyridinyl]piperazine (I)

Following the general procedure of EXAMPLE 9, and making non-critical variations but substituting morpholine for pyrrolidine, the title compound is obtained, mp 135°.

### EXAMPLE 17 1-[6-(2-(2-Hydroxyethoxy)ethoxy)ethoxyindolyl-2-carbonyl]-4-[N-methyl-N-(3-(1-methylethylamino)-2-pyridinyl)amino]piperidine (I)

Following the general procedure of EXAMPLE 5, and making non-critical variations but substituting 4-(N-methyl-N-(3-(1-methylethylamino)-2-pyridinyl)amino)piperidine (PREPARATION 8) for 3-(1-methylethylamino)-2-pyridinyl]piperazine, the title compound is obtained, MS theory 540.3186, found 540.3149.

### EXAMPLE 18 1-[5-(3-Methylureido)-indolyl-2-carbonyl]-4-[N-methyl-N-(3-(1-methylethylamino)-2-pyridinyl)amino]-piperidine (I)

Methyl isocyanate (47 µl) is added to a solution of 1-[5-aminoindolyl-2-carbonyl]-4-[N-methyl-N-(3-(1-methylethylamino)-2-pyridinyl)amino]-piperidine (VII, PREPARATION 10, 310 mg) in dry methylene chloride (3 ml) of at 0°. The mixture is stirred for 30 min at 0°, during which a precipitate forms, and then at 20-25° for 18 hrs. The reaction mixture is filtered and the precipitate rinsed with cold methylene chloride to give the title compound, NMR (methanol-d₄) 7.60, 7.32, 7.10, 6.98, 6.69, 4.46, 3.57, 3.37, 3.08, 2.75, 2.60, 1.82, 1.59 and 1.19 δ.

### EXAMPLE 19 1-[5(2-(4-Pyridyl)-ethanesulfonamido)-indolyl-2-carbonyl]-4-[N-methyl-N-(3-(1-methylethylamino)-2-pyridinyl)amino]-piperidine (I)

A mixture of 4-pyridine ethanesulfonic acid (2.32 g), phosphorus pentachloride (2.86 g) and phosphorus oxychloride (9.4 ml) is heated to 60°, stirred for 1.5 hrs, cooled to 20-25°, and diluted with carbon tetrachloride (10 ml). The mixture is filtered and the precipitate is washed thoroughly with carbon tetrachloride, acetonitrile, and ether and dried under reduced pressure to give the sulfonyl chloride as a solid. To a solution of 1-[5-aminoindolyl-2-carbonyl]-4-[N-methyl-N-(3-(1-methylethylamino)-2-pyridinyl)amino]-piperidine (VII, PREPARATION 10, 400 mg) in dry methylene chloride (5 ml) under nitrogen is added this sulfonyl chloride (262 mg) and triethylamine (275 µl). The mixture is stirred at 20-25° for 24 hrs, during which an additional sulfonyl chloride (25 mg) and triethylamine (14 µl) are added, and then diluted with water (3 ml). The layers are separated and the organic phase is washed with saline (5 ml), dried over sodium sulfate, and concentrated under reduced pressure to give a solid which is chromatographed on silica gel (70-230 mesh, 45 g), eluting with a gradient of methanol/chloroform (2.5/97.5 - 7/93). Pooling of the appropriate fractions, having an R_{f} of about 0.43 by TLC (methanol/chloroform, 10/90) and removal of solvent under reduced pressure gives the title compound, NMR (CDCl₃) 10.51, 8.62, 8.41, 7.71, 7.57, 7.35, 7.13, 6.98, 6.95, 6.85, 6.69, 4.62, 4.50, 3.55, 3.47, 3.40-2.90, 2.63, 1.92, 1.63 and 1.21 δ.

### EXAMPLE 20 1-[5-(4-Methylpiperazin-1-ylcarbonylamino)-indolyl-2-carbonyl]-4-[3-(1-methylethylamino)-2-pyridinyl)]piperazine (I)

Carbonyldiimidazole (0.086 g) is dissolved in dry THF (5 ml). 1-[5-Aminoindolyl-2-carbonyl]-4-[3-(1-methylethylamino)-2-pyridinyl]piperazine (VII, EXAMPLE 7, 0.19 g) is dissolved in dry THF (4 ml) and added to the carbonyldiimidazole solution over 2.4 hr. Upon the complete addition, N-methylpiperazine (55 µl) is added. The reaction mixture is allowed to stir overnight at 20-25°. The reaction is then concentrated and the concentrate is dissolved in chloroform (15 ml) and washed with water and saline, dried over sodium sulfate, concentrated and dried under reduced pressure. The mixture is chromatographed over silica gel (20 ml) packed in chloroform eluting with 80 ml of chloroform, 100 ml each of 1%, 1.5%, 2%, 2.5%, 3%, 4%, 5%, 6%, 7%, 8% and 10% and 150 ml of 15% methanol/chloroform. Fractions having an Rf value of about 0.28 in methanol/chloroform (10/90) are collected and concentrated to give a solid. The solid is recrystallized from methanol/chloroform, adding ethyl ether after crystals start to form to give the title compound, mp 200°.

### EXAMPLE 21 1-[6-Aminoindolyl-2-carbonyl]-4-[3-(1-methylethylamino)-2-pyridinyl]piperazine (VII)

Following the general procedure of EXAMPLE 7 and making non-critical variations but starting with 1-[6-nitroindolyl-2-carbonyl]-4-[3-(1-methylethylamino)-2-pyridinyl]piperazine (PREPARATION 21, 0.32 g), the title compound is obtained, mp 158° (decomp).

### EXAMPLE 22 1-[5-(2-(4-pyridyl)ethanesulfonamido)indole-2-carbonyl]-4-[3-(1-methylethylamino)-2-pyridinyl]piperazine (I)

Following the general procedure of EXAMPLE 19, and making non-critical variations but substituting 1-[5-aminoindole-2-carbonyl]-4-[3-(1-methylethylamino)-2-pyridinyl]piperazine (EXAMPLE 7) for 1-[5-aminoindole-2-carbonyl]-4-[N-methyl-N-(3-(1-methylethylamino)-2-pyridinyl)amino]piperidine, the title compound is obtained, NMR (CDCl₃) 10.67, 8.72, 8.41, 7.68, 7.56, 7.36, 7.13, 6.97, 6.92, 6.84, 6.72, 4.20, 4.07, 3.55, 3.28, 3.16, 3.08 and 1.25 δ.

### EXAMPLE 23 1-[5-(2-Dimethylaminoethanesulfonamido)indole-2-carbonyl]-4-[N-methyl-N-(3-(1-methylethylamino)-2-pyridinyl)amino]piperidine (I)

To a solution of 1-[5-aminoindole-2-carbonyl]-4-[N-methyl-N-(3-(1-methylethylamino)-2-pyridinyl)amino]piperidine (VII, PREPARATION 10, 400 mg) in dry methylene chloride (5 ml) under nitrogen at 0° is added triethylamine (275 µl) and 2-(dimethylamino)ethanesulfonyl chloride hydrochloride (225 mg). The mixture is stirred at 0° for 1 hr and at 20-25° for 23 hrs, during which additional sulfonyl chloride (20 mg) and triethylamine (14 µl) are added. The mixture is then diluted with 4 ml of water, the layers are separated, and the organic phase is washed with saline, dried over sodium sulfate and concentrated under reduced pressure to give a solid which is chromatographed on silica gel (70-230 mesh, 46 g), eluting with a gradient of methanol/chloroform (2.5/97.5 - 6/94). Pooling of fractions having an R_{f} of 0.36 by TLC (methanol/chloroform, 10/90) and removal of solvent under reduced pressure gives an impure solid. Purification of this solid on two 2000 µ preparative silica gel plates, eluting with methanol/chloroform (10/90) and extracting the appropriate band, gives the title compound, NMR (CDCl₃) 10.17, 7.71, 7.55, 7.35, 7.11, 6.94, 6.84, 6.69, 4.63, 4.50, 3.56, 3.45, 3.18, 3.30-3.00, 2.84, 2.63, 2.25, 1.93, 1.65 and 1.22 δ.

### EXAMPLE 24 1-[5-(2-Dimethylaminoethanesulfonamido)indole-2-carbonyl]-4-[3-(1-methylethylamino)-2-pyridinyl]piperazine (I)

Following the general procedure of EXAMPLE 23, and making non-critical variations but substituting 1-[5-aminoindole-2-carbonyl]-4-[3-(1-methylethylamino)-2-pyridinyl]piperazine (EXAMPLE 7, 400 mg) for 1-[5-aminoindole-2-carbonyl]-4-[N-methyl-N-(3-(1-methylethylamino)-2-pyridinyl)amino]piperidine, the title compound is obtained, mp 185-188° (decomp).

### PREPARATION 39 1-[5-(2-Phthalimidoethanesulfonamido)indole-2-carbonyl]-4-[N-methyl-N-(3-(1-methylethylamino)-2-pyridinyl)amino]piperidine (I)

To a solution of 1-[5-aminoindole-2-carbonyl]-4-[N-methyl-N-(3-(1-methylethylamino)-2-pyridinyl)amino]piperidine (PREPARATION 10, 606 mg) in dry methylene chloride (5 ml) under nitrogen is added pyridine (127 µl, 1.05 equivalents) and 2-phthalimidoethanesulfonyl chloride (400 mg). The mixture is stirred at 20-25° for 3 days and then diluted with methylene chloride (40 ml) and water (20 ml). The layers are separated and the organic phase is washed with saline (15 ml), dried over sodium sulfate, and concentrated under reduced pressure to give a residue which is then chromatographed on silica gel (230-400 mesh, 85g, 63 kPa (8 psi)), eluting with a gradient of methanol/chloroform (1/99-2.5/97.5). The appropriate fractions (R_{f} = 0.33, TLC, methanol/chloroform, 5/95) are pooled and concentrated to give the title compound, NMR (CDCl₃) 10.33, 7.80, 7.76-7.63, 7.35, 7.22, 6.95, 6.85, 6.69, 4.65, 4.51, 4.13, 3.60-3.40, 3.40-2.90, 2.64, 1.94, 1.65 and 1.22 δ.

### EXAMPLE 25 1-[5-(2-Aminoethanesulfonamido)indole-2-carbonyl]-4-[N-methyl-N-(3-(1-methylethylamino)-2-pyridinyl)amino]piperidine (I)

To a solution of 1-[5-(2-phthalimidoethanesulfonamido)indole-2-carbonyl]-4-[N-methyl-N-(3-(1-methylethylamino)-2-pyridinyl)amino]piperidine (PREPARATION 39, 675 mg) in 95% ethanol (10 ml) under nitrogen is added hydrazine monohydrate (53 µl). The mixture is stirred at 70-75° for 20 hrs during which additional hydrazine monohydrate (10 µl) is added, concentrated to remove ethanol, diluted with water (10 ml), acidified to pH 2 with 1M hydrochloric acid and stirred for 10 min. The mixture is then filtered, the filtrate is adjusted to pH 10-11, and the resulting precipitate is isolated by filtration to give the title compound, NMR (CDCl₃) 9.86, 7.70, 7.56, 7.29, 7.10, 6.94, 6.84, 6.68, 4.60, 4.50, 3.56, 3.44, 3.40-2.80, 2.62, 1.93, 1.65 and 1.22 δ. The basic filtrate is extracted with methylene chloride (2 x 15 ml) which is then washed with saline (10 ml), dried over sodium sulfate, and concentrated under reduced pressure to give an additional amount of the title compound.

### PREPARATION 40 1-[5-(2-Phthalimidoethanesulfonamido)indole-2-carbonyl]-4-[3-(1-methylethylamino)-2-pyridinyl]piperazine (I)

Following the general procedure of PREPARATION 39, and making non-critical variations but substituting 1-[5-aminoindole-2-carbonyl]-4-[3-(1-methylethylamino)-2-pyridinyl]piperazine (EXAMPLE 7, 553 mg) for 1-[5-aminoindole-2-carbonyl]-4-[N-methyl-N-(3-(1-methylethylamino)-2-pyridinyl)amino]piperidine and using 2 equivalents of pyridine instead of 1.05 equivalents, the title compound is obtained, NMR (CDCl₃) 10.30, 7.78, 7.66, 7.36, 7.23, 6.95, 6.86, 6.73, 4.25-4.02, 3.57, 3.44, 3.18 and 1.26 δ.

### EXAMPLE 26 1-[5-(2-Aminoethanesulfonamido)indole-2-carbonyl]-4-[3-(1-methylethylamino)-2-pyridinyl]piperazine (I)

Following the general procedure of EXAMPLE 25, and making non-critical variations but substituting-[5-(2-phthalimidoethanesulfonamido)indole-2-carbonyl]-4-[3-(1-methylethylamino)-2-pyridinyl]piperazine (PREPARATION 40, 605 mg) for 1-[5-(2-(phthalimidoethanesulfonamido)indole-2-carbonyl]-4-[N-methyl-N-(3-(1-methylethylamino)-2-pyridinyl)amino]piperidine,thetitlecompound is obtained, NMR (CDCl₃) 10.62, 7.68, 7.55, 7.25, 7.07, 6.93, 6.84, 6.69, 4.20, 4.04, 3.90-3.50, 3.14 and 1.25 δ.

### EXAMPLE 27 1-[5-(4-Methyl-1-piperazinosulfonylamino)indole-2-carbonyl]-4-[N-methyl-N-(3-(1-methylethylamino)-2-pyridinyl)amino]piperidine (I)

To a solution of 1-methylpiperazine (1.11 ml) in dry methylene chloride (20 ml) at 0° under argon is added sulfuryl chloride (1.6 ml) dropwise. The mixture is warmed to 20-25°, stirred for 1.5 hrs, and concentrated to give the crude sulfamoyl chloride intermediate. This intermediate (187 mg) is added to a solution of 1-[5-aminoindole-2-carbonyl]-4-[N-methyl-N-(3-(1-methylethylamino)-2-pyridinyl)amino]- piperidine (PREPARATION 10, 162 mg) in pyridine (1.5 ml) under nitrogen, and the resulting mixture is stirred at 20-25° for 20 hrs, diluted with water (25 ml), and extracted with methylene chloride (4x25 ml). The organic phase is then washed with aqueous sodium bicarbonate solution (30 ml) and saline (50 ml), dried over sodium sulfate, and concentrated under reduced pressure to give a solid which is chromatographed on four 2000 µ preparative silica gel plates, eluting with methanol/chloroform (5/95) twice. Extraction of the extract of the appropriate band gives the title compound, NMR (CDCl₃) 9.94, 7.71, 7.54, 7.30, 7.13, 6.95, 6.84, 6.69, 4.64, 4.50, 3.56, 3.45, 3.25, 2.63, 2.36, 2.23, 1.95, 1.65 and 1.22 δ.

### EXAMPLE 28 1-[5-(Dimethylaminosulfonylamino)indole-2-carbonyl]-4-[N-methyl-N-(3-(1-methylethylamino)-2-pyridinyl)amino]piperidine (I)

To a solution of 1-[5-aminoindole-2-carbonyl]-4-[N-methyl-N-(3-(1-methylethylamino)-2-pyridinyl)amino]piperidine (PREPARATION 10, 91 mg) in pyridine (0.5 ml) under nitrogen is added N,N-dimethylsulfamoyl chloride. The mixture is stirred at 20-25° for 16 hr and then diluted with methylene chloride (35 ml) and 1M hydrochloric acid (20 ml). The layers are separated and the organic layer is washed with 1M hydrochloric acid (20 ml) and saline, dried over sodium sulfate, and concentrated to give a solid film which is then chromatographed on two 2000 µ preparative silica gel plates, eluting with methanol/chloroform (5/95). The desired band is extracted and concentrated to give the title compound, NMR (CDCl₃) 10.34, 7.72, 7.64, 7.56, 7.37, 7.16, 6.95, 6.84, 6.70, 4.65, 4.50, 3.56, 3.46, 3.40-3.00, 2.80, 2.63, 1.93, 1.64 and 1.22 δ.

### EXAMPLE 29 1-[5-(4-Methyl-1-piperazinosulfonylamino)indole-2-carbonyl]-4-[3-(1-methylethylamino)-2-pyridinyl]piperazine (I)

To a solution of 1-methylpiperazine (1.11 ml) in dry methylene chloride (10 ml) at 0° under argon is added freshly distilled sulfuryl chloride (1.60 ml) dropwise. The mixture is warmed to 20-25° and stirred for 2.25 hrs, after which a dark brown gum is removed from the mixture. The remaining reaction mixture is concentrated under reduced pressure to give the sulfamoyl chloride intermediate (NMR (pyridine-d₅) 3.56, 2.77 and 2.40 δ). This intermediate (115 mg) is added to a solution of 1-[5-aminoindole-2-carbonyl]-4-[3-(1-methylethylamino)-2-pyridinyl]piperazine (EXAMPLE 7, 200 mg) and pyridine (94 µl) in dry methylene chloride (3 ml) under nitrogen. The mixture is stirred at 20-25° for 6 days during which an additional amount of the sulfamoyl chloride (50 mg) and pyridine (40 µl) is added, concentrated under reduced pressure, and diluted with methylene chloride (30 ml) and water (10 ml). The layers are separated, the aqueous phase is extracted with methylene chloride (2 x 25 ml), and the combined organic layers are washed with saline (20 ml), dried over sodium sulfate, and concentrated under reduced pressure. The resulting solid is chromatographed on silica gel (230-400 mesh, 35 g, 35 kPa (5 psi)), eluting with a gradient of methanol/chloroform (4/96-7/93). Pooling of the appropriate fractions (R_{f} = 0.25, TLC, methanol/chloroform, 10/90) and concentrating gives the title compound, NMR (CDCl₃) 10.45, 8.00, 7.70, 7.55, 7.29, 7.12, 6.93, 6.85, 6.72, 4.21, 4.10, 3.57, 3.23, 3.18, 2.36, 2.22 and 1.26.

### PREPARATION 41 1-[5-(4-Benzyl-1-piperazinosulfonylamino)indole-2-carbonyl]-4-[3-(1-methylethylamino)-2-pyridinyl]piperazine (I)

Following the general procedure of EXAMPLE 37, and making non-critical variations but substituting 1-benzylpiperazine for 1-methylpiperazine, the title compound is obtained, mp 173-176°.

### EXAMPLE 30 1-[5-(1-piperazinosulfonylamino)indole-2-carbonyl]-4-[3-(1-methylethylamino)-2-pyridinyl]piperazine (I)

Palladium on carbon (10%, 300 mg) is added to a solution of 1-[5-(4-benzylpiperazinosulfonylamino)indole-2-carbonyl]-4-[3-(1-methylethylamino)-2-pyridinyl]piperazine (PREPARATION 41, 450 mg) in methanol (50 ml). The mixture is stirred under a hydrogen atmosphere (balloon) for 4.5 hrs, under nitrogen for 17 hrs, again under hydrogen for 8 hrs and nitrogen for 17 hrs, and then under hydrogen for an additional 3 hrs. The mixture is filtered through a pad of celite and the filtrate concentrated under reduced pressure to give a solid film which is then chromatographed on silica gel (230-400 mesh, 37 g, 35-42 kPa (5-6 psi)), eluting with a gradient of methanol/chloroform (5/95-10/90). Pooling of the appropriate fractions (R_{f} = 0.18, TLC, methanol/chloroform, 10/90) and concentrating gives the title compound, NMR (methanol-d₄) 7.56, 7.39, 7.18, 6.98, 6.82, 4.02, 3.63, 3.16, 3.09, 2.71 and 1.24.

### EXAMPLE 31 1-[5-(Morpholinosulfonylamino)indole-2-carbonyl]-4-[3-(1-methylethylamino)-2-pyridinyl]piperazine (I)

Following the general procedure of EXAMPLE 29, and making non-critical variations but substituting morpholine (543 µl) for 1-methylpiperazine, the crude product is obtained. This residue is chromatographed on silica gel (230-400 mesh, 100 g, 42-63 kPa (6-8 psi)), eluting with a gradient of ethyl acetate/hexane (35/65-100/0). Pooling of the appropriate fractions (R_{f} = 0.16, TLC, ethyl acetate/hexane, 2 x 50/50), concentrating under reduced pressure, and triturating with methanol gives the title compound, mp 210-211.5°.

### PREPARATION 42 1-[5-Aminoindole-2-carbonyl]-4-[3-(1,1-dimethylpropylamino)-2-pyridinyl]piperazine

To a solution of 1-[5-nitroindole-2-carbonyl]-4-[3-(1,1-dimethylpropylamino)-2-pyridinyl]piperazine (PREPARATION 20, 1.00 g) in dimethylformamide/methanol (200 ml, 50/50) under nitrogen is added palladium on carbon (10 %, 250 mg). The mixture is put under hydrogen (balloon) for 4 hr and nitrogen for 17 hr, filtered through a pad of celite, and concentrated under reduced pressure to give the title compound, NMR (CDCl₃) 9.25, 7.66, 7.24, 7.06, 6.88, 6.76, 6.63, 4.58, 4.05, 3.35, 3.14, 1.73, 1.35 and 0.91.

### EXAMPLE 32 1-[5-(4-Methylpiperazinosulfonylamino)indole-2-carbonyl]-4-[3-(1,1-dimethylpropylamino)-2-pyridinyl]piperazine (I)

Following the general procedure of EXAMPLE 35, and making non-critical variations but substituting 1-[5-aminoindole-2-carbonyl]-4-[3-(1,1-dimethylpropylamino)-2-pyridinyl]piperazine (PREPARATION 42) for 1-[5-aminoindole-2-carbonyl]-4-[3-(1-methylethylamino)-2-pyridinyl]-piperazine, the title compound is obtained, mp 202-205° (decomp).

### PREPARATION 43 1-[5-(Methanesulfonamido)indole-2-carbonyl]-4-[3-(1,1-dimethylpropylamino)-2-pyridinyl]piperazine

To a solution of 1-[5-aminoindole-2-carbonyl]-4-[3-(1,1-dimethylpropylamino)-2-pyridinyl]piperazine (PREPARATION 42, 300 mg) in dry methylene chloride (6 ml) under nitrogen is added pyridine (119 µl) and methanesulfonyl chloride (57 µl), The mixture is stirred at 20-25° for 24 hr and then diluted with methylene chloride (20 ml) and water (8 ml). The layers are separated and the organic phase is washed with saline (8 ml), dried over sodium sulfate, and concentrated to give a solid which is then chromatographed on silica gel (230-400 mesh, 34 g, 35-42 kPa (5-6 psi)), eluting with methanol/chloroform (2.5/97.5). Pooling of the appropriate fractions, concentration to a solid, and recrystallization from chloroform gives the title compound, mp 232-233° (decomp).

### EXAMPLE 33 1-[5-Methanesulfonamidoindole-2-carbonyl]-4-[N-methyl-N-(3-(1,1-dimethylpropylamino)-2-pyridinyl)amino]piperidine

Following the general procedure of PREPARATION 43, but substituting 1-[5-aminoindole-2-carbonyl]-4-[N-methyl-N-(3-(1,1-dimethylpropylamino)-2-pyridinyl)amino]piperidine (PREPARATION 25) for 1-[5-aminoindole-2-carbonyl]-4-[3-(1,1-dimethylpropylamino)-2-pyridinyl]piperazine and eliminating the recrystallization, the title compound is obtained, NMR (CDCl₃) 10.06, 7.70, 7.58, 7.39, 7.16, 7.08, 6.91, 6.71, 4.90, 4.63, 3.45, 3.20, 2.95, 2.64, 1.94, 1.68, 1.32 and 0.87 δ.

### EXAMPLE 34 1-[5-(1-piperazinylcarbonylamino)-indole-2-carbonyl]-4-[3-(1-methylethylamino)-2-pyridinyl]piperazine (I)

Following the general procedure of EXAMPLE 20 and making non-critical variations but substituting piperazine for N-methylpiperazine, the title compound is obtained, mp 209° (decomp).

### EXAMPLE 35 1-[5-(N-(2-dimethylaminoethyl)ureido)-indole-2-carbonyl]-4-[3-(1-methylethylamino)-2-pyridinyl]piperazine (I)

Following the general procedure of EXAMPLE 20 and making non-critical variations such as the addition of N-methyl-2-pyrrolidinone as a cosolvent, and substituting N,N-dimethylethylene-diamine for N-methylpiperazine, the title compound is obtained, mp 124° (decomposition).

### EXAMPLE 36 1-[5-(N-(3-pyridyl)ureido)-indole-2-carbonyl]-4-[3-(1-methylethylamino)-2-pyridinyl]piperazine (I)

Following the general procedure of EXAMPLE 20 and making non-critical variations but substituting 3-aminopyridine for N-methylpiperazine, the title compound is obtained, NMR (chloroform-d) 11.52, 8.79, 8.64, 8.17, 7.98, 7.58, 7.32, 7.20, 6.95, 6.81, 4.50, 3.96, 3.62, 3.03, 1.19 δ.

### EXAMPLE 37 1-[5-(4-(1-methylethyl)-1-piperazinylcarbonylamino)-indole-2-carbonyl]-4-[3-(1-methylethylamino)-2-pyridinyl]piperazine (I)

To a solution of 1-[5-(1-piperazinylcarbonylamino)-indole-2-carbonyl]-4-[3-(1-methylethylamino)-2-pyridinyl]piperazine (EXAMPLE 34, 0.41 g) in methanol (50 ml) is added sodium cyanoborohydride (53 mg) and acetone (1 ml). Additional sodium cyanoborohydride (44.3 mg total) and excess acetone are added in portions until the reaction is complete as judged by TLC on silica gel. The mixture is acidified with acetic acid, neutralized with 50% aqueous sodium hydroxide and concentrated. The residue is dissolved in methylene chloride, washed with water, saturated potassium carbonate solution, and saline and dried over sodium sulfate followed by concentration. The resulting material is purified by chromatography on silica gel (100 ml) packed in methanol/chloroform (1/99) and eluted with a methanol/chloroform gradient (1 → 5%) to give the title compound, NMR (chloroform-d) 9,14, 7.71, 7.33, 7.17, 6.95, 6.86, 6.74, 4.06, 3.58, 3.20, 2.78, 2.60, 1.27, 1.08 δ.

### EXAMPLE 38 1-[5-(4-methyl-1-piperazinylcarbonylamino)-indole-2-carbonyl]-4-[3-(1-methylethylamino)-2-pyridinyl]piperazine (I)

Carbonyldiimidazole (1.54 g) is dissolved in of dry THF (40 ml). 1-[5-aminoindolyl-2-carbonyl]-4-[3-(1-methylethylamino)-2-pyridinylpiperazine (VII, EXAMPLE 7, 3.0 g) and N-methylpiperazine (1.3 ml) are dissolved in dry THF (75 ml) and added to the carbonyldiimidazole solution over 1.5 hr. After an additional hour the reaction is concentrated and the concentrate is dissolved in methylene chloride (125 ml) and washed with water and saline, dried over sodium sulfate, concentrated and dried under reduced pressure. The mixture is chromatographed over silica gel (450 ml) packed in 2% methanol/methylene chloride eluting with a methanol/methylene chloride gradient (2 → 50% ). Fractions with an R_{f} value of about 0.14 in methanol/chloroform (10/90) are collected and concentrated to give a solid. The solid is dissolved in chloroform (100 ml/1 g of solid), filtered and the solution is concentrated to dryness to afford the title compound. The solid is recrystallized from methanol/chloroform, adding ethyl ether as crystals begin to form to give the title compound, mp 200°.

### EXAMPLE 39 1-[5-(4-morpholinocarbonylamino)-indole-2-carbonyl]-4-[3-(1-methylethylamino)-2-pyridinyl]piperazine (I)

Following the general procedure for EXAMPLE 38 and making non-critical variations but substituting morpholine for N-methylpiperazine, the title compound is obtained, mp 133-136°.

### EXAMPLE 40 1-[5-(4-Methyl-1-piperazinylcarbonylamino)indole-2-carbonyl]-4-[3-(1,1-dimethylpropylamino)-2-pyridinyl]piperazine (I)

Following the general procedure for EXAMPLE 38 and making non-critical variations but substituting 1-[5-aminoindole-2-carbonyl]-4-[3-(1,1-dimethylpropylamino)-2-pyridinyl]piperazine (PREPARATION 42) for 1-[5-aminoindolyl-2-carbonyl]-4-[3-(1-methylethylamino)-2-pyridinylpiperazine the title compound is obtained, NMR (chloroform-d) 10.02, 7.68, 7.62, 7.21, 7.07, 6.97, 6.88, 6.61, 4.90, 4.59, 3.50, 3.38, 3.11, 2.61, 2.36, 2.23, 1.90, 1.64, 1.31, 0.86 δ.

### EXAMPLE 41 1-[6-[2-(2-Methoxyethoxy)ethoxyindolyl-2-carbonyl]-4-[3-(1-methylethylamino)-2-pyridinyl]piperazine (I)

6-[2-(2-Methoxyethoxy)ethoxy]indole-2-carboxylic acid (VI, PREPARATION 28, 0.30 g) and 3-(1-methylethylamino)-2-pyridinyl]piperazine (0.26 g) is dissolved in THF (5.35 ml) and 1-(ethyl-3-(dimethylaminopropyl)carbodiimide (EDC, 0.25 g) is added. The reaction is stirred 2.75 hr at 20-25° and poured into chloroform, washed with saturated sodium bicarbonate and saline. The material is concentrated under reduced pressure and purified by flash column chromatography eluting with methanol/chloroform (2.5/97.5). The appropriate fractions are pooled and concentrated to provide the title compound, mp 135-136°.

### EXAMPLE 42 1-[5-(3-(1-Piperidinyl)propyl)sulfonamidoindolyl-2-carbonyl]-4-[3-(1-methylethylamino)-2-pyridinyl]piperazine (I)

1-[5-(3-Chloropropyl)sulfonamidoindolyl-2-carbonyl]-4-[3-(1-methylethylamino)-2-pyridinyl]piperazine (PREPARATION 33, 0.60 g), piperidine (0.115 ml) and sodium iodide (0.174 g) are placed in 5.8 ml of acetonitrile and refluxed for 28 hr. Then the reaction is diluted with chloroform, washed with water, saline, dried over anhydrous sodium sulfate and concentrated under reduced pressure. Purification by flash column chromatography eluting with methanol/chloroform (2.5/97.5), pooling and concentrating the appropriate fractions gives the title compound, mp 145-146°.

### EXAMPLE 43 1-[5-(3-(1-Piperidinyl)propyl)sulfonamidoindolyl-2-carbonyl]-4-[3-(1,1-dimethylethylamino)-2-pyridinyl]piperazine (I)

Following the general procedure of EXAMPLE 5, and making non-critical variations but substituting 5-[(3-(1-piperidinyl)propyl)sulfonamido]indole-2-carboxylic acid (PREPARATION 32) for 6-[(2-(2-hydroxyethoxy)ethoxy)ethoxy]indole-2-carboxylic acid, the title compound is obtained, mp 118-120°.

### EXAMPLE 44 1-[5-(3-(Morpholin-1-yl)propyl)sulfonamidoindolyl-2-carbonyl]-4-[3-(1,1-dimethylethylamino)-2-pyridinyl]piperazine (I)

1-[5-Aminoindolyl-2-carbonyl]-4-[3-(1-methylethylamino)-2-pyridinyl]piperazine (EXAMPLE 7, 0.50 g) and 0.37 ml of triethylamine are dissolved in 2.6 ml of methylene chloride and cooled to 20-25°. Then 0.35 g of 3-(morpholin-1-ylpropyl)sulfonyl chloride hydrochloride (Prepared by dissolving 5.0 g of 4-morpholinepropanesulfonic acid in 10 ml of phosphorus oxychloride and adding 4.97 g of phosphorus pentachloride and stirring overnight at 20-25°. Then concentrating under reduced pressure, chasing with benzene, collecting the solids and washing with carbon tetrachloride and ether.) is added and the reaction is slowly allowed to warm to 20-25° and stirred for 4 hr. Then the reaction is diluted with chloroform, washed with saturated aqueous sodium bicarbonate, dried over anhydrous sodium sulfate and concentrated under reduced pressure. Purification by flash column chromatography eluting with methanol/chloroform (3/97), pooling and concentrating the appropriate fractions and crystallizing from ethyl acetate gives the title compound, mp 183-185°.

### EXAMPLE 45 1-[5-Dimethylaminosulfamoylaminoindolyl-2-carbonyl]-4-[3-(1-methylethylamino)-2-pyridinyl]piperazine (I)

1-[5-Aminoindolyl-2-carbonyl]-4-[3-(1-methylethylamino)-2-pyridinyl]piperazine (EXAMPLE 7, 0.485 g) is dissolved in 3 ml of acetonitrile and 0.10 ml of pyridine is added. The reaction is cooled to 0° and the dimethylaminosulfamoylchloride (0.14 ml) is added in one portion. The reaction is warmed to 20-25° and stirred for 48 hr. Then it is poured into saturated aqueous sodium bicarbonate and extracted with methylene chloride, dried over anhydrous sodium sulfate and concentrated under reduced pressure. Purification by flash column chromatography eluting with a gradient of 50% ethyl acetate/hexane to 100 % ethyl acetate), pooling and concentrating the appropiate fractions gives the title compound, mp 174-175°.

### EXAMPLE 46 1-[5-Trifluoroacetamidoindolyl-2-carbonyl]-4-[3-(1-methylethylamino)-2-pyridyl] piperazine (I)

1-[5-Aminoindolyl-2-carbonyl]-4-[3-(1-methylethylamino)-2-pyridyl]piperazine (EXAMPLE 7, 0.30 g, 0.79 mmol) is dissolved in 1.6 ml of methylene chloride and pyridine (0.064 ml, 0.79 mmol) is added. The reaction was cooled to 0° and trifluoroacetic anhydride (0.11 ml, 0.79 mmol) is added dropwise. The reaction is stirred for 2 hr at 0° and then an additional 0.2 equivalents of trifluoroacetic anhydride is added and the reaction was stored at 0° for 18 hr. The mixture is diluted with chloroform, washed with water, saline, dried over anhydrous sodium sulfate and concentrated under reduced pressure. Purification by flash column chromatography, eluting with methanol/chloroform (2/98), pooling and concentration of the appropriate fractions gives the title compound, mp 215-218°; NMR(300 MHz, CD₃OD) 7.88, 7.81, 7.47, 7.36, 7.30, 6.90, 6.78, 3.95, 3.54, 3.01, 1.16 δ.

### EXAMPLE 47 1-[5-Methoxycarbamoylindolyl-2-carbonyl]4-[3-(1-methylethylamino)-2-pyridyl] piperazine

1-[5-Aminoindolyl-2-carbonyl]-4-[3-(1-methylethylamino)-2-pyridyl]piperazine (EXAMPLE 7, 0.30 g, 0.79 mmol) is dissolved in 1.6 ml of methylene chloride and pyridine (0.064 ml, 0.79 mmol) is added. The reaction is cooled to 0° and methyl chloroformate (0.06 ml, 0.79 mmol) is added dropwise. The reaction is stirred for 2 hr at 0° and the reaction was stored at 0° for 18 hr. Then it was diluted with chloroform, washed with water, saline, dried over anhydrous sodium sulfate and concentrated under reduced pressure. Purification by recrystallization from methanol/hexane gives the title compound, mp 230-231°.

### EXAMPLE 48 1-[5-(4-Methylpiperazin-1-ylcarbonylamino)indolyl-2-carbonyl]-4-[3-(1,1-dimethylethylamino)-2-pyridyl]piperazine (I)

Following the general procedure of EXAMPLE 6, and making non-critical variations but substituting 1-[3-(1,1-dimethylethylamino)-2-pyridyl]piperazine (PREPARATION 36) for 1-[3-(1-methylethylamino)-2-pyridinyl]piperazine and 5-(4-methylpiperazin-1-ylcarbonylamino)indole-2-carboxylic acid (PREPARATION 38) for 5-nitroindole-2-carboxylic acid, the title compound is obtained, mp 183-185°.

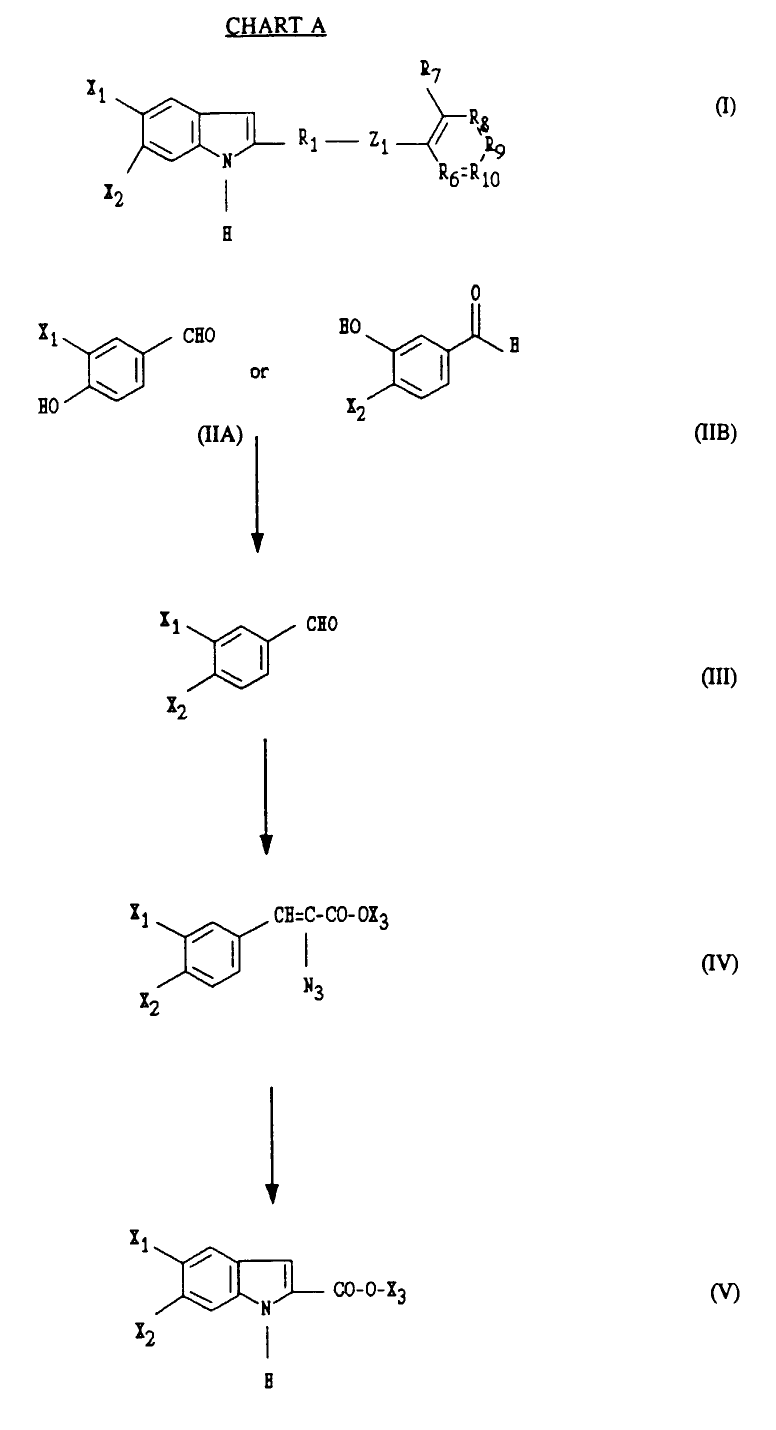

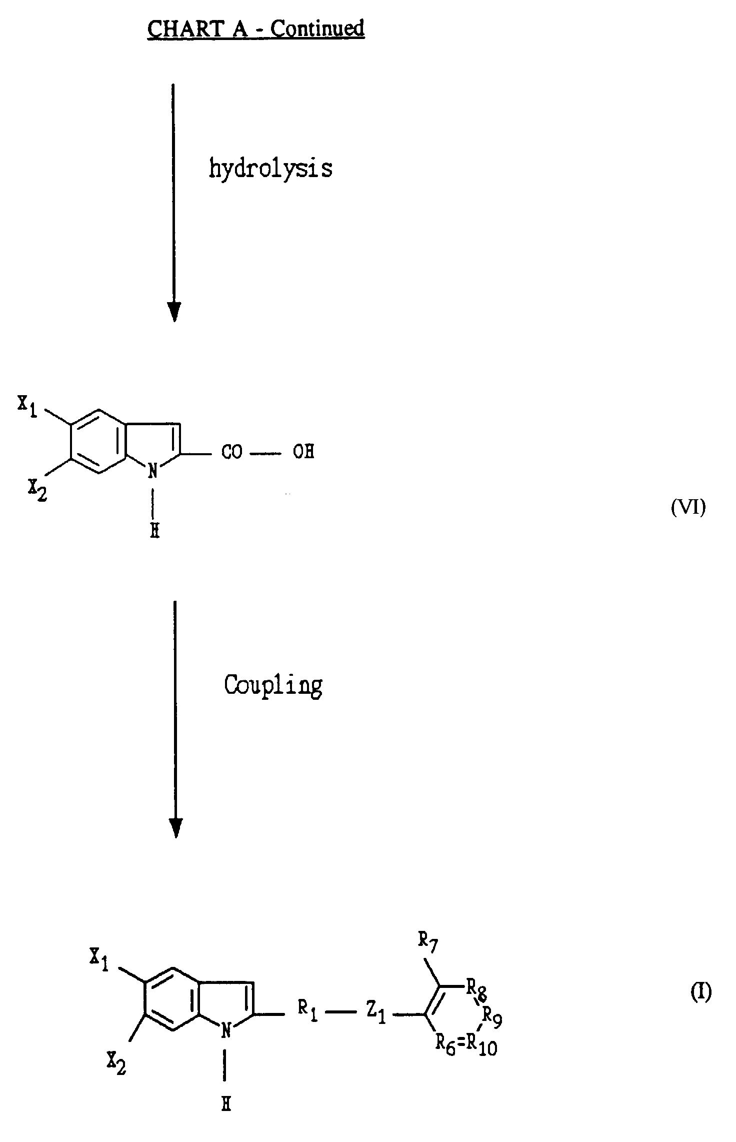

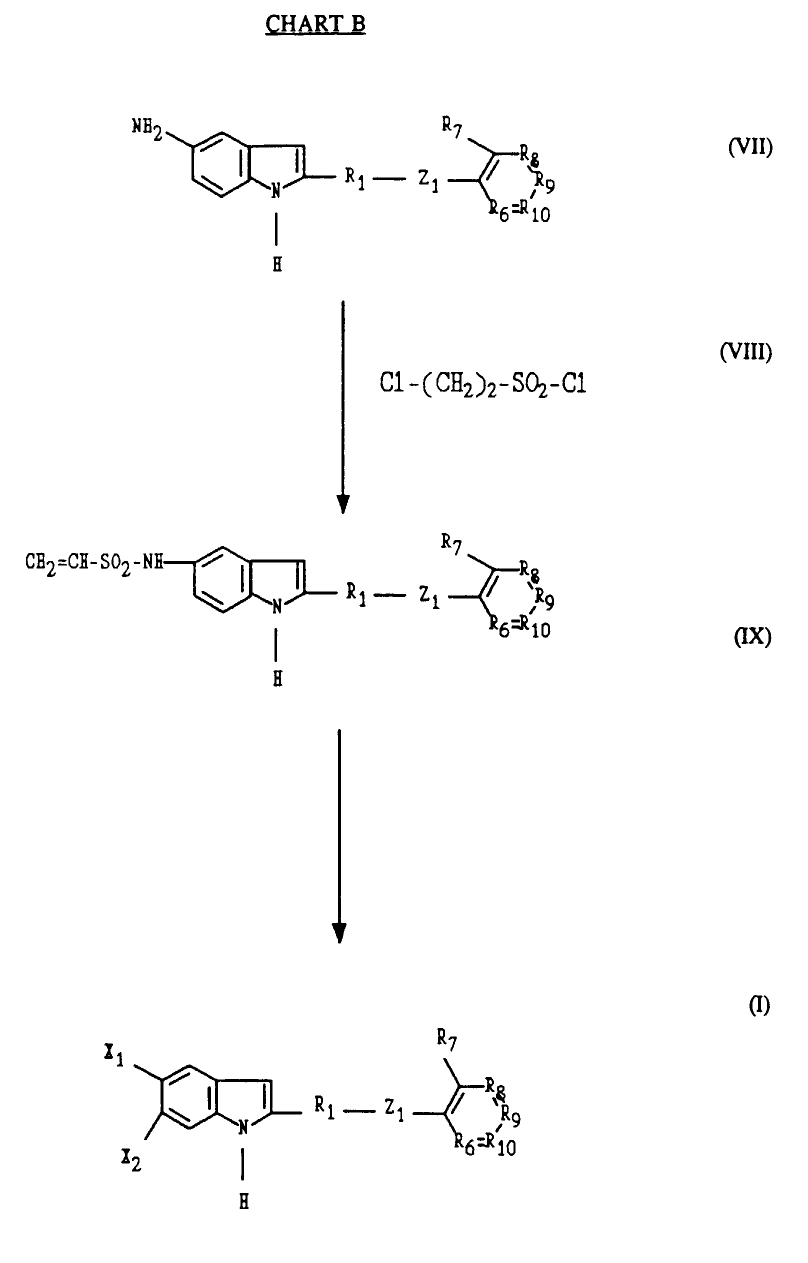

## Claims (Claims for the following Contracting State(s): AT, BE, CH, DE, DK, FR, GB, GR, IT, LI, LU, MC, NL, SE)

1. A substituted indole of formula (I) 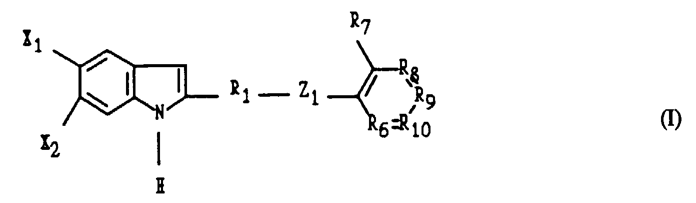 where R₁ is -CH₂- or -CO-; where Z₁ is 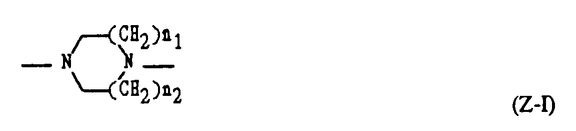 where n₁ is 1 or 2 and n₂ is 1 or 2, or 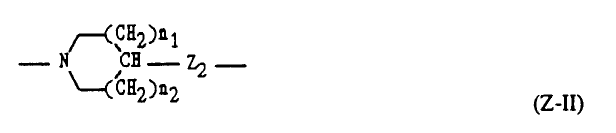 where Z₂ is -N(Z₂₋₁)- where Z₂₋₁ is C₁-C₄ alkyl and where n₁ and n₂ are as defined above;
where one of X₁ and X₂ is H and the other is
-O-(CH₂CH₂O-)ₙ₃-X₁₋₁ where n₃ is 1 thru 4, where X₁₋₁ is -H or C₁-C₄ alkyl,
-O-SO₂-(CH₂)ₙ₄-N(X₁₋₂)(X₁₋₃) where n₄ is 1 thru 3, where either X₁₋₂ and X₁₋₃ are the same or different and are -H or C₁-C₆ alkyl, or X₁₋ ₂ and X₁₋₃ are taken together with the attached nitrogen atom, to form a ring selected from 1-pyrrolidinyl, 1-piperidinyl, 1-piperazinyl, 1-morpholinyl and 1-piperazinyl substituted
in the 4-position with C₁-C₄ alkyl,
-O-SO₂-(CH₂)ₙ₄-X₁₋₈ where X₁₋₈ is 2-pyridinyl, 3-pyridinyl and 4-pyridinyl and
where n₄ is as defined above,
-O-CO-(CH₂)ₙ₄-NX₁₋₂X₁₋₃ where n₄, X₁₋₂ and X₁₋₃ are as defined above,
-NH-CO-(CH₂)ₙ₄-NX₁₋₂X₁₋₃ where n₄, X₁₋₂ and X₁₋₃ are as defined above,
-N(X₁₋₄)-SO₂-X₁₋₅ where X₁₋₄ is C₁-C₃ alkyl and X₁₋₅ is C₁-C₄ alkyl,
-N(X₁₋₇)-CO-NH-(CH₂)ₙ₄-N(X₁₋₂)(X₁₋₃) where X₁₋₇ is -H or C₁-C₃ alkyl, and where n₄, X₁₋₂ and X₁₋₃ are as defined above,
-N(X₁₋₇)-SO₂-(CH₂)ₙ₄-X₁₋₈ where n₄, X₁₋₇ and X₁₋₈ are as defined above,
-N(X₁₋₇)-CO-NH-(CH₂)ₙ₅-X₁₋₈ where n₅ is 0 thru 3 and where X₁₋₇ and X₁₋₈ are as defined above,
-NX(₁₋₇)-SO₂-(CH₂)ₙ₄-N(X₁₋₂)(X₁₋₃) where n₄, X₁₋₂, X₁₋₃ and X₁₋₇ are as defined above,
-NX(₁₋₇)-CO-O-X₁₋₆ where X₁₋₆ is C₁-C₄ alkyl or -(CH₂)ₙ₄-N(X₁₋₂)(X₁₋₃) where n₄, X₁₋₂, X₁₋₃ and X₁₋₇ are as defined above,
-N(X₁₋₇)-CO-N(X₁₋₂)(X₁₋₃) where X₁₋₂, X₁₋₃ and X₁₋₇ are as defined above,
-NH-CO-CF₃, or
-N(X₁₋₇)-SO₂-N(X₁₋₂)(X₁₋₃) where X₁₋₂, X₁₋₃ and X₁₋₇ are as defined above,
R₆ is
-N=,
-CH=, or
-N(O)=,
R₇ is
-COO-R₇₋₁₁ where R₇₋₁₁ is C₁-C₆ alkyl,
-CO-N(R₇₋₃)(R₇₋₄) where R₇₋₃ and R₇₋₄ are the same or different and are -H or C₁-C₆ alkyl,
-N(R₇₋₅)(R₇₋₆) where R₇₋₅ is
C₁-C₆ alkyl,
-C(R₇₋₁₅)(R₇₋₁₆)-(R₇₋₁₇) where R₇₋₁₅ and R₇₋₁₆ are the same or different and are -H or C₁-C₃ alkyl and where R₇₋₁₇ is C₂-C₅ alkenyl containing 1 or 2 double bonds or C₂-C₅ alkynyl containing 1 triple bond,
-CH₂-CH₂-OH,
-CH₂-CH₂-CH₂-OH,
-CH(CH₃)CH₂-O-CH₃,
-CH(CH₃)CH₂-OH,
-CH₂-CF₃,
-CH₂-cyclopropyl,
-CH₂-CH₂F,
-CH₂-CH₂-C ≡ N,
-C^{*}R₇₋₁₈-(CH₂)ₙ₁₄-C^{*}H₂ where R₇₋₁₈ is -H or -CH₃, n₁₄ is 1 thru 5 and the carbon atoms marked with an asterisk (^{*}) are bonded to each other to resulting in the formation of a ring,
-(CH₂)n₁-N(R₇₋₇)(R₇₋₈) where n₁ is as defined above and where R₇₋₇ and R₇₋₈ are the same or different and are -H or C₁-C₄ alkyl, and where R₇₋₇ and R₇₋₈ are taken together with the attached nitrogen atom to form a heterocyclic ring selected from 1-pyrrolidinyl, 1-piperidinyl, 1-piperazinyl, N-morpholinyl and 1-aziridinyl,
and where R₇₋₆ is
-H,
C₁-C₆ alkyl,
-C(R₇₋₁₅)(R₇₋₁₆)-(R₇₋₁₇) where R₇₋₁₅, R₇₋₁₆ and R₇₋₁₇ are as defined above,
-CH₂-CH₂-OH,
-CH₂-CH₂-CH₂-OH,
-CH₂CF₃,
-CH₂-CH₂F,
-CH₂-CH₂-C ≡ N,
or where R₇₋₅ and R₇₋₆ are taken together with the attached nitrogen atom to form a heterocyclic ring selected from 1-pyrrolidinyl, 1-piperidinyl, 1-piperazinyl, N-morpholinyl and 1-aziridinyl,
-(CH₂)ₙ₄-N(R₇₋₉)(R₇₋₁₀) where n₄ is as defined above and where R₇₋₉ and R₇₋₁₀ are the same or different and are -H or C₁-C₄ alkyl, and where R₇₋₉ and R₇₋₁₀ are taken together with the attached nitrogen atom to form a heterocyclic ring selected from 1-pyrrolidinyl, 1-piperidinyl, 1-piperazinyl and N-morpholinyl,
R₈ is
-N=,
-CR₈₋₁= where R₈₋₁ is -H, -F, -Cl, -Br, -CF₃,
-NO₂, -COCF₃,
C₁-C₆ alkyl,
C₁-C₃ alkylthio,
-OH,
-O-R₈₋₂ where R₈₋₂ is C₁-C₆ alkyl, -φ, -CO-R₈₋₃ where R₈₋₃ is C₁-C₆ alkyl or -φ,
-NH(R₈₋₄) where R₈₋₄ is
C₁-C₆ alkyl,
-C(R₈₋₇)(R₈₋₈)(R₈₋₉) where R₈₋₇ and R₈₋₈ are the same or different and are -H or C₁-C₃ alkyl and where R₈₋₉ is C₂-C₅ alkenyl containing 1 or 2 double bonds or C₂-C₅ alkynyl containing 1 triple bond, or
-NR₈₋₅-CO-R₈₋₆ where R₈₋₅ is -H or C₁-C₆ alkyl and R₈₋₆ is -H, C₁-C₆ alkyl or C₁-C₃ alkoxy;
R₉ is
-N=,
-CR₉₋₁= where R₉₋₁ is -H, -F, -Cl, -Br,
-NO₂, -COCF₃,
C₁-C₆ alkyl,
C₁-C₃ alkylthio,
-OH,
-O-R₉₋₂ where R₉₋₂ is C₁-C₆ alkyl, -φ, -CO-R₉₋₃ where R₉₋₃ is C₁-C₆ alkyl or -φ,
-N(R₉₋₄)(R₉₋₅) where R₉₋₄ and R₉₋₅ are the same or different and are
-H,
C₁-C₆ alkyl,
-C(R₉₋₈)(R₉₋₉)-(R₉₋₁₀) where R₉₋₈ and R₉₋₉ are the same or different and are -H or C₁-C₃ alkyl and where R₉₋₁₀ is C₂-C₅ alkenyl containing 1 or 2 double bonds or C₂-C₅ alkynyl containing 1 triple bond,
R₉₋₄ and R₉₋₅ are taken together with the attached nitrogen atom to form a heterocyclic ring selected from 1-pyrrolidinyl, 1-piperidinyl, 1-piperazinyl and N-morpholinyl, or
-NR₉₋₆-CO-R₉₋₇ where R₉₋₆ is -H or C₁-C₆ alkyl and R₉₋₇ is -H, C₁-C₆ alkyl or C₁-C₃ alkoxy;
R₁₀ is
-N=,
-CR₁₀₋₁ = where R₁₀₋₁ is -H, -F, -Cl, -Br, -CF₃,
-NO₂, -COCF₃,
C₁-C₆ alkyl,
C₁-C₃ alkylthio,
-OH,
-O-R₁₀₋₂ where R₁₀₋₂ is C₁-C₆ alkyl, -φ, -CO-R₁₀₋₃ where R₁₀₋₃ is C₁-C₆ alkyl or -φ,
-N(R₁₀₋₄)(R₁₀₋₅) where R₁₀₋₄ and R₁₀₋₅ are the same or different and are -H,
C₁-C₆ alkyl,
-C(R₁₀₋₈)(R₁₀₋₉)-(R₁₀₋₁₀) where R₁₀₋₈ and R₁₀₋₉ are the same or different and are -H or C₁-C₃ alkyl and where R₁₀₋₁₀ is C₂-C₅ alkenyl containing 1 or 2 double bonds or C₂-C₅ alkynyl containing 1 triple bond, -NR₁₀₋₆-CO-R₁₀₋₇ where R₁₀₋₆ is -H or C₁-C₆ alkyl and R₁₀₋₇ is -H, C₁-C₆ alkyl or C₁-C₃ alkoxy;
with the proviso that not more than two of R₆, R₈, R₉ and R₁₀ are -N = ; or an enantiomer, pharmaceutically-acceptable salt, hydrate or solvate thereof.

2. A substituted indole (I) according to claim 1 where Z₁ is (Z-I) where n₁ and n₂ are 1.

3. A substituted indole (I) according to claim 1 where Z₁ is (Z-II) where n₁ and n₂ are 1.

4. A substituted indole (I) according to claim 3 where Z₂₋₁ is C₁ alkyl.

5. A substituted indole (I) according to claim 1 where R₁ is -CO-.

6. A substituted indole (I) according to claim 1 where R₆ is -N=.

7. A substituted indole (I) according to claim 1 where R₈, R₉ and R₁₀ are each -CH=.

8. A substituted indole (I) according to claim 1 where R₇ is -N(R₇₋₅)(R₇₋₆) where R₇₋₅ is C₂-C₄ alkyl and where R₇₋₆ is -H.

9. A substituted indole (I) according to claim 1 where one of X₁ or X₂ is selected from
-N(X₁₋₇)-CO-N(X₁₋₂)(X₁₋₃) and
-N(X₁₋₇)-SO₂-N(X₁₋₂)(X₁₋₃).

10. A substituted indole (I) according to claim 1 which is selected from
1-[6-(2-(2-hydroxyethoxy)ethoxy)ethoxyindolyl-2-carbonyl]-4-[3-(1-methylethylamino)-2-pyridinyl]piperazine,
1-[5-(2-(1-pyrrolidino)ethyl)sulfonamidoindolyl-2-carbonyl]-4-(3-(1-methylethylamino)-2-pyridinyl]piperazine,
1-[6-(2-hydroxyethoxy)ethoxyindolyl-2-carbonyl]-4-[3-(1-methylethylamino)-2-pyridinyl]piperazine,
1-[5-(2-(1-piperidinyl)ethyl)sulfonamidoindolyl-2-carbonyl]-4-[3-(1-methylethylamino)-2-pyridinyl]piperazine,
1-[5-(2-(1-morpholinyl)ethyl)sulfonamidoindolyl-2-carbonyl]-4-[3-(1-methylethylamino)-2-pyridinyl]piperazine,
1-[6-(2-(2-hydroxyethoxy)ethoxy)ethoxyindolyl-2-carbonyl]-4-[N-methyl-N-(3-(1-methylethylamino)-2-pyridinyl)amino]piperidine,
1-[5-(3-methylureido)-indolyl-2-carbonyl]4-[N-methyl-N-(3-(1-methylethylamino)-2-pyridinyl)amino]piperidine,
1-[5-(2-(4-pyridyl)-ethanesulfonamido)-indolyl-2-carbonyl]-4-[N-methyl-N-(3-(1-methylethylamino)-2-pyridinyl)amino]piperidine, and
1-[5-(4-methylpimrazin-1-ylcarbonylamino)-indolyl-2-carbonyl]-4-[3-(1-methylethylamino)-2-pyridinyl)amino]piperazine.

11. Use of a substituted indole (I) according to any preceding claim, for the manufacture of a medicament for use in the treatment of AIDS.

## Claims (Claims for the following Contracting State(s): ES)

1. A process for preparing a substituted indole of formula (I) 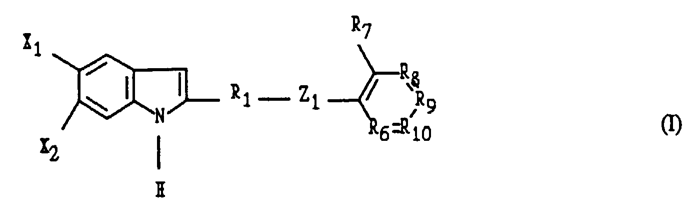 where R₁ is -CH₂- or -CO-;
where Z₁ is 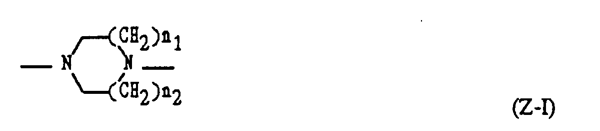 where n₁ is 1 or 2 and n₂ is 1 or 2, or 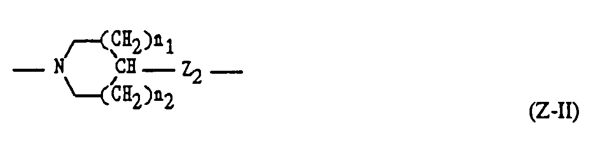 where Z₂ is -N(Z₂₋₁)- where Z₂₋₁ is C₁-C₄ alkyl and where n₁ and n₂ are as defined
above; where one of X₁ and X₂ is H and the other is
-O-(CH₂CH₂-O-)ₙ₃-X₁₋₁ where n₃ is 1 thru 4, where X₁₋₁ is -H or C₁-C₄ alkyl,
-O-SO₂-(CH₂)ₙ₄-N(X₁₋₂)(X₁₋₃) where n₄ is 1 thru 3, where either X₁₋₂ and X₁₋₃ are the same or different and are -H or C₁-C₆ alkyl, or X₁₋ ₂ and X₁₋₃ are taken together with the attached nitrogen atom, to form a ring selected from 1-pyrrolidinyl, 1-piperidinyl, 1-piperazinyl, 1-morpholinyl and 1-piperazinyl substituted
in the 4-position with C₁-C₄ alkyl,
-O-SO₂-(CH₂)ₙ₄-X₁₋₈ where X₁₋₈ is 2-pyridinyl, 3-pyridinyl and 4-pyridinyl and
where n₄ is as defined above, or
-O-CO-(CH₂)ₙ₄-NX₁₋₂X₁₋₃ where n₄, X₁₋₂ and X₁₋₃ are as defined above,
R₆ is
-N=,
-CH=, or
-N(O)=,
R₇ is -COO-R₇₋₁₁ where R₇₋₁₁ is C₁-C₆ alkyl,
-CO-N(R₇₋₃)(R₇₋₄) where R₇₋₃ and R₇₋₄ are the same or different and are -H or C₁-C₆ alkyl,
-N(R₇₋₅)(R₇₋₆) where R₇₋₅ is
C₁-C₆ alkyl,
-C(R₇₋₁₅)(R₇₋₁₆)-(R₇₋₁₇) where R₇₋₁₅ and R₇₋₁₆ are the same or different and are -H or C₁-C₃ alkyl and where R₇₋₁₇ is C₂-C₅ alkenyl containing 1 or 2 double bonds or C₂-C₅ alkynyl containing 1 triple bond,
-CH₂-CH₂-OH,
-CH₂-CH₂-CH₂-OH,
-CH(CH₃)CH₂-O-CH₃,
-CH(CH₃)CH₂-OH,
-CH₂-CF₃,
-CH₂-cyclopropyl,
-CH₂-CH₂F,
-CH₂-CH₂-C ≡ N,
-C^{*}R₇₋₁₈-(CH₂)ₙ₁₄-C^{*}H₂ where R₇₋₁₈ is -H or -CH₃, n₁₄ is 1 thru 5 and the carbon atoms marked with an asterisk (^{*}) are bonded to each other to resulting in the formation of a ring,
-(CH₂)n₁-N(R₇₋₇)(R₇₋₈) where n₁ is as defined above and where R₇₋₇ and R₇₋₈ are the same or different and are -H or C₁-C₄ alkyl, and where R₇₋₇ and R₇₋₈ are taken together with the attached nitrogen atom to form a heterocyclic ring selected from 1-pyrrolidinyl, 1-piperidinyl, 1-piperazinyl, N-morpholinyl and 1-aziridinyl,
and where R₇₋₆ is
-H,
C₁-C₆ alkyl,
-C(R₇₋₁₅)(R₇₋₁₆)-(R₇₋₁₇) where R₇₋₁₅, R₇₋₁₆ and R₇₋₁₇ are as defined above,
-CH₂-CH₂-OH,
-CH₂-CH₂-CH₂-OH,
-CH₂CF₃,
-CH₂-CH₂F,
-CH₂-CH₂-C ≡ N,
or where R₇₋₅ and R₇₋₆ are taken together with the attached nitrogen atom to form a heterocyclic ring selected from 1-pyrrolidinyl, 1-piperidinyl, 1-piperazinyl, N-morpholinyl and 1-aziridinyl,
-(CH₂)ₙ₄-N(R₇₋₉)(R₇₋₁₀) where n₄ is as defined above and where R₇₋₉ and R₇₋₁₀ are the same or different and are -H or C₁-C₄ alkyl, and where R₇₋₉ and R₇₋₁₀ are taken together with the attached nitrogen atom to form a heterocyclic ring selected from 1-pyrrolidinyl, 1-piperidinyl, 1-piperazinyl and N-morpholinyl,
R₈ is
-N=,
-CR₈₋₁ = where R₈₋₁ is -H, -F, -Cl, -Br, -CF₃,
-NO₂, -COCF_{3,}
C₁-C₆ alkyl,
C₁-C₃ alkylthio,
-OH,
-O-R₈₋₂ where R₈₋₂ is C₁-C₆ alkyl, -φ, -CO-R₈₋₃ where R₈₋₃ is C₁-C₆ alkyl or -φ,
-NH(R₈₋₄) where R₈₋₄ is
C₁-C₆ alkyl,
-C(R₈₋₇)(R₈₋₈)-(R₈₋₉) where R₈₋₇ and R₈₋₈ are the same or different and are -H or C₁-C₃ alkyl and where R₈₋₉ is C₂-C₅ alkenyl containing 1 or 2 double bonds or C₂-C₅ alkynyl containing 1 triple bond, or
-NR₈₋₅-CO-R₈₋₆ where R₈₋₅ is -H or C₁-C₆ alkyl and R₈₋₆ is -H, C₁-C₆ alkyl or C₁-C₃ alkoxy;
R₉ is
-N=,
-CR₉₋₁= where R₉₋₁ is -H, -F, -Cl, -Br,
-NO₂, -COCF₃,
C₁-C₆ alkyl,
C₁-C₃ alkylthio,
-OH,
-O-R₉₋₂ where R₉₋₂ is C₁-C₆ alkyl, -φ, -CO-R₉₋₃ where R₉₋₃ is C₁-C₆ alkyl or -φ,
-N(R₉₋₄)(R₉₋₅) where R₉₋₄ and R₉₋₅ are the same or different and are
-H,
C₁-C₆ alkyl,
-C(R₉₋₈)(R₉₋₉)-(R₉₋₁₀) where R₉₋₈ and R₉₋₉ are the same or different and are -H or C₁-C₃ alkyl and where R₉₋₁₀ is C₂-C₅ alkenyl containing 1 or 2 double bonds or C₂-C₅ alkynyl containing 1 triple bond,
R₉₋₄ and R₉₋₅ are taken together with the attached nitrogen atom to form a heterocyclic ring selected from 1-pyrrolidinyl, 1-piperidinyl, 1-piperazinyl and N-morpholinyl, or
-NR₉₋₆-CO-R₉₋₇ where R₉₋₆ is -H or C₁-C₆ alkyl and R₉₋₇ is -H, C₁-C₆ alkyl or C₁-C₃ alkoxy;
R₁₀ is
-N=,
-CR₁₀₋₁= where R₁₀₋₁ is -H, -F, -Cl, -Br, -CF₃,
-NO₂, -COCF₃,
C₁-C₆ alkyl,
C₁-C₃ alkylthio,
-OH,
-O-R₁₀₋₂ where R₁₀₋₂ is C₁-C₆ alkyl, -φ, -CO-R₁₀₋₃ where R₁₀₋₃ is C₁-C₆ alkyl or -φ,
-N(R₁₀₋₄)(R₁₀₋₅) where R₁₀₋₄ and R₁₀₋₅ are the same or different and are -H,
C₁-C₆ alkyl,
-C(R₁₀₋₈) (R₁₀₋₉)-(R₁₀₋₁₀) where R₁₀₋₈ and R₁₀₋₉ are the same or different and are -H or C₁-C₃ alkyl and where R₁₀₋₁₀ is C₂-C₅ alkenyl containing 1 or 2 double bonds or C₂-C₅ alkynyl containing 1 triple bond, -NR₁₀₋₆-CO-R₁₀₋₆ where R₁₀₋₆ is -H or C₁-C₆ alkyl and R₁₀₋₇ is -H, C₁-C₆ alkyl or C₁-C₃ alkoxy;
with the proviso that not more than two of R₆, R₈, R₉ and R₁₀ are -N=; or an enantiomer, pharmaceutically-acceptable salt, hydrate or solvate thereof;
which comprises coupling a corresponding indole acid (VI) with a corresponding amine (Z₁-aromatic portion).

2. A process for preparing a substituted indole of formula (I) as defined in claim 1, except that the other of X₁ and X₂ is
-NH-CO-(CH₂)ₙ₄-NX₁₋₂X₁₋₃ where n₄ , X₁₋₂ and X₁₋₃ are as defined in claim 1,
-N(X₁₋₄) -SO₂-X₁₋₅ where X₁₋₄, is C₁-C₃ alkyl and X₁₋₅ is C₁-C₄ alkyl,
-N(X₁₋₇)-CO-NH-(CH₂)ₙ₄-N(X₁₋₂) (X₁₋₃) where X₁₋₇ is -H or C₁-C₃ alkyl, and where n₄, X₁₋₂ and X₁₋₃ are as defined in claim 1,
-N(X₁₋₇)-SO₂-(CH₂)ₙ₄-X₁₋₈ where n₄, X₁₋₇ and X₁₋₈ are as defined in claim 1,
-N(X₁₋₇)-CO-NH-(CH₂)ₙ₅-X₁₋₈ where n₅ is 0 thru 3 and where X₁₋₇ and X₁₋₈ are as defined in claim 1,
-N(X₁₋₇)-SO₂-(CH₂)ₙ₄-N(X₁₋₂)(X₁₋₃) where n₄, X₁₋₂, X₁₋₃ and X₁₋₇ are as defined in claim 1,
-NX₍₁₋₇₎-CO-O-X₁₋₆ where X₁₋₆ is C₁-C₄ alkyl or -(CH₂)n₄-N(X₁₋₂)(X₁₋₃) where n₄, X₁₋₂, X₁₋₃ and X₁₋₇ are as defined in claim 1,
-N(X₁₋₇)-CO-N(X₁₋₂)(X₁₋₃) where X₁₋₂, X₁₋₃ and X₁₋₇ are as defined in claim 1,
-NH-CO-CF₃, or
-N(X₁₋₇)-SO₂-N(X₁₋₂) (X₁₋₃) where X₁₋₂, X₁₋₃ and X₁₋₇ are as defined in claim 1,
which comprises reaction of the corresponding compound, when the other of X₁ and X₂ is NH₂,
(i) with sulfonyl chloride and then with the desired amine and powdered copper catalyst under reflux
(ii) with a functionalised sulfonyl chloride such as 2-(4-pyridyl)ethanesulfonyl chloride
(iii) with an isocyanate
(iv) with phosgene or a phosgene equivalent, and trapping with an amine
(v) with X-SO₂-X followed by reaction with an amine.

3. A process according to claim 1 or claim 2, where Z₁ is (Z-I) where n₁ and n₂ are 1.

4. A process according to claim 1 or claim 2, where Z₁ is (Z-II) where n₁ and n₂ are 1.

5. A process according to claim 4, where Z₂₋₁ is C₁ alkyl.

6. A process according to claim 1 or claim 2, where R₁ is -CO-.

7. A process according to claim 1 or claim 2, where R₆ is -N=.

8. A process according to claim 1 or claim 2, where R₈, R₉ and R₁₀ are each -CH=.

9. A process according to claim 1 or claim 2, where R₇ is -N(R₇₋₅)(R₇₋₆) where R₇₋₅ is C₂-C₄ alkyl and where R₇₋₆ is -H.

10. A process according to claim 2, where the other of X₁ and X₂ is selected from
-N(X₁₋₇)-CO-N(X₁₋₂)(X₁₋₃) and
-N(X₁₋₇)-SO₂-N(X₁₋₂)(X₁₋₃).

11. A process according to claim 1, where the substituted indole (I) is selected from
1-[6-(2-(2-hydroxyethoxy)ethoxy)ethoxyindolyl-2-carbonyl]-4-[3-(1-methylethylamino)-2-pyridinyl]piperazine,
1-[6-(2-hydroxyethoxy)ethoxyindolyl-2-carbonyl]-4-[3-(1-methylethylamino)-2-pyridinyl]piperazine, and
1-[6-(2-(2-hydroxyethoxy)ethoxy)ethoxyindolyl-2-carbonyl]-4-[N-methyl-N-(3-(1-methylethylamino)-2-pyridinyl)amino]piperidine.

12. A process according to claim 2, where the substituted indole (I) is selected from
1-[5-(2-(1-pyrrolidino)ethyl)sulfonamidoindolyl-2-carbonyl]-4-[3-(1-methylethylamino)-2-pyridinyl]piperazine,
1-[5-(2-(1-piperidinyl)ethyl)sulfonamidoindolyl-2-carbonyl]-4-[3-(1-methylethylamino)-2-pyridinyl]piperazine,
1-[5-(2-(1-morpholinyl)ethyl)sulfonamidoindolyl-2-carbonyl]-4-[3-(1-methylethylamino)-2-pyridinyl]piperazine,
1-[5-(3-methylureido)indolyl-2-carbonyl]-4[N-methyl-N-(3-(1-methylethylamino)-2-pyridinyl)amino]piperidine,
1-[5-(4-pyridyl)ethanesulfonamido)indolyl-2-carbonyl]-4-[N-methyl-N-(3-(1-methylethylamino)-2-pyridinyl)amino]piperidine, and
1-[5-(4-methylpiperazin-1-ylcarbonylamino)indolyl-2-carbonyl]-4-[3-(1-methylethylamino)-2-pyridinyl)amino]piperazine.

13. Use of a substituted indole (I) as defined in any preceding claim, for the manufacture of a medicament for use in the treatment of AIDS.

## Patentansprüche (Patentansprüche für folgende(n) Vertragsstaat(en): AT, BE, CH, DE, DK, FR, GB, GR, IT, LU, MC, NL, SE)

1. Substituiertes Indol der Formel (I): 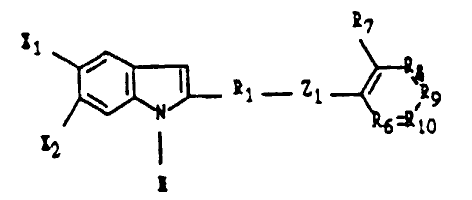 worin R₁ = -CH₂- oder -CO-;
worin Z₁ = 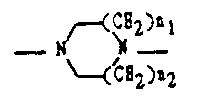 worin n₁ = 1 oder 2 und n₂ = 1 oder 2 oder 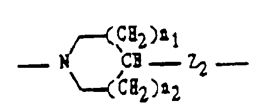 worin Z₂ = -N(Z₂₋₁)- mit Z₂₋₁ gleich C₁-C₄-Alkyl und mit n₁ und n₂ in der zuvor angegebenen Bedeutung;
worin einer der Reste X₁ und X₂ = H und der andere =
-O-(CH₂CH₂-O-)ₙ₃-X₁₋₁ mit n₃ gleich 1 bis 4 und X₁₋₁ gleich -H oder C₁-C₄-Alkyl,
-O-SO₂-(CH₂)ₙ₄-N(X₁₋₂)(X₁₋₃) mit n₄ gleich 1 bis 3 und entweder X₁₋₂ und X₁₋₃, die gleich oder verschieden sind, gleich -H oder C₁-C₆-Alkyl oder X₁₋₂ und X₁₋₃ zusammen mit dem daranhängenden Stickstoffatom gleich einem Ring, ausgewählt aus 1-Pyrrolidinyl, 1-Piperidinyl, 1-Piperazinyl, 1-Morpholinyl und 1-Piperazinyl, substituiert in 4-Stellung durch C₁-C₄-Alkyl,
-O-SO₂-(CH₂)ₙ₄-X₁₋₈ mit X₁₋₈ gleich 2-Pyridinyl, 3-Pyridinyl und 4-Pyridinyl und n₄ in der zuvor angegebenen Bedeutung,
-O-CO-(CH₂)ₙ₄-NX₁₋₂X₁₋₃ mit n₄, X₁₋₂ und X₁₋₃ in der zuvor angegebenen Bedeutung,
-NH-CO-(CH₂)ₙ₄-NX₁₋₂X₁₋₃ mit n₄, X₁₋₂ und X₁₋₃ in der zuvor angegebenen Bedeutung,
-N(X₁₋₄)-SO₂-X₁₋₅ mit X₁₋₄ gleich C₁-C₃-Alkyl und X₁₋₅ gleich C₁-C₄-Alkyl,
-N(X₁₋₇)-CO-NH-(CH₂)ₙ₄-N(X₁₋₂)(X₁₋₃) mit X₁₋₇ gleich -H oder C₁-C₃-Alkyl und n₄, X₁₋₂ und X₁₋₃ in der zuvor angegebenen Bedeutung,
-N(X₁₋₇)-SO₂-(CH₂)ₙ₄-X₁₋₈ mit n₄, X₁₋₇ und X₁₋₈ in der zuvor angegebenen Bedeutung,
-N(X₁₋₇)-CO-NH-(CH₂)ₙ₅-X₁₋₈ mit n₅ gleich 0 bis 3 und X₁₋₇ und X₁₋₈ in der zuvor angegebenen Bedeutung,
-N(X₁₋₇)-SO₂-(CH₂)ₙ₄-N(X₁₋₂)(X₁₋₃) mit n₄, X₁₋₂, X₁₋₃ und X₁₋₇ in der zuvor angegebenen Bedeutung,
-N(X₁₋₇)-CO-O-X₁₋₆ mit X₁₋₆ gleich C₁-C₄-Alkyl oder -(CH₂)ₙ₄-N(X₁₋₂)(X₁₋₃) mit n₄, X₁₋₂, X₁₋₃ und X₁₋₇ in der zuvor angegebenen Bedeutung,
-N(X₁₋₇)-CO-N(X₁₋₂)(X₁₋₃) mit X₁₋₂, X₁₋₃ und X₁₋₇ in der zuvor angegebenen Bedeutung,
-NH-CO-CF₃ oder
-N(X₁₋₇)-SO₂-N(X₁₋₂)(X₁₋₃) mit X₁₋₂, X₁₋₃ und X₁₋₇ in der zuvor angegebenen Bedeutung,
R₆ =
-N=,
-CH= oder
-N(O)=,
R₇ = -COO-R₇₋₁₁ mit R₇₋₁₁ gleich C₁-C₆-Alkyl,
-CO-N(R₇₋₃)(R₇₋₄) mit R₇₋₃ und R₇₋₄ die gleich oder verschieden sind, gleich -H oder C₁-C₆-Alkyl,
-N(R₇₋₅)(R₇₋₆) mit R₇₋₅ gleich
C₁-C₆-Alkyl,
-C(R₇₋₁₅)(R₇₋₁₆)-(R₇₋₁₇) mit R₇₋₁₅ und R₇₋₁₆, die gleich oder verschieden sind, gleich -H oder C₁-C₃-Alkyl und R₇₋₁₇ gleich C₂-C₅-Alkenyl mit 1 oder 2 Doppelbindung(en) oder C₂-C₅-Alkinyl mit einer Dreifachbindung,
-CH₂-CH₂-OH,
-CH₂-CH₂-CH₂-OH,
-CH(CH₃)CH₂-O-CH₃,
-CH(CH₃)CH₂-OH,
-CH₂-CF₃,
-CH₂-Cyclopropyl,
-CH₂-CH₂F,
-CH₂-CH₂-C≡N,
-C^{*}R₇₋₁₈-(CH₂)ₙ₁₄-C^{*}H₂ mit R₇₋₁₈ gleich -H oder -CH₃ und n₁₄ gleich 1 bis 5 und wobei die mit einem Sternchen (^{∗}) markierten Kohlenstoffatome miteinander unter Ringbildung verbunden sind,
-(CH₂)ₙ₁-N(R₇₋₇)(R₇₋₈) mit n₁ in der zuvor angegebenen Bedeutung und R₇₋₇ und R₇₋₈ die gleich oder verschieden sind, gleich -H oder C₁-C₄-Alkyl oder R₇₋₇ und R₇₋₈ zusammen mit dem daranhängenden Stickstoffatom gleich einem heterocyclischen Ring, ausgewählt aus 1-Pyrrolidinyl, 1-Piperidinyl, 1-Piperazinyl, N-Morpholinyl und 1-Aziridinyl,
und mit R₇₋₆ gleich -H,
C₁-C₆-Alkyl,
-C(R₇₋₁₅)(R₇₋₁₆)-(R₇₋₁₇) mit R₇₋₁₅, R₇₋₁₆ und R₇₋₁₇ in der zuvor angegebenen Bedeutung,
-CH₂-CH₂-OH,
-CH₂-CH₂-CH₂-OH,
-CH₂CF₃,
-CH₂-CH₂F,
-CH₂-CH₂-C≡N,
oder worin R₇₋₅ und R₇₋₆ zusammen mit dem daranhängenden Stickstoffatom einen heterocyclischen Ring, ausgewählt aus 1-Pyrrolidinyl, 1-Piperidinyl, 1-Piperazinyl, N-Morpholinyl und 1-Aziridinyl, bilden,
-(CH₂)ₙ₄-N(R₇₋₉)(R₇₋₁₀) mit n₄ in der zuvor angegebenen Bedeutung und R₇₋₉ und R₇₋₁₀, die gleich oder verschieden sind, gleich -H oder C₁-C₄-Alkyl oder R₇₋₉ und R₇₋₁₀ zusammen mit dem daranhängenden Stickstoffatom gleich einem heterocyclischen Ring, ausgewählt aus 1-Pyrrolidinyl, 1-Piperidinyl, 1-Piperazinyl und N-Morpholinyl,
R₈ = -N=,
-CR₈₋₁= mit R₈₋₁ gleich -H, -F, -Cl, -Br, -CF₃,
-NO₂, -COCF₃,
C₁-C₆-Alkyl,
C₁-C₃-Alkylthio,
-OH,
-O-R₈₋₂ mit R₈₋₂ gleich C₁-C₆-Alkyl, -φ, -CO-R₈₋₃ mit R₈₋₃ gleich C₁-C₆-Alkyl oder -φ,
-NH(R₈₋₄) mit R₈₋₄ gleich
C₁-C₆-Alkyl,
-C(R₈₋₇)(R₈₋₈)-(R₈₋₉) mit R₈₋₇ und R₈₋₈, die gleich oder verschieden sind, gleich -H oder C₁-C₃-Alkyl und R₈₋₉ gleich C₂-C₅-Alkenyl mit 1 oder 2 Doppelbindung(en) oder C₂-C₅-Alkinyl mit einer Dreifachbindung oder
-NR₈₋₅-CO-R₈₋₆ mit R₈₋₅ gleich -H oder C₁-C₆-Alkyl und R₈₋₆ gleich -H, C₁-C₆-Alkyl oder C₁-C₃-Alkoxy;
R₉ =
-N=,
-CR₉₋₁= mit R₉₋₁ gleich -H, -F, -Cl, -Br,
-NO₂, -COCF₃,
C₁-C₆-Alkyl,
C₁-C₃-Alkylthio,
-OH,
-O-R₉₋₂ mit R₉₋₂ gleich C₁-C₆-Alkyl, -φ oder -CO-R₉₋₃ mit R₉₋₃ gleich C₁-C₆-Alkyl oder -φ,
-N(R₉₋₄)(R₉₋₅) mit R₉₋₄ und R₉₋₅, die gleich oder verschieden sind, gleich
-H,
C₁-C₆-Alkyl,
-C(R₉₋₈)(R₉₋₉)-(R₉₋₁₀) mit R₉₋₈ und R₉₋₉, die gleich oder verschieden sind, gleich -H oder C₁-C₃-Alkyl und R₉₋₁₀ gleich C₂-C₅-Alkenyl mit 1 oder 2 Doppelbindung(en) und C₂-C₅-Alkinyl mit einer Dreifachbindung,
oder mit R₉₋₄ und R₉₋₅ zusammen mit dem daranhängenden Stickstoffatom gleich einem heterocyclischen Ring, ausgewählt aus 1-Pyrrolidinyl, 1-Piperidinyl, 1-Piperazinyl und N-Morpholinyl, oder
-NR₉₋₆-CO-R₉₋₇ mit R₉₋₆ gleich -H oder C₁-C₆-Alkyl und R₉₋₇ gleich -H, C₁-C₆-Alkyl oder C₁-C₃-Alkoxy;
R₁₀ =
-N=,
-CR₁₀₋₁= mit R₁₀₋₁ gleich -H, -F, -Cl, -Br, -CF₃,
-NO₂, -COCF₃,
C₁-C₆-Alkyl,
C₁-C₃-Alkylthio,
-OH,
-O-R₁₀₋₂ mit R₁₀₋₂ gleich C₁-C₆-Alkyl, -φ, -CO-R₁₀₋₃ mit R₁₀₋₃ gleich C₁-C₆-Alkyl oder -φ,
-N(R₁₀₋₄)(R₁₀₋₅) mit R₁₀₋₄ und R₁₀₋₅, die gleich oder verschieden sind, gleich -H,
C₁-C₆-Alkyl,
-C(R₁₀₋₈)(R₁₀₋₉)-(R₁₀₋₁₀) mit R₁₀₋₈ und R₁₀₋₉, die gleich oder verschieden sind, gleich -H oder C₁-C₃-Alkyl und R₁₀₋₁₀ gleich C₂-C₅-Alkenyl mit 1 oder 2 Doppelbindung(en) oder C₂-C₅-Alkinyl mit einer Dreifachbindung, -NR₁₀₋₆-CO-R₁₀₋₇ mit R₁₀₋₆ gleich -H oder C₁-C₆-Alkyl und R₁₀₋₇ gleich -H, C₁-C₆-Alkyl oder C₁-C₃-Alkoxy;
wobei gilt, daß nicht mehr als zwei der Reste von R₆, R₈, R₉ und R₁₀ für -N= stehen;
oder ein Enantiomer, pharmazeutisch akzeptables Salz, Hydrat oder Solvat desselben.

2. Substituiertes Indol (I) nach Anspruch 1 mit Z₁ gleich (Z-I), worin n₁ und n₂ für 1 stehen.

3. Substituiertes Indol (I) nach Anspruch 1 mit Z₁ gleich (Z-II), worin n₁ und n₂ für 1 stehen.

4. Substituiertes Indol (I) nach Anspruch 3, worin Z₂₋₁ für C₁-Alkyl steht.

5. Substituiertes Indol (I) nach Anspruch 1, worin R₁ für -CO- steht.

6. Substituiertes Indol (I) nach Anspruch 1, worin R₆ für -N= steht.

7. Substituiertes Indol (I) nach Anspruch 1, worin R₈, R₉ und R₁₀ jeweils für -CH= stehen.

8. Substituiertes Indol (I) nach Anspruch 1, worin R₇ für -N(R₇₋₅)(R₇₋₆) mit R₇₋₅ gleich C₂-C₄-Alkyl und R₇₋₆ gleich -H steht.

9. Substituiertes Indol (I) nach Anspruch 1, worin einer der Reste X₁ oder X₂ aus
-N(X₁₋₇)-CO-N(X₁₋₂)(X₁₋₃) und
-N(X₁₋₇)-SO₂-N(X₁₋₂)(X₁₋₃)
ausgewählt ist.

10. Substituiertes Indol (I) nach Anspruch 1, ausgewählt aus
1-[6-(2-(2-Hydroxyethoxy)ethoxy)ethoxyindolyl-2-carbonyl]-4-[3-(1-methylethylamino)-2-pyridinyl]piperazin,
1-[5-(2-(1-Pyrrolidino)ethyl)sulfonamidoindolyl-2-carbonyl]-4-[3-(1-methylethylamino)-2-pyridinyl]piperazin,
1-[6-(2-Hydroxyethoxy)ethoxyindolyl-2-carbonyl]-4-[3-(1-methylethylamino)-2-pyridinyl]piperazin,
1-[5-(2-(1-Piperidinyl)ethyl)sulfonamidoindolyl-2-carbonyl]-4-[3-(1-methylethylamino)-2-pyridinyl]piperazin,
1-[5-(2-(1-Morpholinyl)ethyl)sulfonamidoindolyl-2-carbonyl]-4-[3-(1-methylethylamino)-2-pyridinyl]piperazin,
1-[6-(2-(2-Hydroxyethoxy)ethoxy)ethoxyindolyl-2-carbonyl]-4-[N-methyl-N-(3-(1-methylethylamino)-2-pyridinyl]amino]piperidin,
1-[5-(3-Methylureido)-indolyl-2-carbonyl]-4-[N-methyl-N-(3-(1-methylethylamino)-2-pyridinyl)amino]piperidin,
1-[5-(2-(4-Pyridyl)-ethansulfonamido)-indolyl-2-carbonyl]-4-[N-methyl-N-(3-(1-methylethylamino)-2-pyridinyl)amino]piperidin und
1-[5-(4-Methylpiperazin-1-ylcarbonylamino)-indolyl-2-carbonyl]-4-[3-(1-methylethylamino)-2-pyridinyl)amino]piperazin.

11. Verwendung eines substituierten Indols (I) nach einem der vorhergehenden Ansprüche zur Herstellung eines Medikaments zur Verwendung bei der Behandlung von AIDS.

## Patentansprüche (Patentansprüche für folgende(n) Vertragsstaat(en): ES)

1. Verfahren zur Herstellung eines substituierten Indols der Formel (I) 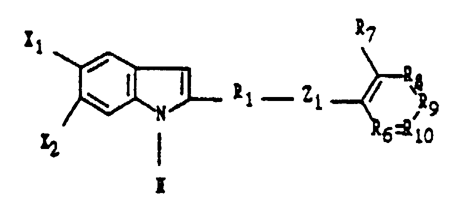 worin R₁ = -CH₂- oder -CO-; worin Z₁ = 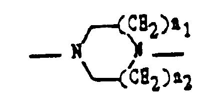 worin n₁ = 1 oder 2 und n₂ = 1 oder 2 oder 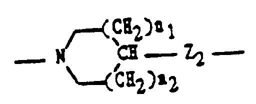 worin Z₂ = -N(Z₂₋₁)- mit Z₂₋₁ = C₁-C₄-Alkyl und mit n₁ und n₂ in der zuvor angegebenen Bedeutung;
worin einer der Reste X₁ und X₂ = H und der andere =
-O-(CH₂CH₂-O-)ₙ₃-X₁₋₁ mit n₃ = 1 bis 4 und X₁₋₁ gleich -H oder C₁-C₄-Alkyl,
-O-SO₂-(CH₂)ₙ₄-N(X₁₋₂)(X₁₋₃) mit n₄ = 1 bis 3 und entweder X₁₋₂ und X₁₋₃, die gleich oder verschieden sind, = -H oder C₁-C₆-Alkyl oder X₁₋₂ und X₁₋₃ zusammen mit dem daranhängenden Stickstoffatom gleich einem Ring, ausgewählt aus 1-Pyrrolidinyl, 1-Piperidinyl, 1-Piperazinyl, 1-Morpholinyl und 1-Piperazinyl, substituiert in 4-Stellung durch C₁-C₄-Alkyl,
-O-SO₂-(CH₂)ₙ₄-X₁₋₈ mit X₁₋₈ = 2-Pyridinyl, 3-Pyridinyl und 4-Pyridinyl und n₄ in der zuvor angegebenen Bedeutung,
-O-CO-(CH₂)ₙ₄-NX₁₋₂X₁₋₃ mit n₄, X₁₋₂ und X₁₋₃ in der zuvor angegebenen Bedeutung,
R₆ = -N=,
-CH= oder
-N(O)=,
R₇ = -COO-R₇₋₁₁ mit R₇₋₁₁ = C₁-C₆-Alkyl,
-CO-N(R₇₋₃)(R₇₋₄) mit R₇₋₃ und R₇₋₄, die gleich oder verschieden sind, gleich -H oder C₁-C₆-Alkyl,
-N(R₇₋₅)(R₇₋₆) mit R₇₋₅ gleich
C₁-C₆-Alkyl,
-C(R₇₋₁₅)(R₇₋₁₆)-(R₇₋₁₇) mit R₇₋₁₅ und R₇₋ ₁₆, die gleich oder verschieden sind, gleich -H oder C₁-C₃-Alkyl und R₇₋₁₇ gleich C₂-C₅-Alkenyl mit 1 oder 2 Doppelbindung(en) oder C₂-C₅-Alkinyl mit einer Dreifachbindung,
-CH₂-CH₂-OH,
-CH₂-CH₂-CH₂-OH,
-CH(CH₃)CH₂-O-CH₃,
-CH(CH₃)CH₂-OH,
-CH₂-CF₃,
-CH₂-Cyclopropyl,
-CH₂-CH₂F,
-CH₂-CH₂-C≡N,
-C*R₇₋₁₈-(CH₂)ₙ₁₄-C*H₂ mit R₇₋₁₈ gleich -H oder -CH₃ und n₁₄ gleich 1 bis 5 und wobei die mit einem Sternchen (*) markierten Kohlenstoffatome miteinander unter Ringbildung verbunden sind,
-(CH₂)ₙ₁-N(R₇₋₇)(R₇₋₈) mit n₁ in der zuvor angegebenen Bedeutung und R₇₋₇ und R₇₋₈, die gleich oder verschieden sind, gleich -H oder C₁-C₄-Alkyl oder R₇₋₇ und R₇₋₈ zusammen mit dem daranhängenden Stickstoffatom gleich einem heterocyclischen Ring, ausgewählt aus 1-Pyrrolidinyl, 1-Piperidinyl, 1-Piperazinyl, N-Morpholinyl und 1-Aziridinyl,
und mit R₇₋₆ gleich -H,
-C₁-C₆-Alkyl,
-C(R₇₋₁₅)(R₇₋₁₆)-(R₇₋₁₇) mit R₇₋₁₅, R₇₋₁₆ und R₇₋₁₇ in der zuvor angegebenen Bedeutung,
-CH₂-CH₂-OH,
-CH₂-CH₂-CH₂-OH,
-CH₂CF₃,
-CH₂-CH₂F,
-CH₂-CH₂-C≡N,
oder worin R₇₋₅ und R₇₋₆ zusammen mit dem daranhängenden Stickstoffatom einen heterocyclischen Ring, ausgewählt aus 1-Pyrrolidinyl, 1-Piperidinyl, 1-Piperazinyl, N-Morpholinyl und 1-Aziridinyl, bilden,
-(CH₂)ₙ₄-N(R₇₋₉)(R₇₋₁₀) mit n₄ in der zuvor angegebenen Bedeutung und R₇₋₉ und R₇₋₁₀, die gleich oder verschieden sind, gleich -H oder C₁-C₄-Alkyl oder R₇₋₉ und R₇₋₁₀ zusammen mit dem daranhängenden Stickstoffatom gleich einem heterocyclischen Ring, ausgewählt aus 1-Pyrrolidinyl, 1-Piperidinyl, 1-Piperazinyl und N-Morpholinyl,
R₈ = -N=,
-CR₈₋₁= mit R₈₋₁ gleich -H, -F, -Cl, -Br, -CF₃,
-NO₂, -COCF₃,
C₁-C₆-Alkyl,
C₁-C₃-Alkylthio,
-OH,
-O-R₈₋₂ mit R₈₋₂ gleich C₁-C₆-Alkyl, -φ, -CO-R₈₋₃ mit R₈₋₃ gleich C₁-C₆-Alkyl oder -φ,
-NH(R₈₋₄) mit R₈₋₄ gleich
C₁-C₆-Alkyl,
-C(R₈₋₇)(R₈₋₈)-(R₈₋₉) mit R₈₋₇ und R₈₋₈, die gleich oder verschieden sind, gleich -H oder C₁-C₃-Alkyl und R₈₋₉ gleich C₂-C₅-Alkenyl mit 1 oder 2 Doppelbindung(en) oder C₂-C₅-Alkinyl mit einer Dreifachbindung oder
-NR₈₋₅-CO-R₈₋₆ mit R₈₋₅ gleich -H oder C₁-C₆-Alkyl und R₈₋₆ gleich -H, C₁-C₆-Alkyl oder C₁₋C₃-Alkoxy;
R₉ = -N=,
-CR₉₋₁= mit R₉₋₁ gleich -H, -F, -Cl, -Br,
-NO₂, -COCF₃,
C₁-C₆-Alkyl,
C₁-C₃-Alkylthio,
-OH,
-O-R₉₋₂ mit R₉₋₂ gleich C₁-C₆-Alkyl, -φ, -CO-R₉₋₃ mit R₉₋₃ gleich C₁-C₆-Alkyl oder -φ,
-N(R₉₋₄)(R₉₋₅) mit R₉₋₄ und R₉₋₅, die gleich oder verschieden sind, gleich
-H,
C₁-C₆-Alkyl,
-C(R₉₋₈)(R₉₋₉)-(R₉₋₁₀) mit R₉₋₈ und R₉₋₉, die gleich oder verschieden sind, gleich -H oder C₁-C₃-Alkyl und R₉₋₁₀ gleich C₂-C₅-Alkenyl mit 1 oder 2 Doppelbindung(en) oder C₂-C₅-Alkinyl mit einer Dreifachbindung,
oder mit R₉₋₄ und R₉₋₅ zusammen mit dem daranhängenden Stickstoffatom gleich einem heterocyclischen Ring, ausgewählt aus 1-Pyrrolidinyl, 1-Piperidinyl, 1-Piperazinyl und N-Morpholinyl, oder
-NR₉₋₆-CO-R₉₋₇ mit R₉₋₆ gleich -H oder C₁-C₆-Alkyl und R₉₋₇ gleich -H, C₁-C₆-Alkyl oder C₁-C₃-Alkoxy;
R₁₀ = -N=,
-CR₁₀₋₁= mit R₁₀₋₁ gleich -H, -F, -Cl, -Br, -CF₃,
-NO₂, -COCF₃,
C₁-C₆-Alkyl,
C₁-C₃-Alkylthio,
-OH,
-O-R₁₀₋₂ mit R₁₀₋₂ gleich C₁-C₆-Alkyl, -φ, -CO-R₁₀₋₃ mit R₁₀₋₃ gleich C₁-C₆-Alkyl oder -φ,
-N(R₁₀₋₄)(R₁₀₋₅) mit R₁₀₋₄ und R₁₀₋₅, die gleich oder verschieden sind, gleich -H,
C₁-C₆-Alkyl,
-C(R₁₀₋₈)(R₁₀₋₉)-(R₁₀₋₁₀) mit R₁₀₋₈ und R₁₀₋₉, die gleich oder verschieden sind, gleich -H oder C₁-C₃-Alkyl und R₁₀₋₁₀ gleich C₂-C₅-Alkenyl mit 1 oder 2 Doppelbindung(en) oder C₂-C₅-Alkinyl mit einer Dreifachbindung, -N₁₀₋₆-CO-R₁₀₋₇ mit R₁₀₋₆ gleich -H oder C₁-C₆-Alkyl und R₁₀₋₇ gleich -H, C₁-C₆-Alkyl oder C₁-C₃-Alkoxy;
wobei gilt, daß nicht mehr als zwei der Reste von R₆, R₈, R₉ und R₁₀ für -N= stehen;
oder eines Enantiomeren, pharmazeutisch akzeptablen Salzes, Hydrats oder Solvats desselben durch Kupplung einer entsprechenden Indolssäure (VI) mit einem entsprechenden Amin (Z₁-aromatischer Teil).

2. Verfahren zur Herstellung eines substituierten Indols der Formel (I) gemäß der Definition in Anspruch 1 mit der Ausnahme, daß der andere der Reste X₁ und X₂ gleich
-NH-CO-(CH₂)ₙ₄-NX₁₋₂X₁₋₃ mit n₄, X₁₋₂ und X₁₋₃ in der in Anspruch 1 angegebenen Bedeutung,
-N(X₁₋₄)-SO₂-X₁₋₅ mit X₁₋₄ gleich C₁-C₃-Alkyl und X₁₋₅ gleich C₁-C₄-Alkyl,
-N(X₁₋₇)-CO-NH-(CH₂)ₙ₄-N(X₁₋₂)(X₁₋₃) mit X₁₋₇ gleich -H oder C₁-C₃-Alkyl und n₄, X₁₋₂ und X₁₋₃ in der in Anspruch 1 angegebenen Bedeutung,
-N(X₁₋₇)-SO₂-(CH₂)ₙ₄-X₁₋₈ mit n₄, X₁₋₇ und X₁₋₈ in der in Anspruch 1 angegebenen Bedeutung,
-N(X₁₋₇)-CO-NH-(CH₂)ₙ₅-X₁₋₈ mit n₅ gleich 0 bis 3 und X₁₋₇ und X₁₋₈ in der in Anspruch 1 angegebenen Bedeutung,
-N(X₁₋₇)-SO₂-(CH₂)ₙ₄-N(X₁₋₂)(X₁₋₃) mit n₄, X₁₋₂, X₁₋₃ und X₁₋₇ in der in Anspruch 1 angegebenen Bedeutung,
-N(X₁₋₇)-CO-O-X₁₋₆ mit X₁₋₆ gleich C₁-C₄-Alkyl oder -(CH₂)ₙ₄-N(X₁₋₂)(X₁₋₃), worin n₄, X₁₋₂, X₁₋₃ und X₁₋₇ die in Anspruch 1 angegebene Bedeutung besitzen,
-N(X₁₋₇)-CO-N(X₁₋₂)(X₁₋₃) mit X₁₋₂, X₁₋₃ und X₁₋₇ in der in Anspruch 1 angegebenen Bedeutung,
-NH-CO-CF₃ oder
-N(X₁₋₇)-SO₂-N(X₁₋₂)(X₁₋₃) mit X₁₋₂, X₁₋₃ und X₁₋₇ in der in Anspruch 1 angegebenen Bedeutung,
durch Umsetzen der entsprechenden Verbindung, wenn der andere Rest von X₁ und X₂ für NH₂ steht,
(i) mit Sulfonylchlorid und anschließend mit dem gewünschten Amin und dem pulverisierten Kupferkatalysator unter Rückfluß,
(ii) mit einem funktionalisierten Sulfonylchlorid, z.B. 2-(4-Pyridyl)ethansulfonylchlorid,
(iii) mit einem Isocyanat,
(iv) mit Phosgen oder einem Phosgenäquivalent und Einfangen mit einem Amin,
(v) mit X-SO₂-X und anschließende Umsetzung mit einem Amin.

3. Verfahren nach Anspruch 1 oder Anspruch 2, wobei Z₁ für (Z-I) mit n₁ und n₂ gleich 1 steht.

4. Verfahren nach Anspruch 1 oder Anspruch 2, wobei Z₁ für (Z-II) mit n₁ und n₂ für 1 steht.

5. Verfahren nach Anspruch 4, wobei Z₂₋₁ für C₁-Alkyl steht.

6. Verfahren nach Anspruch 1 oder Anspruch 2, wobei R₁ für -CO- steht.

7. Verfahren nach Anspruch 1 oder Anspruch 2, wobei R₆ für -N= steht.

8. Verfahren nach Anspruch 1 oder Anspruch 2, wobei R₈, R₉ und R₁₀ jeweils für -CH= stehen.

9. Verfahren nach Anspruch 1 oder Anspruch 2, wobei R₇ für -N(R₇₋₅)(R₇₋₆) mit R₇₋₅ gleich C₂-C₄-Alkyl und R₇₋₆ gleich -H steht.

10. Verfahren nach Anspruch 2, wobei der andere der Reste von X₁ und X₂ aus
-N(X₁₋₇)-CO-N(X₁₋₂)(X₁₋₃) und
-N(X₁₋₇)-SO₂-N(X₁₋₂)(X₁₋₃)
ausgewählt ist.

11. Verfahren nach Anspruch 1, wobei das substituierte Indol (I) aus
1-[6-(2-(2-Hydroxyethoxy)ethoxy)ethoxyindolyl-2-carbonyl]-4-[3-(1-methylethylamino)-2-pyridinyl]piperazin,
1-[6-(2-Hydroxyethoxy)ethoxyindolyl-2-carbonyl]-4-[3-(1-methylethylamino)-2-pyridinyl]piperazin und
1-[6-(2-(2-Hydroxyethoxy)ethoxy)ethoxyindolyl-2-carbonyl]-4-[N-methyl-N-(3-(1-methylethylamino)-2-pyridinyl)amino]piperidin
ausgewählt ist.

12. Verfahren nach Anspruch 2, wobei das substituierte Indol (I) aus
1-[5-(2-(1-Pyrrolidino)ethyl)sulfonamidoindolyl-2carbonyl]-4-[3-(1-methylethylamino)-2-pyridinyl]piperazin,
1-[5-(2-(1-Piperidinyl)ethyl)sulfonamidoindolyl-2-carbonyl]-4-[3-(1-methylethylamino)-2-pyridinyl]piperazin,
1-[5-(2-(1-Morpholinyl)ethyl)sulfonamidoindolyl-2-carbonyl]-4-[3-(1-methylethylamino)-2-pyridinyl]piperazin,
1-[5-(3-Methylureido)indolyl-2-carbonyl]-4-[N-methyl-N-(3-(1-methylethylamino)-2-pyridinyl)amino]piperidin,
1-[5-(4-Pyridyl)ethansulfonamido)indolyl-2-carbonyl]-4-[N-methyl-N-(3-(1-methylethylamino)-2-pyridinyl)amino]piperidin und
1-[5-(4-Methylpiperazin-1-ylcarbonylamino)indolyl-2-carbonyl]-4-[3-(1-methylethylamino)-2-pyridinyl)amino]piperazin
ausgewählt ist.

13. Verwendung eines substituierten Indols (I) gemäß Definition in einem der vorhergehenden Ansprüche zur Herstellung eines Medikaments zur Verwendung bei der Behandlung von AIDS.

## Revendications (Revendications pour l'(les) Etat(s) contractant(s) suivant(s): AT, BE, CH, DE, DK, FR, GB, GR, IT, LI, LU, MC, NL, SE)

1. Indole substitué de formule I 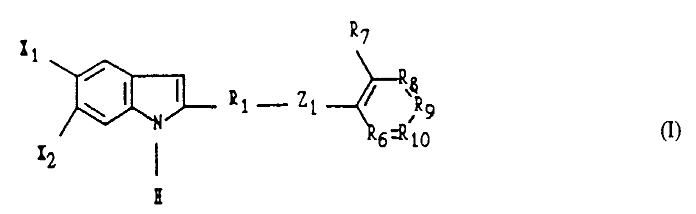 dans laquelle
R₁ représente un groupe -CH₂- ou -CO- ;
Z₁ représente un groupe 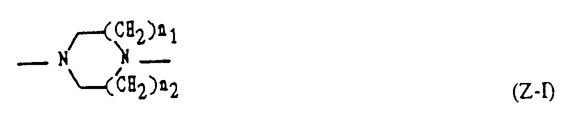 dans lequel n₁ est égal à 1 ou 2 et n₂ est égal à 1 ou 2, ou un groupe 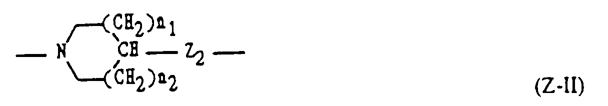
dans lequel Z₂ représente un groupe -N(Z₂₋₁) dans lequel Z₂₋₁ représente un groupe alkyle en C₁ à C₄ et dans lequel n₁ et n₂ répondent aux définitions précitées ;
un des groupes X₁ et X₂ représente H et l'autre représente un groupe
-O- (CH₂CH₂-O-)ₙ₃-X₁₋₁ dans lequel n₃ a une valeur de 1 à 4, X₁₋₁ représente H ou un groupe alkyle en C₁ à C₄,
-O-SO₂-(CH₂)ₙ₄-N(X₁₋₂)(X₁₋₃) dans lequel n₄ aune valeur de 1 à 3, X₁₋₂ et X₁₋₃ sont identiques ou différents et représentent H ou un groupe alkyle en C₁ à C₆, ou bien X₁₋₂ et X₁₋₃ sont pris conjointement avec l'atome d'azote auquel ils sont fixés en formant un noyau choisi entre les noyaux 1-pyrrolidinyle, 1-pipéridinyle, 1-pipérazinyle, 1-morpholinyle et 1-pipérazinyle substitués en position 4 avec un groupe alkyle en C₁ à C₄,
-O-SO₂-(CH₂)ₙ₄-X₁₋₈ dans lequel X₁₋₈ représente un groupe 2-pyridinyle, 3-pyridinyle ou 4-pyridinyle et n₄ répond à la définition précitée,
O-CO-(CH₂)ₙ₄-NX₁₋₂X₁₋₃ dans lequel n₄, X₁₋₂ et X₁₋₃ répondent aux définitions précitées,
-NH-CO-(CH₂ )ₙ₄-NX₁₋₂X₁₋₃ dans lequel n₄, X₁₋₂ et X₁₋₃ répondent aux définitions précitées,
-N(X₁₋₄)-SO₂-X₁₋₅ dans lequel X₁₋₄ représente un groupe alkyle en C₁ à C₃ et X₁₋₅ représente un groupe alkyle en C₁ à C₄,
-N(X₁₋₇)-CO-NH-(CH₂)ₙ₄-N(X₁₋₂) (X₁₋₃) dans lequel X₁₋₇ représente H ou un groupe alkyle en C₁ à C₃, et n₄, X₁₋₂ et X₁₋₃ répondent aux définitions précitées,
-N(X₁₋₇)-SO₂-(CH₂)ₙ₄-X₁₋₈ dans lequel n₄, X₁₋₇ et X₁₋₈ répondent aux définitions précitées,
-N(X₁₋₇)-CO-NH-(CH₂)ₙ₅-X₁₋₈ dans lequel n₅ a une valeur de 0 à 3 et X₁₋₇ et X₁₋₈ répondent aux définitions précitées,
-NX(₁₋₇)-SO₂-(CH₂)ₙ₄-N(X₁₋₂)(X₁₋₃) dans lequel n₄, X₁₋₂, X₁₋₃ et X₁₋₇ répondent aux définitions précitées,
-NX(₁₋₇)-CO-O-X₁₋₆ dans lequel X₁₋₆ représente un groupe alkyle en C₁ à C₄ ou -(CH₂)ₙ₄-N(X₁₋₂)(X₁₋₃) dans lequel n₄, X₁₋₂, X₁₋₃ et X₁₋₇ répondent aux définitions précitées,
-N(X₁₋₇)-CO-N(X₁₋₂)(X₁₋₃) dans lequel X₁₋₂, X₁₋₃ et X₁₋₇ répondent aux définitions précitées,
-NH-CO-CF₃, ou
-N(X₁₋₇)-SO₂-N(X₁₋₂)(X₁₋₃) dans lequel X₁₋₂, X₁₋₃ et X₁₋₇ répondent aux définitions précitées,
R₆ représente -N=,
un groupe -CH=, ou
un groupe -N(O)=,
R₇ représente un groupe -COO-R₇₋₁₁ dans lequel R₇₋₁₁ représente un groupe alkyle en C₁ à C₆,
-CO-N(R₇₋₃)(R₇₋₄) dans lequel R₇₋₃ et R₇₋₄ sont identiques ou différents et représentent -H ou un groupe alkyle en C₁ à C₆,
-N(R₇₋₅)(R₇₋₆) dans lequel R₇₋₅ représente un groupe
alkyle en C₁ à C₆,
-C(R₇₋₁₅)(R₇₋₁₆)-(R₇₋₁₇) dans lequel R₇₋₁₅ et R₇₋₁₆ sont identiques ou différents et représentent -H ou un groupe alkyle en C₁ à C₃ et R₇₋₁₇ représente un groupe alcényle en C₂ à C₅ contenant une ou deux doubles liaisons ou alcynyle en C₂ à C₅ contenant une triple liaison
-CH₂-CH₂-OH,
-CH₂-CH₂-CH₂-OH,
-CH(CH₃)CH₂-O-CH₃,
-CH(CH₃)CH₂-OH,
-CH₂-CF₃,
-CH₂-cyclopropyle
-CH₂-CH₂F,
-CH₂-CH₂-C≡N,
-C*R₇₋₁₈-(CH₂)ₙ₁₄-C*H₂ dans lequel R₇₋₁₈ représente -H ou un groupe -CH₃, n₁₄ a une valeur de 1 à 5 et les atomes de carbone marqués d'un astérisque (*) sont liés l'un à l'autre avec pour résultat la formation d'un noyau,
-(CH₂)ₙ₁-N(R₇₋₇)(R₇₋₈) dans lequel n₁ répond à la définition précitée et R₇₋₇ et R₇₋₈ sont identiques ou différents et représentent -H ou un groupe alkyle en C₁ à C₄, ou dans lequel R₇₋₇ et R₇₋₈ sont pris conjointement avec l'atome d'azote auquel ils sont fixés en formant un noyau hétérocyclique choisi entre les noyaux 1-pyrrolidinyle, 1-pipéridinyle, 1-pipérazinyle, N-morpholinyle et 1-aziridinyle,
et dans lequel R₇₋₆ représente -H, ou un groupe alkyle en C₁ à C₆,
-C(R₇₋₁₅)(R₇₋₁₆)(R₇₋₁₇) dans lequel R₇₋₁₅, R₇₋₁₆ et R₇₋₁₇ répondent aux définitions précitées,
-CH₂-CH₂-OH,
-CH₂-CH₂-CH₂-OH,
-CH₂-CF₃,
-CH₂-CH₂F,
-CH₂-CH₂-C=N,
ou dans lequel R₇₋₅ et R₇₋₆ sont pris conjointement avec l'atome d'azote auquel ils sont fixés en formant un noyau hétérocyclique choisi entre les noyaux 1-pyrrolidinyle, 1-pipéridinyle, 1-pipérazinyle, N-morpholinyle et 1-aziridinyle,
-(CH₂)ₙ₄-N(R₇₋₉)(R₇₋₁₀) dans lequel n₄ répond à la définition précitée et R₇₋₉ et R₇₋₁₀ sont identiques ou différents et représentent -H ou un groupe alkyle en C₁ à C₄, ou dans lequel R₇₋₉ et R₇₋₁₀ sont pris conjointement avec l'atome d'azote auquel ils sont fixés en formant un noyau hétérocyclique choisi entre les noyaux 1-pyrrolidinyle, 1-pipéridinyle, 1-pipérazinyle et N-morpholinyle,
R₈ représente -N=,
un groupe CR₈₋₁ dans lequel R₈₋₁ représente -H, -F, -Cl, -Br, un groupe -CF₃,
-NO₂, -COCF₃,
alkyle en C₁ à C₆,
alkylthio en C₁ à C₃,
-OH,
-O-R₈₋₂ dans lequel R₈₋₂ représente un groupe alkyle en C₁ à C₆, -φ, -CO-R₈₋₃ dans lequel R₈₋₃ représente un groupe alkyle en C₁ à C₆ ou -φ,
-NH(R₈₋₄) dans lequel R₈₋₄ représente un groupe alkyle en C₁ à C₆,
-C(R₈₋₇)(R₈₋₈)-(R₈₋₉) dans lequel R₈₋₇ et R₈₋₈ sont identiques ou différents et représentent -H ou un groupe alkyle en C₁ à C₃ et R₈₋₉ représente un groupe alcényle en C₂ à C₅ contenant une ou deux doubles liaisons ou un groupe alcynyle en C₂ à C₅ contenant une triple liaison, ou
-NR₈₋₅-CO-R₈₋₆ dans lequel R₈₋₅ représente -H ou un groupe alkyle en C₁ à C₆ et R₈₋₆ représente -H, un groupe alkyle en C₁ à C₆ ou alkoxy en C₁ à C₃ ;
R₉ représente -N=, ou un groupe
-CR₉₋₁dans lequel R₉₋₁ représente -H, -F, -Cl, -Br, un groupe
-NO₂, -COCF₃
alkyle en C₁ à C₆,
alkylthio en C₁ à C₃,
-OH,
-O-R₉₋₂ dans lequel R₉₋₂ représente un groupe alkyle en C₁ à C₆, -φ, -CO-R₉₋₃ dans lequel R₉₋₃ représente un groupe alkyle en C₁ à C₆ ou -φ,
-N(R₉₋₄)(R₉₋₅) dans lequel R₉₋₄ et R₉₋₅ sont identiques ou différents et représentent -H, ou un groupe
alkyle en C₁ à C₆,
-C(R₉₋₈)(R₉₋₉)-(R₉₋₁₀) dans lequel R₉₋₈ et R₉₋₉ sont identiques ou différents et représentent -H ou un groupe alkyle en C₁ à C₃ et R₉₋₁₀ représente un groupe alcényle en C₂ à C₅ contenant une ou deux doubles liaisons ou alcynyle en C₂ à C₅ contenant une triple liaison,
R₉₋₄ et R₉₋₅ sont pris conjointement avec l'atome d'azote auquel ils sont fixés en formant un noyau hétérocyclique choisi entre les noyaux 1-pyrrolidinyle, 1-pipéridinyle, 1-pipérazinyle et N-morpholinyle, ou
-NR₉₋₆-CO-R₉₋₇ dans lequel R₉₋₆ représente -H ou un groupe alkyle en C₁ à C₆ et R₉₋₇ représente -H, un groupe alkyle en C₁ à C₆ ou alkoxy en C₁ à C₃,
R₁₀ représente -N=, ou un groupe
-CR₁₀₋₁ dans lequel R₁₀₋₁ représente -H, -F, -Cl, -Br, un groupe -CF₃,
-NO₂, -COCF₃,
alkyle en C₁ à C₆,
alkylthio en C₁ à C₃,
-OH,
-O-R₁₀₋₂ dans lequel R₁₀₋₂ représente un groupe alkyle en C₁ à C₆, -φ, -CO-R₁₀₋₃ dans lequel R₁₀₋₃ représente un groupe alkyle en C₁ à C₆ ou -φ,
-N(R₁₀₋₄)(R₁₀₋₅) dans lequel R₁₀₋₄ et R₁₀₋₅ sont identiques ou différents et représentent -H,
un groupe alkyle en C₁ à C₆,
-C(R₁₀₋₈)(R₁₀₋₉)-(R₁₀₋₁₀) dans lequel R₁₀₋₈ et R₁₀₋₉ sont identiques ou différents et représentent -H ou un groupe alkyle en C₁ à C₃ et R₁₀₋₁₀ représente un groupe alcényle en C₂ à C₅ contenant une ou deux doubles liaisons ou un groupe alcynyle en C₂ à C₅ contenant une triple liaison, ou -NR₁₀₋₆-CO-R₁₀₋₇ dans lequel R₁₀₋₆ représente -H ou un groupe alkyle en C₁ à C₆ et R₁₀₋₇ représente -H, un groupe alkyle en C₁ à C₆ ou alkoxy en C₁ à C₃ ; sous réserve que pas plus de deux des groupes R₆, R₈, R₉ et R₁₀ ne représentent -N= ; ou un de ses énantiomères, sels pharmaceutiquement acceptables, hydrates ou produits de solvatation.

2. Indole substitué (I) suivant la revendication 1, dans lequel Z₁ représente un groupe (Z-I) dans lequel n₁ et n₂ sont égaux à 1.

3. Indole substitué (I) suivant la revendication 1, dans lequel Z₁ représente un groupe (Z-II) dans lequel n₁ et n₂ sont égaux à 1.

4. Indole substitué (I) suivant la revendication 3, dans lequel Z₂₋₁ représente un groupe alkyle en C₁.

5. Indole substitué (I) suivant la revendication 1, dans lequel R₁ représente un groupe -CO-.

6. Indole substitué (I) suivant la revendication 1, dans lequel R₆ représente un groupe -N=.

7. Indole substitué (I) suivant la revendication 1, dans lequel R₈, R₉ et R₁₀ représentent chacun un groupe -CH=.

8. Indole substitué (I) suivant la revendication 1, dans lequel R₇ représente un groupe -N(R₇₋₅)(R₇₋₆) dans lequel R₇₋₅ représente un groupe alkyle en C₁ à C₄ et R₇₋₆ représente -H.

9. Indole substitué (I) suivant la revendication 1, dans lequel un des groupes X₁ et X₂ est choisi entre les groupes
-N(X₁₋₇)-CO-N(X₁₋₂)(X₁₋₃) et
-N(X₁₋₇)-SO₂-N(X₁₋₂)(X₁₋₃).

10. Indole substitué (I) suivant la revendication 1, qui est choisi entre
la 1-[6-(2-(2-hydroxyéthoxy)éthoxy)éthoxyindolyl-2-carbonyl]-4-[3-(1-méthyléthylamino)-2-pyridinyl]pipérazine,
la 1-[5-(2-(1-pyrrolidino)éthyl)sulfonamidoindolyl-2-carbonyl]-4-[3-(1-méthyléthylamino)-2-pyridinyl]pipérazine,
la 1-[6-(2-hydroxyéthoxy)éthoxyindolyl-2-carbonyl]-4-[3-(1-méthyléthylamino)-2-pyridinyl]pipérazine,
la 1-[5-(2-(1-pipéridinyl)éthyl)sulfonamidoindolyl-2-carbonyl]-4-[3-(1-méthyléthylamino)-2-pyridinyl]pipérazine,
la 1-[5-(2-(1-morpholinyl)éthyl)sulfonamidoindolyl-2-carbonyl]-4-[3-(1-méthyléthylamino)-2-pyridinyl]pipérazine,
la 1-[6-(2-(2-hydroxyéthoxy)éthoxy)éthoxyindolyl-2-carbonyl]-4-[N-méthyl-N-(3-(1-méthyléthylamino)-2-pyridinyl)amino]pipéridine,
la 1-[5-(3-méthyluréido)indolyl-2-carbonyl]-4-[N-méthyl-N-(3-(1-méthyléthylamino)-2-pyridinyl)amino]pipéridine,
la 1-[5-(2-(4-pyridyl)éthane-sulfonamido))indolyl-2-carbonyl]-4-[N-méthyl-N-(3-(1-méthyléthylamino)-2-pyridinyl)amino]pipéridine, et
la 1-[5-(4-méthylpipérazine-1-ylcarbonylamino)indolyl-2-carbonyl]-4-[3-(1-méthyléthylamino)-2-pyridinyl)amino]pipérazine.

11. Utilisation d'un indole substitué (I) suivant l'une quelconque des revendications précédentes, pour la production d'un médicament destiné à être utilisé dans le traitement du SIDA.

## Revendications (Revendications pour l'(les) Etat(s) contractant(s) suivant(s): ES)

1. Procédé de préparation d'un indole substitué de formule I 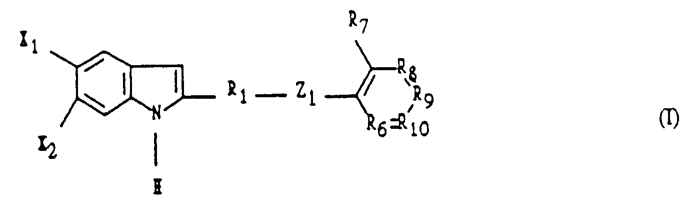
dans laquelle R₁ représente un groupe -CH₂- ou -CO- ;
Z₁ représente un groupe 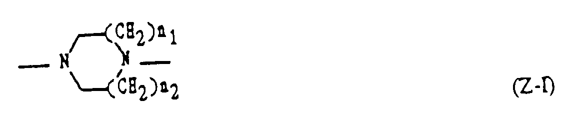 dans lequel n₁ est égal à 1 ou 2 et n₂ est égal à 1 ou 2, ou 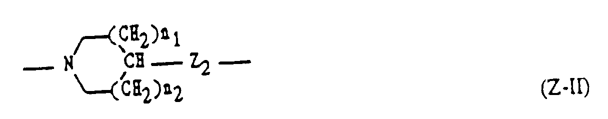 dans lequel Z₂ représente un groupe -N(Z₂₋₁) dans lequel Z₂₋₁ représente un groupe alkyle en C₁ à C₄ et n₁ et n₂ répondent aux définitions précitées ;
un des groupes X₁ et X₂ représente H et l'autre représente un groupe
-O-(CH₂CH₂-O-)ₙ₃-X₁₋₁ dans lequel n₃ a une valeur de 1 à 4, X₁₋₁ représente -H ou un groupe alkyle en C₁ à C₄,
O-SO₂(CH₂)ₙ₄-N(X₁₋₂)(X₁₋₃) dans lequel n₄ aune valeur de 1 à 3, X₁₋₂ et X₁₋₃ sont identiques ou différents et représentent -H ou un groupe alkyle en C₁ à C₆, ou bien X₁₋₂ et X₁₋₃ sont pris conjointement avec l'atome d'azote auquel ils sont fixés en formant un noyau choisi entre les noyaux 1-pyrrolidinyle, 1-pipéridinyle, 1-pipérazinyle, 1-morpholinyle et 1-pipérazinyle substitués
en position 4 avec un groupe alkyle en C₁ à C₄,
-O-SO₂-(CH₂)ₙ₄-X₁₋₈ dans lequel X₁₋₈ représente un groupe 2-pyridinyle, 3-pyridinyle ou 4-pyridinyle et n₄ répond à la définition précitée, ou
-O-CO-(CH₂)ₙ₄-NX₁₋₂X₁₋₃ dans lequel n₄, X₁₋₂ et X₁₋₃ répondent aux définitions précitées,
R₆ représente -N=, un groupe
-CH=, ou
-N(O)=,
R₇ représente un groupe -COO-R₇₋₁₁ dans lequel R₇₋₁₁ représente un groupe alkyle en C₁ à C₆,
-CO-N(R₇₋₃)(R₇₋₄) dans lequel R₇₋₃ et R₇₋₄ sont identiques ou différents et représentent -H ou un groupe alkyle en C₁ à C₆,
-N(R₇₋₅)(R₇₋₆) dans lequel R₇₋₅ représente un groupe
alkyle en C₁ à C₆,
-C(R₇₋₁₅)(R₇₋₁₆)-(R₇₋₁₇) dans lequel R₇₋₁₅ et R₇₋₁₆ sont identiques ou différents et représentent -H ou un groupe alkyle en C₁ à C₃ et R₇₋₁₇ représente un groupe alcényle en C₂ à C₅ contenant une ou deux doubles liaisons ou alcynyle en C₂ à C₅ contenant une triple liaison
-CH₂-CH₂-OH,
-CH₂-CH₂-CH₂-OH,
-CH(CH₃)CH₂-O-CH₃,
-CH(CH₃)CH₂-OH,
-CH₂-CF₃,
-CH₂-cyclopropyle
-CH₂-CH₂F,
-CH₂-CH₂-C=N,
-C*R₇₋₁₈-(CH₂)ₙ₁₄-C*H₂ dans lequel R₇₋₁₈ représente -H ou un groupe -CH₃, n₁₄ a une valeur de 1 à 5 et les atomes de carbone marqués d'un astérisque (*) sont liés l'un à l'autre avec pour résultat la formation d'un noyau,
-(CH₂)ₙ₁-N(R₇₋₇)(R₇₋₈) dans lequel n₁ répond à la définition précitée et R₇₋₇ et R₇₋₈ sont identiques ou différents et représentent -H ou un groupe alkyle en C₁ à C₄, ou bien R₇₋₇ et R₇₋₈ sont pris conjointement avec l'atome d'azote auquel ils sont fixés en formant un noyau hétérocyclique choisi entre les noyaux 1-pyrrolidinyle, 1-pipéridinyle, 1-pipérazinyle, N-morpholinyle et 1-aziridinyle,
et dans lequel R₇₋₆ représente -H, ou un groupe alkyle en C₁ à C₆,
-C(R₇₋₁₅)(R₇₋₁₆)-(R₇₋₁₇) dans lequel R₇₋₁₅, R₇₋₁₆ et R₇₋₁₇ répondent aux définitions précitées,
-CH₂-CH₂-OH,
-CH₂-CH₂-CH₂-OH,
-CH₂-CF₃,
-CH₂-CH₂F,
-CH₂-CH₂-C=N,
ou dans lequel R₇₋₅ et R₇₋₆ sont pris conjointement avec l'atome d'azote auquel ils sont fixés en formant un noyau hétérocyclique choisi entre les noyaux 1-pyrrolidinyle, 1-pipéridinyle, 1-pipérazinyle, N-morpholinyle et 1-aziridinyle,
-(CH₂)ₙ₄-N(R₇₋₉)(R₇₋₁₀) dans lequel n₄ répond à la définition précitée et R₇₋₉ et R₇₋₁₀ sont identiques ou différents et représentent -H ou un groupe alkyle en C₁ à C₄, ou bien R₇₋₉ et R₇₋₁₀ sont pris conjointement avec l'atome d'azote auquel ils sont fixés en formant un noyau hétérocyclique choisi entre les noyaux 1-pyrrolidinyle, 1-pipéridinyle, 1-pipérazinyle et N-morpholinyle,
R₈ représente -N=,
un groupe CR₈₋₁ dans lequel R₈₋₁ représente -H, -F, -Cl, -Br, un groupe -CF₃,
-NO₂, -COCF₃,
alkyle en C₁ à C₆,
alkylthio en C₁ à C₃,
-OH,
-O-R₈₋₂ dans lequel R₈₋₂ représente un groupe alkyle en C₁ à C₆ -CO-R₈₋₃ dans lequel R₈₋₃ représente un groupe alkyle en C₁ à C₆ ou -φ,
-NH(R₈₋₄) dans lequel R₈₋₄ représente un groupe alkyle en C₁ à C₆,
-C(R₈₋₇)(R₈₋₈)-(R₈₋₉) dans lequel R₈₋₇ et R₈₋₈ sont identiques ou différents et représentent -H ou un groupe alkyle en C₁ à C₃ et R₈₋₉ représente un groupe alcényle en C₂ à C₅ contenant une ou deux doubles liaisons ou un groupe alcynyle en C₂ à C₅ contenant une triple liaison, ou
-NR₈₋₅-CO-R₈₋₆ dans lequel R₈₋₅ représente -H ou un groupe alkyle en C₁ à C₆ et R₈₋₆ représente -H, un groupe alkyle en C₁ à C₆ ou alkoxy en C₁ à C₃ ;
R₉ représente -N=, ou un groupe
-CR₉₋₁ dans lequel R₉₋₁ représente -H, -F, -Cl, -Br, un groupe
-NO₂, -COCF₃
alkyle en C₁ à C₆,
alkylthio en C₁ à C₃,
-OH,
-O-R₉₋₂ dans lequel R₉₋₂ représente un groupe alkyle en C₁ à C₆ -CO-R₉₋₃ dans lequel R₉₋₃ représente un groupe alkyle en C₁ à C₆ ou -φ,
-N(R₉₋₄)(R₉₋₅) dans lequel R₉₋₄ et R₉₋₅ sont identiques ou différents et représentent
-H, ou un groupe
alkyle en C₁ à C₆,
-C(R₉₋₈)(R₉₋₉)-(R₉₋₁₀) dans lequel R₉₋₈ et R₉₋₉ sont identiques ou différents et représentent -H ou un groupe alkyle en C₁ à C₃ et R₉₋₁₀ représente un groupe alcényle en C₂ à C₅ contenant une ou deux doubles liaisons ou alcynyle en C₂ à C₅ contenant une triple liaison,
R₉₋₄ et R₉₋₅ sont pris conjointement avec l'atome d'azote auquel ils sont fixés en formant un noyau hétérocyclique choisi entre les noyaux 1-pyrrolidinyle, 1-pipéridinyle, 1-pipérazinyle et N-morpholinyle, ou
-NR₉₋₆-CO-R₉₋₇ dans lequel R₉₋₆ représente -H ou un groupe alkyle en C₁ à C₆ et R₉₋₇ représente -H, un groupe alkyle en C₁ à C₆ ou alkoxy en C₁ à C₃,
R₁₀ représente -N=,
un groupe
-CR₁₀₋₁ dans lequel R₁₀₋₁ représente -H, -F, -Cl, -Br, un groupe -CF₃,
-NO₂, -COCF₃,
alkyle en C₁ à C₆,
alkylthio en C₁ à C₃,
-OH,
-O-R₁₀₋₂ dans lequel R₁₀₋₂ représente un groupe alkyle en C₁ à C₆ ,-φ, -CO-R₁₀₋₃ dans lequel R₁₀₋₃ représente un groupe alkyle en C₁ à C₆ ou -φ,
-N(R₁₀₋₄)(R₁₀₋₅) dans lequel R₁₀₋₄ et R₁₀₋₅ sont identiques ou différents et représentent -H, un groupe
alkyle en C₁ à C₆,
-C(R₁₀₋₈)(R₁₀₋₉)-(R₁₀₋₁₀) dans lequel R₁₀₋₈ et R₁₀₋₉ sont identiques ou différents et représentent -H ou un groupe alkyle en C₁ à C₃ et R₁₀₋₁₀ représente un groupe alcényle en C₂ à C₅ contenant une ou deux doubles liaisons ou alcynyle en C₂ à C₅ contenant une triple liaison, un groupe -NR₁₀₋₆-CO-R₁₀₋₇ dans lequel R₁₀₋₆ représente -H ou un groupe alkyle en C₁ à C₆ et R₁₀₋₇ représente -H, un groupe alkyle en C₁ à C₆ ou alkoxy en C₁ à C₃ ;
sous réserve que pas plus de deux des groupes R₆, R₈, R₉ et R₁₀ ne représentent -N= ;
ou d'un de ses énantiomères, sels pharmaceutiquement acceptables, hydrates ou produits de solvatation ; qui comprend le couplage d'un acide à fonction indole (VI) correspondant avec une amine correspondante (Z₁-portion aromatique).

2. Procédé pour la préparation d'un indole substitué de formule (I) répondant à la définition suivant la revendication 1, sauf que l'autre des groupes X₁ et X₂ représente un groupe
-NH-CO-(CH₂)ₙ₄-NX₁₋₂X₁₋₃ dans lequel n₄, X₁₋₂ et X₁₋₃ répondent aux définitions suivant la revendication 1,
-N(X₁₋₄)-SO₂-X₁₋₅ dans lequel X₁₋₄ représente un groupe alkyle en C₁ à C₃ et X₁₋₅ représente un groupe alkyle en C₁ à C₄,
-N(X₁₋₇)-CO-NH-(CH₂)ₙ₄-N(X₁₋₂)(X₁₋₃) dans lequel X₁₋₇ représente -H ou un groupe alkyle en C₁ à C₃, et n₄, X₁₋₂ et X₁₋₃ répondent aux définitions suivant la revendication 1,
-N(X₁₋₇)-SO₂-(CH₂)ₙ₄-X₁₋₈ dans lequel n₄, X₁₋₇ et X₁₋₈ répondent aux définitions suivant la revendication 1,
-N(X₁₋₇)-CO-NH-(CH₂)ₙ₅-X₁₋₈ dans lequel n₅ a une valeur de 0 à 3 et X₁₋₇ et X₁₋₈ répondent aux définitions suivant la revendication 1,
-N(X₁₋₇)-SO₂-(CH₂)ₙ₄-N(X₁₋₂)(X₁₋₃) dans lequel n₄, X₁₋₂, X₁₋₃ et X₁₋₇ répondent aux définitions suivant la revendication 1,
-NX₍₁₋₇₎-CO-O-X₁₋₆ dans lequel X₁₋₆ représente un groupe alkyle en C₁ à C₄ ou -(CH₂)ₙ₄-N(X₁₋₂)(X₁₋₃) dans lequel n₄, X₁₋₂, X₁₋₃ et X₁₋₇ répondent aux définitions suivant la revendication 1,
-N(X₁₋₇)-CO-N(X₁₋₂)(X₁₋₃) dans lequel X₁₋₂, X₁₋₃ et X₁₋₇ répondent aux définitions suivant la revendication 1,
-NH-CO-CF₃, ou
-N(X₁₋₇)-SO₂-N(X₁₋₂)(X₁₋₃) dans lequel X₁₋₂, X₁₋₃ et X₁₋₇ répondent aux définitions suivant la revendication 1, qui comprend la réaction du composé correspondant, lorsque l'autre des composés X₁ et X₂ représente un groupe -NH₂,
(i) avec le chlorure de sulfonyle et ensuite avec l'amine désirée et un catalyseur au cuivre pulvérisé, dans des conditions de reflux,
(ii) avec un chlorure de sulfonyle fonctionnalisé tel que le chlorure de 2-(4-pyridyl)éthane-sulfonyle,
(iii) avec un isocyanate,
(iv) avec le phosgène ou un équivalent du phosgène, et un piégeage avec une amine,
(v) avec un groupe composé de formule X-SO₂-X, puis réaction avec une amine.

3. Procédé suivant la revendication 1 ou la revendication 2, dans lequel Z₁ représente un groupe (Z-I) dans lequel n₁ et n₂ sont égaux à 1.

4. Procédé suivant la revendication 1 ou la revendication 2, dans lequel Z₁ représente un groupe (Z-II) dans lequel n₁ et n₂ sont égaux à 1.

5. Procédé suivant la revendication 4, dans lequel Z₂₋₁ représente un groupe alkyle en C₁.

6. Procédé suivant la revendication 1 ou la revendication 2, dans lequel R₁ représente un groupe -CO-.

7. Procédé suivant la revendication 1 ou la revendication 2, dans lequel R₆ représente -N=.

8. Procédé suivant la revendication 1 ou la revendication 2, dans lequel R₈, R₉ et R₁₀ représentent chacun un groupe -CH=.

9. Procédé suivant la revendication 1 ou la revendication 2, dans lequel R₇ représente un groupe -N(R₇₋₅)(R₇₋₆) dans lequel R₇₋₅ représente un groupe alkyle en C₂ à C₄ et R₇₋₆ représente -H.

10. Procédé suivant la revendication 2, dans lequel l'autre des groupes X₁ et X₂ est choisi entre des groupes
-N(X₁₋₇)-CO-N(X₁₋₂)(X₁₋₃) et
-N(X₁₋₇)-SO₂-N(X₁₋₂)(X₁₋₃) .

11. Procédé suivant la revendication 1, dans lequel l'indole substitué (I) est choisi entre
la 1-[6-(2-(2-hydroxyéthoxy)éthoxy)éthoxyindolyl-2-carbonyl]-4-[3-(1-méthyléthylamino)-2-pyridinyl]pipérazine,
la 1-[6-(2-hydroxyéthoxy)éthoxyindolyl-2-carbonyl]-4-[3-(1-méthyléthylamino)-2-pyridinyl]pipérazine, et la 1-[6-(2-(2-hydroxyéthoxyléthoxy)éthoxyindolyl-2-carbonyl]-4-[N-méthyl-N-(3-(1-méthyléthylamino)-2-pyridinyl)amino]pipéridine.

12. Procédé suivant la revendication 2, dans lequel l'indole substitué (I) est choisi entre
la 1-[5-(2-(1-pyrrolidino)éthyl)sulfonamidoindolyl-2-carbonyl]-4-[3-(1-méthyléthylamino)-2-pyridinyl]pipérazine,
la 1-[5-(2-(1-pipéridinyl)éthyl)sulfonamidoindolyl-2-carbonyl]-4-[3-(1-méthyléthylamino)-2-pyridinyl]pipérazine,
la 1-[5-(2-(1-morpholinyl)éthyl)sulfonamidoindolyl-2-carbonyl]-4-[3-(1-méthyléthylamino)-2-pyridinyl]pipérazine,
la 1-[5-(3-méthyluréido)indolyl-2-carbonyl]-4-[N-méthyl-N-(3-(1-méthyléthylamino)-2-pyridinyl)amino]pipéridine,
la 1-[5-(4-pyridyl)éthane-sulfonamido))indolyl-2-carbonyl]-4-[N-méthyl-N-(3-(1-méthyléthylamino)-2-pyridinyl)amino]pipéridine, et
la 1-[5-(4-méthylpipérazine-1-ylcarbonylamino)indolyl-2-carbonyl]-4-[3-(1-méthyléthylamino)-2-pyridinyl)aminol pipérazine.

13. Utilisation d'un indole substitué (I) répondant à la définition suivant l'une quelconque des revendications précédentes, pour la production d'un médicament destiné à être utilisé dans le traitement du SIDA.
